# EUROPEAN PATENT APPLICATION

(11) **EP 4 843 986 A1**
(43) Date of publication of application: **25.11.2026**
(21) Application number: 25741572.9
(22) Date of filing: 16.01.2025
(51) Int. Cl.: C07D 487/08, C07D 241/38, A61P 25/02, A61K 31/49

(54) **ANGIOTENSIN II RECEPTOR ANTAGONIST AND USE THEREOF IN TREATMENT OF PERIPHERALLY-INDUCED NEUROPATHIC PAIN**

(30) Priority: 17.01.2024 WO PCT/CN2024/072719; 29.11.2024 WO PCT/CN2024/135584
(71) Applicant: Beijing Tide Pharmaceutical Co., Ltd., Beijing 100176 (CN)
(72) Inventor: WANG, Hongjun, Beijing 100176 (CN); LIANG, Huining, Beijing 100176 (CN); WANG, Xiaoqian, Beijing 100176 (CN); ZHAO, Yanping, Beijing 100176 (CN); LAN, Zhou, Beijing 100176 (CN); HOU, Siyu, Beijing 100176 (CN)
(74) Representative: Turner, Craig Robert
(86) International application number: PCT/CN2025/072712
(87) International publication number: WO 2025/153011

(57) **Abstract**

The present invention relates to an angiotensin II receptor antagonist and a use thereof in the treatment of peripherally-induced neuropathic pain. In particular, the present invention provides a compound of Formula (IV) or a pharmaceutically acceptable salt thereof or a stereoisomer thereof, and a use thereof in the treatment of peripherally-induced neuropathic pain.

## Description

### Technical Field

The present invention relates to angiotensin II type 2 (AT₂) receptor antagonists and use thereof for preventing or treating peripheral neuropathic pain and associated disorders.

### Background Art

The renin-angiotensin system (RAS) plays an important role in neuroprotection and nerve regeneration, and can regulate arterial blood pressure and ionic homeostasis in vivo. AngII has two major receptors, namely AngII type 1 receptor (AT1R) and AngII type 2 receptor (AT2R). Both AT1R and AT2R are seven-transmembrane receptors and exhibit similar affinity for AngII. In the rat brain, the predominant AngII receptor subtype is the AT₂ receptor. AT₂ receptor-specific antagonists are valuable for the treatment of various cerebrovascular, cognitive and central nervous system (CNS) disorders. In addition, AT₂ receptors have been found in neuronal tumor cells and transformed human neural cells. The AT₂ receptor is further involved in the differentiation and regeneration of neuronal tissue as well as bone maintenance. AT2R is expressed in injured nerves and infiltrating immune cells and is associated with neurological pain.

The International Association for the Study of Pain defines neuropathic pain as pain arising from a lesion or disease of somatosensory system (Jensen TS, Baron R, Haanpää M, et al . A new definition of neuropathic pain[J]. Pain, 2011,152(10):2204-2205). According to the anatomical location of the lesion or disease, neuropathic pain can be classified into peripheral neuropathic pain (peripherally-induced neuropathic pain, pNP) and central neuropathic pain (Dworkin RH, Backonja M, Rowbotham MC, et al. Advances in neuropathic pain: diagnosis, mechanisms, and treatment recommendations[J]. Arch Neurol, 2003,60:1524-1534).

According to the International Classification of Diseases 11th Revision (ICD-11), peripheral neuropathic pain refers to chronic pain arising from a lesion or disease of peripheral sensory nervous system. Peripheral neuropathic pain can be divided into five subtypes: trigeminal neuralgia, chronic neuropathic pain following peripheral nerve injury, painful polyneuropathy, postherpetic neuralgia, and painful radiculopathy. (Chronic Neuropathic Pain, Chinese Journal of Pain Medicine 2021, 27 (7)).

Peripheral neuropathic pain is relatively common in clinical practice. Common syndromes include painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy.

After peripheral nerve lesions lead to pNP, the disease course is prolonged with complex clinical manifestations, such as sensory impairment, motor dysfunction, autonomic nerve dysfunction, and painful symptoms in the corresponding innervated areas. The pain may be spontaneous, persistent, or paroxysmal pain. In addition, patients may present with muscle spasm, stiffness, weakness, atrophy, and other symptoms. Physical examination reveals decreased muscle tension, muscular atrophy, attenuated or absent tendon reflexes, and paresthesia, and the like. Even if the primary cause is eliminated and the injury heals or is effectively controlled, the pain persists chronically. It seriously impairs patients' quality of life and is accompanied by emotional disorders (Editorial Committee of Chinese Expert Consensus on Peripheral Neuropathic Pain. Chinese Expert Consensus on the Diagnosis and Treatment of Peripheral Neuropathic Pain. Chinese Journal of Pain Medicine, 2020, 26(05): 321-328). The clinical characteristics include spontaneous pain, allodynia, hyperalgesia, and paresthesia.

Painful diabetic peripheral neuropathy (PDPN) is the most common chronic complication of diabetes. It affects 16% of diabetic patients; many remain undiagnosed (12.5%) and untreated (39%). In 2013, the prevalence of type 2 diabetes in China reached as high as 10.4%. Based on this, it is estimated that approximately 22 million patients suffer from PDPN.

Postherpetic neuralgia (PHN) is also a common type of pNP, with an annual incidence of 3.9 to 42.0 per 100,000 persons. PHN develops in 9% to 34% of patients with herpes zoster. Among patients aged ≥40 years who visited the departments of dermatology, neurology, and pain management in urban hospitals across China, the overall prevalence of herpes zoster was 7.7%, and that of PHN was 2.3%. The prevalence rates of both conditions exhibit a gradual upward trend with advancing age.

Trigeminal neuralgia is a common cranial nerve disorder in clinical practice, with a prevalence of 182 per 100,000 persons and an annual incidence of 3 to 5 per 100,000 persons. It mostly occurs in adults and the elderly, with a peak onset age ranging from 48 to 59 years old.

Thus, peripheral neuropathic pain has profound emotional and socioeconomic impacts on patients. However, existing therapeutic drugs and methods yield limited efficacy, with relatively severe side effects. Therefore, there is a substantial unmet medical need for pNP.

Specifically, drugs are the primary current treatment modality for pNP, yet they have limited efficacy and pose safety risks. The most commonly used drugs to relieve chronic peripheral neuropathic pain are opioids, anticonvulsants, and antidepressants. Opioids are effective for acute pain; however, they are limited as treatment options for chronic peripheral neuropathic pain due to a high risk of drug abuse and potentially severe adverse reactions. Anticonvulsants such as gabapentin and pregabalin, as well as tricyclic antidepressants, produce marked central side effects.

Gabapentin and pregabalin are currently the most widely used centrally acting analgesics for clinical treatment of peripheral neuropathic pain (pNP). A large amount of clinical data shows that gabapentin and pregabalin have marked central side effects. Fatigue and dizziness are the most common side effects of such drugs; other side effects include headache and anxiety, as well as digestive system-related symptoms such as nausea, constipation, flatulence, and stomachache. Opioid analgesics provide favorable analgesic efficacy in clinical practice, but their severe side effects include respiratory depression and addiction, together with nausea, vomiting, and dizziness.

Therefore, there remains a need in the art for drugs for treating peripheral neuropathic pain with higher efficacy and fewer side effects. The present invention substantially addresses this need.

### Disclosure of the Invention

The present invention provides compounds acting as AT₂ receptor antagonists, which possess superior properties including excellent inhibitory activity against AT₂ receptors, enhanced physicochemical properties (e.g., solubility, physical and/or chemical stability), improved pharmacokinetic properties (e.g., improved bioavailability, appropriate half-life and duration of action), and improved safety (lower toxicity and/or fewer side effects, and a wider therapeutic window).

The present invention particularly provides use of AT₂ receptor antagonist compounds in the prevention or treatment of peripheral neuropathic pain (pNP).

The mechanism underlying the present invention is that AngII, acting on AT₂ receptors, induces the generation of inflammatory factors such as reactive oxygen species (ROS) in macrophages in the skin at the injured nerves or macrophages surrounding the injured nerves; ROS act on dorsal root ganglia to cause calcium influx in dorsal root ganglion neurons, thereby resulting in allodynia. AT₂ receptor antagonists can reduce the recruitment of macrophages at pain sites and inhibit high expression of AT₂ receptors within macrophages; by antagonizing AT₂ receptors, such antagonists suppress the generation of reactive oxygen/nitrogen species and further inhibit pain signal transmission. The compounds of the present invention exert analgesic effects via immune cells of the peripheral nervous system rather than through the central nervous system, thereby avoiding central side effects induced by existing drugs.

Therefore, more specifically, the present invention provides use of compounds acting as AT₂ receptor antagonists in the prevention or treatment of macrophage-associated peripheral neuropathic pain, in particular peripheral neuropathic pain associated with the recruitment/accumulation of macrophages, peripheral neuropathic pain associated with high expression of AT₂ receptors in macrophages, and/or peripheral neuropathic pain associated with elevated levels of reactive oxygen/nitrogen species generated by macrophages.

The inventors discovered that, in an assay to assess the effect of AT₂ receptor antagonists on angiotensin II (AngII)-induced reactive oxygen species generation in isolated mouse peritoneal macrophages, AT₂ receptor antagonists inhibit the AngII-induced elevation of reactive oxygen species (ROS) in mouse macrophages by antagonizing AT₂ receptors. Furthermore, in vivo pharmacodynamic studies confirm that AT₂ receptor antagonists produce potent analgesic effects via the above-described mechanism; such antagonists exhibit significant analgesic effects in a rat diabetic foot pain model, a rat model of chronic constriction injury of the sciatic nerve, a rat spinal nerve ligation model, and a mouse spared nerve injury model.

Accordingly, in a first aspect, the present application provides the following embodiments of AT₂ receptor antagonist compounds:
1. A compound, or a pharmaceutically acceptable salt, ester, stereoisomer, polymorph, solvate, N-oxide, isotopically labeled compound, metabolite, or prodrug thereof, wherein the compound has the structure of Formula (IV): 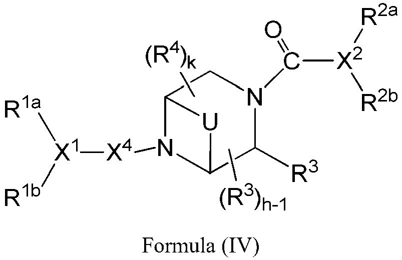 wherein:
   U is C₁₋₃ alkylene;
   R^{1a} is selected from the group consisting of C₂₋₈ alkenyl and C₂₋₈ alkynyl, wherein the C₂₋₈ alkenyl and C₂₋₈ alkynyl are each substituted with one C₆₋₁₀ aryl or 5- to 14-membered heteroaryl; C₆₋₁₀ aryl; -C₁₋₆ alkylene-saturated or partially unsaturated C₃₋₁₀ cyclic hydrocarbyl; -C₁₋₆ alkylene-saturated or partially unsaturated 3- to 10-membered heterocyclyl; -C₁₋₆ alkylene-C₆₋₁₀ aryl; and -C₁₋₆ alkylene-(5- to 14-membered heteroaryl);
   R^{1b} is absent, or is selected from the group consisting of H; C₁₋₈ alkyl optionally substituted with 1, 2, 3, or more R¹³ groups; saturated or partially unsaturated C₃₋₁₀ cyclic hydrocarbyl; C₆₋₁₀ aryl; -C₁₋₆ alkylene-saturated or partially unsaturated C₃₋₁₀ cyclic hydrocarbyl; and -C₁₋₆ alkylene-C₆₋₁₀ aryl;
   X¹ is absent, or is CR¹⁰ or N;
   X⁴ is selected from the group consisting of C(=O); -O-C(=O)- and -S-C(=O)-, wherein the O and S are attached to X¹;
   R^{2a} is C₆₋₁₀ aryl;
   R^{2b} is C₆₋₁₀ aryl;
   X² is CR¹⁰ or N;
   R³ is -C(=O)OR¹¹;
   R⁴ is H;
   R¹⁰ is, at each occurrence, selected from the group consisting of H, -OR¹¹, -SR¹¹, and C₁₋₆ alkyl;
   R¹¹ and R¹² are, at each occurrence, independently H or C₁₋₆ alkyl;
   h and k are each independently 1;
   the alkylene, cyclic hydrocarbyl, heterocyclyl, aryl and heteroaryl groups recited above are, at each occurrence, optionally substituted with 1, 2, 3, or more R¹³ groups;
   R¹³ is, at each occurrence, independently selected from the group consisting of halogen, cyano, nitro, C₁₋₆ alkyl, C₂₋₆ alkenyl, C₂₋₆ alkynyl, C₃₋₁₀ cyclic hydrocarbyl, C₆₋₁₀ aryl, 5- to 14-membered heteroaryl, -OR¹¹, -SR¹¹, -P(O)R¹¹R¹², and -NR¹¹R¹²; wherein the alkyl, alkylene, aryl and heteroaryl groups recited for substituent R¹³ are each optionally further substituted with 1, 2, 3, or more substituents independently selected from halogen and C₁₋₆ alkyl.
2. The compound according to Embodiment 1, or a pharmaceutically acceptable salt, ester, stereoisomer, polymorph, solvate, N-oxide, isotopically labeled compound, metabolite, or prodrug thereof, wherein:
   R¹³ is, at each occurrence, independently selected from the group consisting of halogen, cyano, nitro, C₁₋₆ alkyl, C₃₋₇ cyclic hydrocarbyl, C₆₋₁₀ aryl, 5- to 14-membered heteroaryl, -OR¹¹, -SR¹¹, and -NR¹¹R¹²; wherein the alkyl, aryl and heteroaryl groups recited for substituent R¹³ are each optionally further substituted with 1, 2, 3, or more substituents independently selected from halogen and C₁₋₆ alkyl.
3. The compound according to Embodiment 1, or a pharmaceutically acceptable salt, ester, stereoisomer, polymorph, solvate, N-oxide, isotopically labeled compound, metabolite, or prodrug thereof, wherein U is methylene or ethylene.
4. The compound according to Embodiment 1, or a pharmaceutically acceptable salt, ester, stereoisomer, polymorph, solvate, N-oxide, isotopically labeled compound, metabolite, or prodrug thereof, wherein R³ is - COOH.
5. The compound according to Embodiment 1, or a pharmaceutically acceptable salt, ester, stereoisomer, polymorph, solvate, N-oxide, isotopically labeled compound, metabolite, or prodrug thereof, wherein R¹⁰ is, at each occurrence, independently H, C₁₋₄ alkyl, OH, or SH.
6. The compound according to Embodiment 1, or a pharmaceutically acceptable salt, ester, stereoisomer, polymorph, solvate, N-oxide, isotopically labeled compound, metabolite, or prodrug thereof, wherein R¹¹ and R¹² are, at each occurrence, independently selected from the group consisting of H and C₁₋₄ alkyl.
7. The compound according to Embodiment 1, or a pharmaceutically acceptable salt, ester, stereoisomer, polymorph, solvate, N-oxide, isotopically labeled compound, metabolite, or prodrug thereof, wherein R¹³ is, at each occurrence, independently selected from the group consisting of F, Cl, Br, I, amino, cyano, nitro; C₁₋₄ alkyl optionally substituted with 1, 2, 3, or more substituents independently selected from halogen; C₅₋₇ cyclic hydrocarbyl; phenyl, 5- to 6-membered heteroaryl, and 9- to 10-membered heteroaryl, each optionally substituted with 1, 2, 3, or more substituents independently selected from halogen, OH, amino, cyano and C₁₋₄ alkyl; -OR¹¹ wherein R¹¹ is C₁₋₆ alkyl optionally substituted with 1, 2, 3, or more halogens; -SR¹¹ wherein R¹¹ is C₁₋₆ alkyl optionally substituted with 1, 2, 3, or more halogens; and -NR¹¹R¹² or -P(O)R¹¹R¹² wherein R¹¹ and R¹² are, at each occurrence, independently C₁₋₆ alkyl optionally substituted with 1, 2, 3, or more halogens.
8. The compound according to Embodiment 1, or a pharmaceutically acceptable salt, ester, stereoisomer, polymorph, solvate, N-oxide, isotopically labeled compound, metabolite, or prodrug thereof, wherein R¹³ is, at each occurrence, independently selected from the group consisting of F, Cl, Br, I, amino, cyano, nitro; C₁₋₄ alkyl optionally substituted with 1, 2, or 3 F or Cl; -OR¹¹ wherein R¹¹ is C₁₋₃ alkyl optionally substituted with 1, 2, or 3 F or Cl; -SR¹¹ wherein R¹¹ is C₁₋₃ alkyl optionally substituted with 1, 2, or 3 F or Cl; -NR¹¹R¹² or -P(O)R¹¹R¹² wherein R¹¹ and R¹² are, at each occurrence, independently C₁₋₃ alkyl; and phenyl, 5- to 6-membered heteroaryl, and 9- to 10-membered heteroaryl, each optionally substituted with 1, 2, 3, or more substituents independently selected from F, Cl, Br, I, and methyl.
9. The compound according to Embodiment 8, or a pharmaceutically acceptable salt, ester, stereoisomer, polymorph, solvate, N-oxide, isotopically labeled compound, metabolite, or prodrug thereof, wherein the C₁₋₃ alkyl is methyl, ethyl, propyl, or isopropyl.
10. The compound according to Embodiment 1, or a pharmaceutically acceptable salt, ester, stereoisomer, polymorph, solvate, N-oxide, isotopically labeled compound, metabolite, or prodrug thereof, wherein:
   R^{1a} is selected from the group consisting of C₂₋₆ alkenyl and C₂₋₆ alkynyl, wherein the C₂₋₆ alkenyl and C₂₋₆ alkynyl are each substituted with one phenyl or 5- to 10-membered heteroaryl; phenyl; -C₁₋₃ alkylene-C₃₋₇ cyclic hydrocarbyl; -C₁₋₃ alkylene-(5- to 7-membered monocyclic heterocyclyl); -C₁₋₃ alkylene-(8- to 10-membered benzo-fused heterocyclyl); -C₁₋₃ alkylene-phenyl; and -C₁₋₃ alkylene-(5- to 10-membered heteroaryl);
   R^{1b} is absent, or is selected from the group consisting of H; C₁₋₆ alkyl; C₃₋₇ cyclic hydrocarbyl; phenyl; -C₁₋₃ alkylene-C₃₋₇ cyclic hydrocarbyl; and -C₁₋₃ alkylene-phenyl; and
   the alkyl, alkylene, cyclic hydrocarbyl, heterocyclyl, aryl and heteroaryl groups recited above are, at each occurrence, optionally substituted with 1, 2, 3, or more R¹³ groups as defined in Embodiment 1.
11. The compound according to Embodiment 10, or a pharmaceutically acceptable salt, ester, stereoisomer, polymorph, solvate, N-oxide, isotopically labeled compound, metabolite, or prodrug thereof, wherein R^{1a} is selected from the group consisting of phenyl, -C₁₋₃ alkylene-C₃₋₇ cyclic hydrocarbyl, -C₁₋₃ alkylene-phenyl, -C₁₋₃ alkylene-(5- to 7-membered monocyclic heterocyclyl), -C₁₋₃ alkylene-(9- to 10-membered benzo-fused heterocyclyl), -C₁₋₃ alkylene-(5- to 6-membered heteroaryl), and -C₁₋₃ alkylene-(9- to 10-membered heteroaryl), each optionally substituted with 1, 2, 3, or more R¹³ groups.
12. The compound according to Embodiment 11, or a pharmaceutically acceptable salt, ester, stereoisomer, polymorph, solvate, N-oxide, isotopically labeled compound, metabolite, or prodrug thereof, wherein R¹³ is selected from the group consisting of C₁₋₄ alkyl-O-; halogen; and C₁₋₄ alkyl optionally substituted with 1, 2, or 3 substituents independently selected from halogen.
13. The compound according to Embodiment 11, or a pharmaceutically acceptable salt, ester, stereoisomer, polymorph, solvate, N-oxide, isotopically labeled compound, metabolite, or prodrug thereof, wherein X⁴ is C(=O).
14. The compound according to Embodiment 10, or a pharmaceutically acceptable salt, ester, stereoisomer, polymorph, solvate, N-oxide, isotopically labeled compound, metabolite, or prodrug thereof, wherein:
   R^{1a} is selected from the group consisting of:
      C₂₋₆ alkenyl and C₂₋₆ alkynyl, wherein the C₂₋₆ alkenyl and C₂₋₆ alkynyl are each substituted with one phenyl, 5- to 6-membered heteroaryl, or 9- to 10-membered heteroaryl, wherein the phenyl, 5- to 6-membered heteroaryl, and 9- to 10-membered heteroaryl are each optionally substituted with 1, 2, or 3 substituents independently selected from F, Cl, Br, I, and C₁₋₄ alkyl;
      phenyl optionally substituted with 1, 2, or 3 substituents independently selected from F, Cl, Br, I, and C₁₋₄ alkyl; and
      -C₁₋₃ alkylene-phenyl, -C₁₋₃ alkylene-(5- to 6-membered heteroaryl), and -C₁₋₃ alkylene-(9- to 10-membered heteroaryl), wherein the alkylene is, at each occurrence, optionally substituted with one - NR¹¹R¹², and the phenyl, 5- to 6-membered heteroaryl, and 9- to 10-membered heteroaryl are each optionally substituted with 1, 2, or 3 substituents independently selected from F, Cl, Br, I, and C₁₋₄ alkyl;
   R^{1b} is absent;
   X¹ is absent; and
   X⁴ is C(=O) or -O-C(=O)-.
15. The compound according to Embodiment 10, or a pharmaceutically acceptable salt, ester, stereoisomer, polymorph, solvate, N-oxide, isotopically labeled compound, metabolite, or prodrug thereof, wherein the C₁₋₆ alkyl is methyl, ethyl, propyl, isopropyl, or t-butyl.
16. The compound according to Embodiment 14, or a pharmaceutically acceptable salt, ester, stereoisomer, polymorph, solvate, N-oxide, isotopically labeled compound, metabolite, or prodrug thereof, wherein the C₂₋₆ alkenyl is ethenyl, 1-propenyl, or 2-propenyl.
17. The compound according to Embodiment 14, or a pharmaceutically acceptable salt, ester, stereoisomer, polymorph, solvate, N-oxide, isotopically labeled compound, metabolite, or prodrug thereof, wherein the C₂₋₆ alkynyl is ethynyl, 1-propynyl, or 2-propynyl.
18. The compound according to Embodiment 14, or a pharmaceutically acceptable salt, ester, stereoisomer, polymorph, solvate, N-oxide, isotopically labeled compound, metabolite, or prodrug thereof, wherein R¹³ is phenyl, pyridyl, indolyl or furanyl, each optionally substituted with 1, 2, or 3 substituents independently selected from F, Cl, Br, and methyl.
19. The compound according to Embodiment 18, or a pharmaceutically acceptable salt, ester, stereoisomer, polymorph, solvate, N-oxide, isotopically labeled compound, metabolite, or prodrug thereof, wherein R^{1a} is selected from the group consisting of: 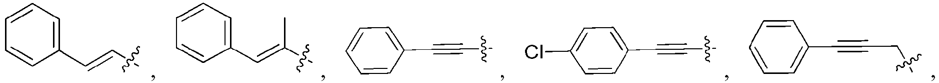 and 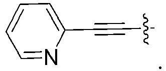
20. The compound according to Embodiment 1, or a pharmaceutically acceptable salt, ester, stereoisomer, polymorph, solvate, N-oxide, isotopically labeled compound, metabolite, or prodrug thereof, wherein:
   R^{1a} is selected from the group consisting of optionally substituted phenyl, -optionally substituted C₁₋₃ alkylene-(optionally substituted C₃₋₇ cyclic hydrocarbyl), -optionally substituted C₁₋₃ alkylene-(optionally substituted 5- to 7-membered monocyclic heterocyclyl), -optionally substituted C₁₋₃ alkylene-(optionally substituted 8- to 10-membered benzo-fused heterocyclyl), -optionally substituted C₁₋₃ alkylene-optionally substituted phenyl, and -optionally substituted C₁₋₃ alkylene-(optionally substituted 5- to 10-membered heteroaryl);
   R^{1b} is selected from the group consisting of H; C₁₋₈ alkyl optionally substituted with 1, 2, 3, or more R¹³ groups; saturated or partially unsaturated C₃₋₁₀ cyclic hydrocarbyl; C₆₋₁₀ aryl; -C₁₋₆ alkylene-saturated or partially unsaturated C₃₋₁₀ cyclic hydrocarbyl; and -C₁₋₆ alkylene-C₆₋₁₀ aryl;
   X¹ is CR¹⁰ or N;
   X⁴ is C(=O);
   wherein "optionally substituted" means substituted with 1, 2, 3, or more R¹³ groups; and
   R ¹³ is as defined in Embodiment 1.
21. The compound according to Embodiment 20, or a pharmaceutically acceptable salt, ester, stereoisomer, polymorph, solvate, N-oxide, isotopically labeled compound, metabolite, or prodrug thereof, wherein R^{1b} is selected from the group consisting of H, optionally substituted C₁₋₄ alkyl, optionally substituted C₃₋₇ cyclic hydrocarbyl, optionally substituted phenyl, -optionally substituted C₁₋₃ alkylene-(optionally substituted C₃₋₇ cyclic hydrocarbyl), and -optionally substituted C₁₋₃ alkylene-optionally substituted phenyl;
   wherein "optionally substituted" means substituted with 1, 2, 3, or more R¹³ groups.
22. The compound according to Embodiment 1, or a pharmaceutically acceptable salt, ester, stereoisomer, polymorph, solvate, N-oxide, isotopically labeled compound, metabolite, or prodrug thereof, wherein:
   R^{2a} is optionally substituted phenyl; and/or
   R^{2b} is optionally substituted phenyl;
   wherein "optionally substituted" means substituted with 1, 2, 3, or more R¹³ groups.
23. The compound according to Embodiment 1, or a pharmaceutically acceptable salt, ester, stereoisomer, polymorph, solvate, N-oxide, isotopically labeled compound, metabolite, or prodrug thereof, wherein the compound has the structure of Formula (II): 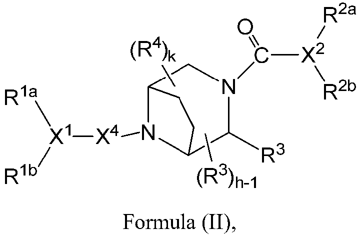 wherein R^{1a}, R^{1b}, X¹, X⁴, R^{2a}, R^{2b}, X², R³, R⁴, h, and k are as defined in Embodiment 1.
24. The compound according to Embodiment 1, or a pharmaceutically acceptable salt, ester, stereoisomer, polymorph, solvate, N-oxide, isotopically labeled compound, metabolite, or prodrug thereof, wherein 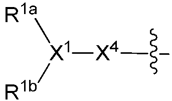 is: 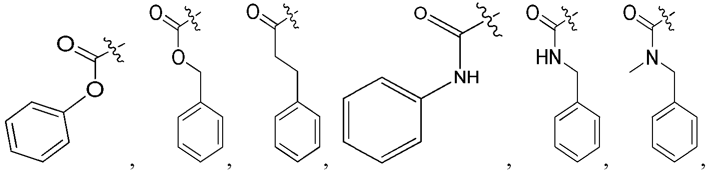 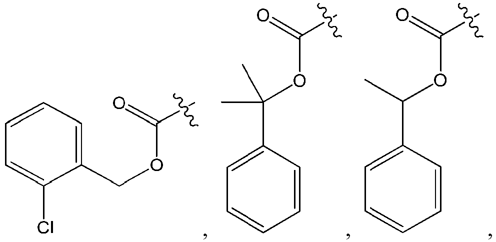 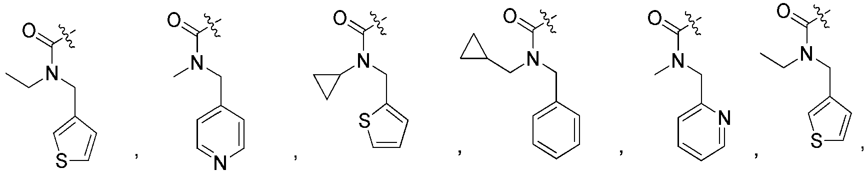 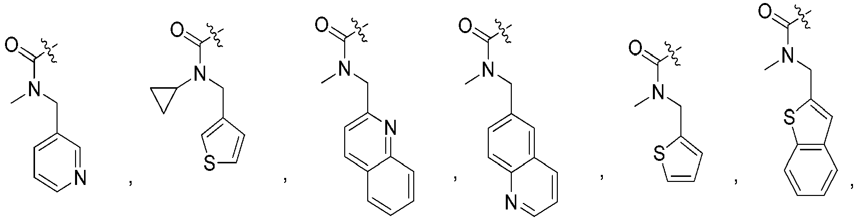 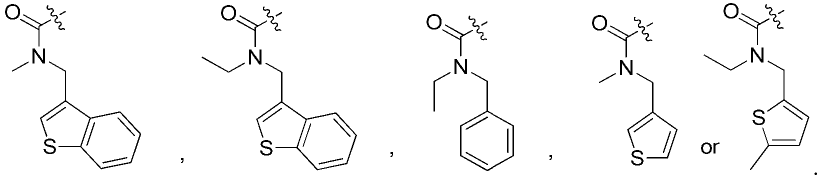
25. The compound according to Embodiment 1, or a pharmaceutically acceptable salt, ester, stereoisomer, polymorph, solvate, N-oxide, isotopically labeled compound, metabolite, or prodrug thereof, wherein 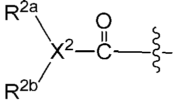 is: 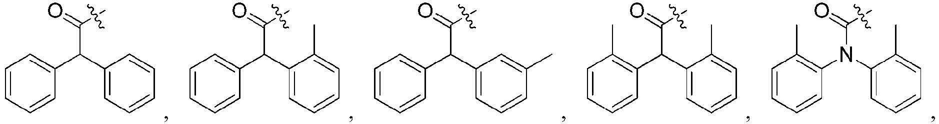 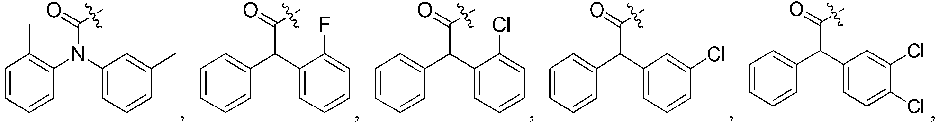 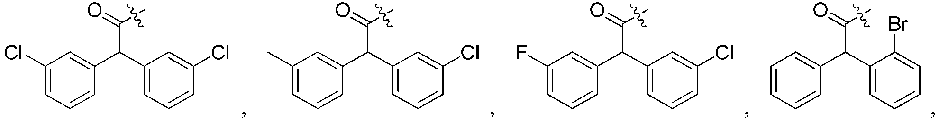 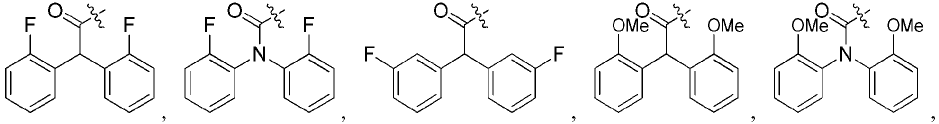 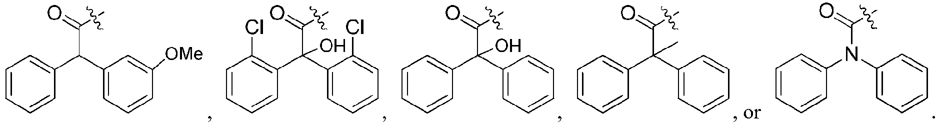
26. The compound according to Embodiment 1, or a pharmaceutically acceptable salt, ester, stereoisomer, polymorph, solvate, N-oxide, isotopically labeled compound, metabolite, or prodrug thereof, wherein R¹⁰ is H or methyl.
27. The compound according to Embodiment 20, or a pharmaceutically acceptable salt, ester, stereoisomer, polymorph, solvate, N-oxide, isotopically labeled compound, metabolite, or prodrug thereof, wherein R¹² is selected from the group consisting of optionally substituted phenyl, -C₁₋₃ alkylene-(optionally substituted C₃₋₇ cyclic hydrocarbyl), -C₁₋₃ alkylene-(optionally substituted 5- to 7-membered monocyclic heterocyclyl), -C₁₋₃ alkylene-(optionally substituted 8- to 10-membered benzo-fused heterocyclyl), -C₁₋₃ alkylene-optionally substituted phenyl, and -C₁₋₃ alkylene-(optionally substituted 5- to 10-membered heteroaryl).
28. The compound according to Embodiment 20, or a pharmaceutically acceptable salt, ester, stereoisomer, polymorph, solvate, N-oxide, isotopically labeled compound, metabolite, or prodrug thereof, wherein R^{1a} is selected from the group consisting of:
   optionally substituted phenyl;
   -C₁₋₃ alkylene-(optionally substituted C₃₋₇ cyclic hydrocarbyl), wherein the cyclic hydrocarbyl is cyclopropyl, cyclobutyl, cyclopentyl, or cyclohexyl;
   -C₁₋₃ alkylene-(optionally substituted 8- to 10-membered benzo-fused heterocyclyl), wherein the heterocyclyl is 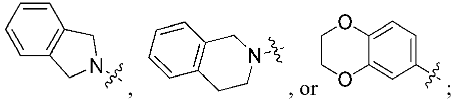
   -C₁₋₃ alkylene-(optionally substituted 5- to 10-membered heteroaryl), wherein the heteroaryl is 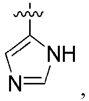 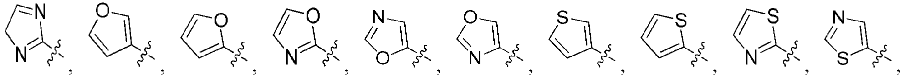 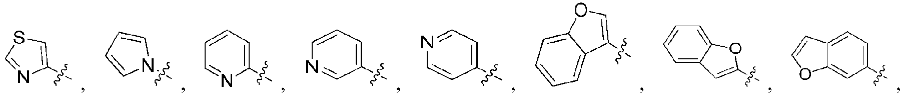 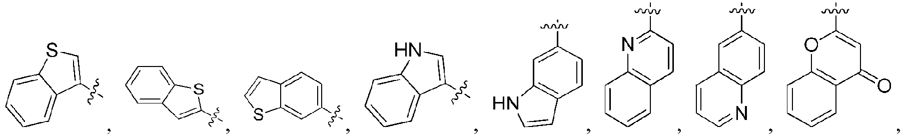 or 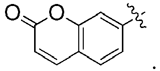
29. The compound according to Embodiment 20, or a pharmaceutically acceptable salt, ester, stereoisomer, polymorph, solvate, N-oxide, isotopically labeled compound, metabolite, or prodrug thereof, wherein R¹³ is selected from the group consisting of: halogen; -OR¹¹ wherein R¹¹ is C₁₋₆ alkyl optionally substituted with 1, 2, 3, or more halogens; cyano; C₃₋₇ cyclic hydrocarbyl; C₁₋₄ alkyl, C₂₋₄ alkenyl, and C₂₋₄ alkynyl, each optionally substituted with 1, 2, 3, or more halogens; -NR¹¹R¹² wherein R¹¹ and R¹² are each independently selected from the group consisting of H and C₁₋₄ alkyl; and -P(O)R¹¹R¹² wherein R¹¹ and R¹² are each independently C₁₋₆ alkyl optionally substituted with 1, 2, 3, or more halogens.
30. The compound according to Embodiment 20, or a pharmaceutically acceptable salt, ester, stereoisomer, polymorph, solvate, N-oxide, isotopically labeled compound, metabolite, or prodrug thereof, wherein R¹³ is selected from the group consisting of: halogen; -OR¹¹ wherein R¹¹ is C₁₋₃ alkyl optionally substituted with 1, 2, or 3 F or Cl; cyano; C₃₋₇ cyclic hydrocarbyl; C₁₋₄ alkyl, C₂₋₄ alkenyl, and C₂₋₄ alkynyl, each optionally substituted with 1, 2, 3, or more halogens; -NR¹¹R¹² wherein R¹¹ and R¹² are each independently selected from the group consisting of H and methyl; and -P(O)R¹¹R¹² wherein R¹¹ and R¹² are each independently C₁₋₃ alkyl optionally substituted with 1, 2, or 3 F or Cl.
31. The compound according to Embodiment 20, or a pharmaceutically acceptable salt, ester, stereoisomer, polymorph, solvate, N-oxide, isotopically labeled compound, metabolite, or prodrug thereof, wherein R¹³ is selected from the group consisting of: F, Cl, Br, OH, -OC₁₋₄ alkyl, -N(C₁₋₄ alkyl)₂, cyano, C₃₋₇ cyclic hydrocarbyl, C₂₋₄ alkenyl, and C₂₋₄ alkynyl; C₁₋₄ alkyl optionally substituted with 1, 2, 3, or more F, Cl, or Br; and -P(O)R¹¹R¹² wherein R¹¹ and R¹² are each independently methyl, ethyl, propyl, or isopropyl.
32. The compound according to Embodiment 20, or a pharmaceutically acceptable salt, ester, stereoisomer, polymorph, solvate, N-oxide, isotopically labeled compound, metabolite, or prodrug thereof, wherein R¹³ is selected from the group consisting of: F, Cl, Br, -OCH₃, -N(CH₃)₂, cyano, cyclopropyl, ethenyl, 1-propenyl, 2-propenyl, ethynyl, 1-propynyl, 2-propynyl, methyl, ethyl, n-propyl, isopropyl, tert-butyl, and CF₃; and - P(O)R¹¹R¹² wherein R¹¹ and R¹² are each independently methyl.
33. The compound according to Embodiment 20, or a pharmaceutically acceptable salt, ester, stereoisomer, polymorph, solvate, N-oxide, isotopically labeled compound, metabolite, or prodrug thereof, wherein R¹² is selected from the group consisting of: 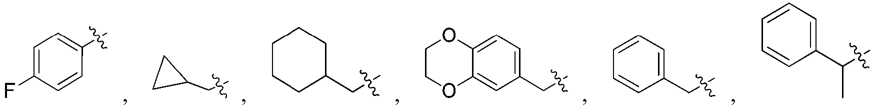 (including 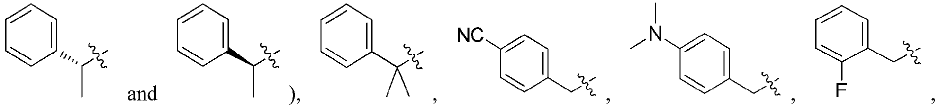 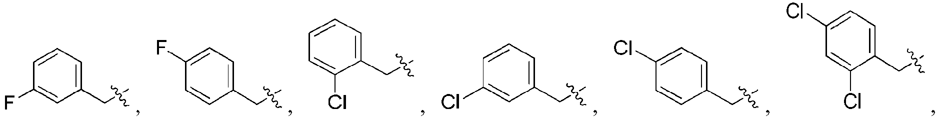 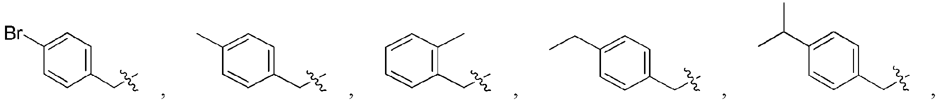 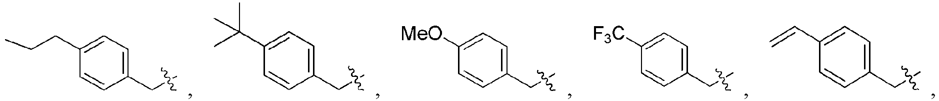 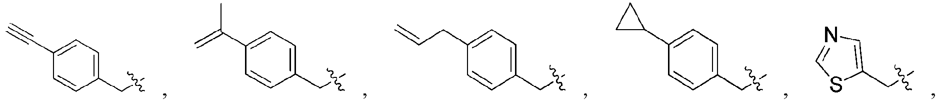 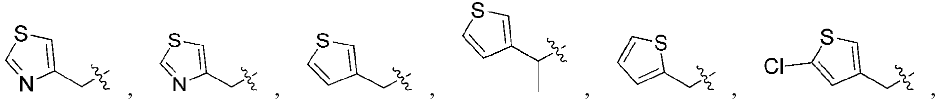 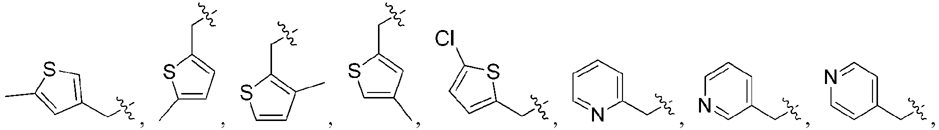 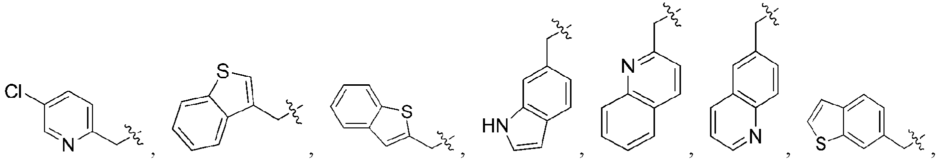 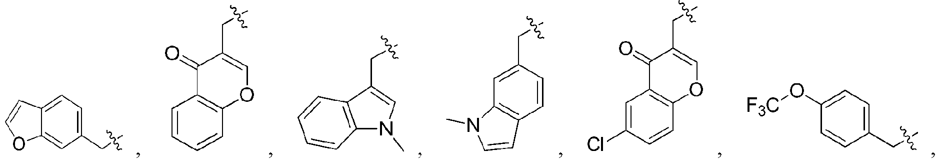 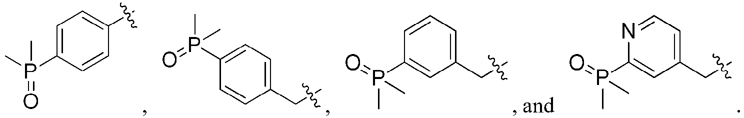
34. The compound according to Embodiment 21, or a pharmaceutically acceptable salt, ester, stereoisomer, polymorph, solvate, N-oxide, isotopically labeled compound, metabolite, or prodrug thereof, wherein R^{1b} is selected from the group consisting of: H, optionally substituted C₁₋₄ alkyl, optionally substituted C₃₋₇ cyclic hydrocarbyl, optionally substituted phenyl, -C₁₋₃ alkylene-(optionally substituted C₃₋₇ cyclic hydrocarbyl), and - C₁₋₃ alkylene-optionally substituted phenyl.
35. The compound according to Embodiment 21, or a pharmaceutically acceptable salt, ester, stereoisomer, polymorph, solvate, N-oxide, isotopically labeled compound, metabolite, or prodrug thereof, wherein R^{1b} is selected from the group consisting of:
   H, phenyl;
   optionally substituted C₁₋₄ alkyl, wherein the alkyl is methyl, ethyl, or isopropyl;
   optionally substituted C₃₋₇ cyclic hydrocarbyl and -C₁₋₃ alkylene-(C₃₋₇ cyclic hydrocarbyl), wherein the cyclic hydrocarbyl is cyclopropyl, cyclobutyl, cyclopentyl, or cyclohexyl; and
   -C₁₋₃ alkylene-phenyl.
36. The compound according to Embodiment 21, or a pharmaceutically acceptable salt, ester, stereoisomer, polymorph, solvate, N-oxide, isotopically labeled compound, metabolite, or prodrug thereof, wherein R¹³ is selected from the group consisting of halogen and C₁₋₄ alkyl.
37. The compound according to Embodiment 21, or a pharmaceutically acceptable salt, ester, stereoisomer, polymorph, solvate, N-oxide, isotopically labeled compound, metabolite, or prodrug thereof, wherein R¹³ is selected from the group consisting of F, Cl, Br, and methyl.
38. The compound according to Embodiment 21, or a pharmaceutically acceptable salt, ester, stereoisomer, polymorph, solvate, N-oxide, isotopically labeled compound, metabolite, or prodrug thereof, wherein R^{1b} is selected from the group consisting of H, methyl, ethyl, isopropyl, CF₃CH₂, cyclopropyl, 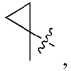 phenyl, 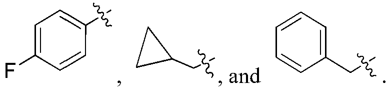
39. The compound according to Embodiment 22, or a pharmaceutically acceptable salt, ester, stereoisomer, polymorph, solvate, N-oxide, isotopically labeled compound, metabolite, or prodrug thereof, wherein R¹³ is selected from the group consisting of halogen and -OR¹¹, and wherein R¹¹ is selected from the group consisting of C₁₋₄ alkyl.
40. The compound according to Embodiment 22, or a pharmaceutically acceptable salt, ester, stereoisomer, polymorph, solvate, N-oxide, isotopically labeled compound, metabolite, or prodrug thereof, wherein R¹³ is selected from the group consisting of F, Cl, Br, and -OCH₃.
41. The compound according to Embodiment 22, or a pharmaceutically acceptable salt, ester, stereoisomer, polymorph, solvate, N-oxide, isotopically labeled compound, metabolite, or prodrug thereof, wherein R^{2a} and R^{2b} are each selected from the group consisting of phenyl, 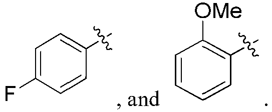
42. The compound according to Embodiment 1, or a pharmaceutically acceptable salt, ester, stereoisomer, polymorph, solvate, N-oxide, isotopically labeled compound, metabolite, or prodrug thereof, wherein:
   U is ethylene;
   R^{1a} is selected from the group consisting of: -C₁₋₆ alkylene-C₆₋₁₀ aryl; and -C₁₋₆ alkylene-(5- to 14-membered heteroaryl);
   R^{1b} is selected from the group consisting of: C₁₋₈ alkyl; saturated C₃₋₁₀ cyclic hydrocarbyl; and -C₁₋₆ alkylene-saturated C₃₋₁₀ cyclic hydrocarbyl;
   X¹ is N; X⁴ is C(=O); X² is N; R^{2a} and R^{2b} are both phenyl;
   R³ is -C(=O)OH;
   R⁴ is H;
   h and k are each independently 1; and
   the cyclic hydrocarbyl, aryl and heteroaryl groups recited above are, at each occurrence, optionally substituted with one R¹³ group; said R¹³ is C₁₋₆ alkyl;
   preferably, the C₆₋₁₀ aryl is phenyl, the saturated C₃₋₁₀ cyclic hydrocarbyl is cyclopropyl, and the 5- to 14-membered heteroaryl is thienyl or benzothienyl.
43. The compound according to Embodiment 1, or a pharmaceutically acceptable salt, ester, stereoisomer, polymorph, solvate, N-oxide, isotopically labeled compound, metabolite, or prodrug thereof, wherein the compound is selected from the group consisting of:

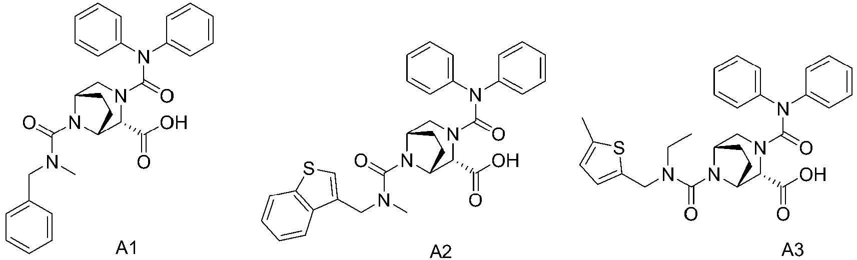

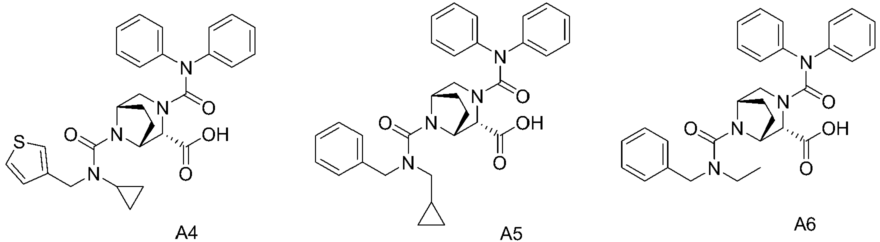

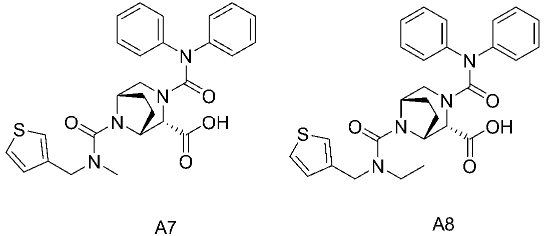

The correspondence between the above-described Compounds A1 to A8 and the Compounds in the Examples set forth below is as follows: A1 = C112, A2 = C246, A3 = C202, A4 = C196, A5 = C154, A6 = C155, A7 = C184, A8 = C207.

In a second aspect, the present application provides the following embodiments relating to the use of the compound according to the first aspect in the manufacture of a medicament for treating a disease.
44. Use of the compound according to any one of Embodiments 1 to 43, or a pharmaceutically acceptable salt, ester, stereoisomer, polymorph, solvate, N-oxide, isotopically labeled compound, metabolite, or prodrug thereof, in the manufacture of a medicament for preventing or treating peripheral neuropathic pain.
45. Use of the compound according to any one of Embodiments 1 to 43, or a pharmaceutically acceptable salt, ester, stereoisomer, polymorph, solvate, N-oxide, isotopically labeled compound, metabolite, or prodrug thereof, in the manufacture of a medicament for preventing or treating macrophage-associated peripheral neuropathic pain.
46. The use according to Embodiment 44 or 45, wherein the medicament is for preventing or treating peripheral neuropathic pain associated with the recruitment/accumulation of macrophages.
47. The use according to Embodiment 44 or 45, wherein the medicament is for preventing or treating peripheral neuropathic pain associated with high expression of AT2 receptors in macrophages.
48. The use according to Embodiment 44 or 45, wherein the medicament is for preventing or treating peripheral neuropathic pain associated with elevated levels of reactive oxygen/nitrogen species generated by macrophages.
49. The use according to any one of Embodiments 44 to 48, wherein the medicament is for preventing or treating peripheral neuropathic pain caused by secondary neuropathy.
50. The use according to Embodiment 49, wherein the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).
51. The use according to any one of Embodiments 44 to 50, wherein the medicament is for preventing or treating a disorder selected from the group consisting of trigeminal neuralgia, chronic neuropathic pain following peripheral nerve injury, painful polyneuropathy, postherpetic neuralgia, and painful radiculopathy, or symptoms associated therewith.
51a. The use according to any one of Embodiments 44 to 50, wherein the medicament is for preventing or treating a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.
52. The use according to any one of Embodiments 44 to 50, wherein the medicament is for preventing or treating a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.
52a. The use according to any one of Embodiments 44 to 50, wherein the medicament is for preventing or treating a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (including chronic post-surgical neuralgia), chemotherapy-associated neuropathic pain, and HIV (Human Immunodeficiency Virus) painful sensory neuropathy, or symptoms associated therewith.

In a third aspect, the present application provides the following embodiments relating to a method for treating a disease, comprising administering to a patient in need thereof a therapeutically effective amount of the compound according to the first aspect.
53. A method for preventing or treating peripheral neuropathic pain, comprising administering to a patient in need thereof a therapeutically effective amount of the compound according to any one of Embodiments 1 to 43, or a pharmaceutically acceptable salt, ester, stereoisomer, polymorph, solvate, N-oxide, isotopically labeled compound, metabolite, or prodrug thereof.
54. A method for preventing or treating macrophage-associated peripheral neuropathic pain, comprising administering to a patient in need thereof a therapeutically effective amount of the compound according to any one of Embodiments 1 to 43, or a pharmaceutically acceptable salt, ester, stereoisomer, polymorph, solvate, N-oxide, isotopically labeled compound, metabolite, or prodrug thereof.
55. The method according to Embodiment 53 or 54, wherein the disease is peripheral neuropathic pain associated with the recruitment/accumulation of macrophages.
56. The method according to Embodiment 53 or 54, wherein the disease is peripheral neuropathic pain associated with high expression of AT₂ receptors in macrophages.
57. The method according to Embodiment 53 or 54, wherein the disease is peripheral neuropathic pain associated with elevated levels of reactive oxygen/nitrogen species generated by macrophages.
58. The method according to any one of Embodiments 53 to 57, wherein the disease is peripheral neuropathic pain caused by secondary neuropathy.
59. The method according to Embodiment 58, wherein the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).
60. The method according to any one of Embodiments 53 to 59, wherein the disease is a disorder selected from the group consisting of trigeminal neuralgia, chronic neuropathic pain following peripheral nerve injury, painful polyneuropathy, postherpetic neuralgia, and painful radiculopathy, or symptoms associated therewith.
60a. The method according to any one of Embodiments 53 to 59, wherein the disease is a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.
61. The method according to any one of Embodiments 53 to 59, wherein the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.
61a. The method according to any one of Embodiments 53 to 59, wherein the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (including chronic post-surgical neuralgia), chemotherapy-associated neuropathic pain, and HIV (Human Immunodeficiency Virus) painful sensory neuropathy, or symptoms associated therewith.

In a fourth aspect, the present application provides the following embodiments relating to the compound according to the first aspect for use in the treatment of a disease.
62. The compound according to any one of Embodiments 1 to 43, or a pharmaceutically acceptable salt, ester, stereoisomer, polymorph, solvate, N-oxide, isotopically labeled compound, metabolite, or prodrug thereof, for use in the prevention or treatment of peripheral neuropathic pain.
63. The compound according to any one of Embodiments 1 to 43, or a pharmaceutically acceptable salt, ester, stereoisomer, polymorph, solvate, N-oxide, isotopically labeled compound, metabolite, or prodrug thereof, for use in the prevention or treatment of macrophage-associated peripheral neuropathic pain.
64. The compound according to Embodiment 62 or 63, or a pharmaceutically acceptable salt, ester, stereoisomer, polymorph, solvate, N-oxide, isotopically labeled compound, metabolite, or prodrug thereof, for use in the prevention or treatment of peripheral neuropathic pain associated with the recruitment/accumulation of macrophages.
65. The compound according to Embodiment 62 or 63, or a pharmaceutically acceptable salt, ester, stereoisomer, polymorph, solvate, N-oxide, isotopically labeled compound, metabolite, or prodrug thereof, for use in the prevention or treatment of peripheral neuropathic pain associated with high expression of AT2 receptors in macrophages.
66. The compound according to Embodiment 62 or 63, or a pharmaceutically acceptable salt, ester, stereoisomer, polymorph, solvate, N-oxide, isotopically labeled compound, metabolite, or prodrug thereof, for use in the prevention or treatment of peripheral neuropathic pain associated with elevated levels of reactive oxygen/nitrogen species generated by macrophages.
67. The compound according to any one of Embodiments 62 to 66, or a pharmaceutically acceptable salt, ester, stereoisomer, polymorph, solvate, N-oxide, isotopically labeled compound, metabolite, or prodrug thereof, for use in the prevention or treatment of peripheral neuropathic pain caused by secondary neuropathy.
68. The compound according to Embodiment 67, or a pharmaceutically acceptable salt, ester, stereoisomer, polymorph, solvate, N-oxide, isotopically labeled compound, metabolite, or prodrug thereof, wherein the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).
69. The compound according to any one of Embodiments 62 to 68, or a pharmaceutically acceptable salt, ester, stereoisomer, polymorph, solvate, N-oxide, isotopically labeled compound, metabolite, or prodrug thereof, for use in the prevention or treatment of a disorder selected from the group consisting of trigeminal neuralgia, chronic neuropathic pain following peripheral nerve injury, painful polyneuropathy, postherpetic neuralgia, and painful radiculopathy, or symptoms associated therewith.
69a. The compound according to any one of Embodiments 62 to 68, or a pharmaceutically acceptable salt, ester, stereoisomer, polymorph, solvate, N-oxide, isotopically labeled compound, metabolite, or prodrug thereof, for use in the prevention or treatment of a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.
70. The compound according to any one of Embodiments 62 to 68, or a pharmaceutically acceptable salt, ester, stereoisomer, polymorph, solvate, N-oxide, isotopically labeled compound, metabolite, or prodrug thereof, for use in the prevention or treatment of a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.
70a. The compound according to any one of Embodiments 62 to 68, or a pharmaceutically acceptable salt, ester, stereoisomer, polymorph, solvate, N-oxide, isotopically labeled compound, metabolite, or prodrug thereof, for use in the prevention or treatment of a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (including chronic post-surgical neuralgia), chemotherapy-associated neuropathic pain, and HIV (Human Immunodeficiency Virus) painful sensory neuropathy, or symptoms associated therewith.

### Detailed Description of the Invention

### Definitions

Unless defined otherwise below, all technical and scientific terms used herein are intended to have the same meaning as those commonly understood by those skilled in the art. Reference to technology used herein is intended to refer to technology commonly understood in the art, including variations of such technologies that would be obvious to those skilled in the art, or substitutions with equivalent techniques. Although the following terms are believed to be well understood by those skilled in the art, the following definitions are set forth to better explain the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a linear or branched saturated aliphatic hydrocarbon. In some embodiments, alkyl has 1-12, particularly 1-8 ("C₁₋₈ alkyl") carbon atoms, e.g., 1-6 ("C₁₋₆ alkyl"), 1-4 ("C₁₋₄ alkyl") carbon atoms, more particularly, 1, 2, 3, 4, 5, 6, 7 or 8 carbon atoms. For example, as used herein, the term "C₁₋₈ alkyl" refers to a linear or branched group having 1-8 carbon atoms (such as methyl, ethyl, n-propyl, isopropyl, n-butyl, isobutyl, sec-butyl, tert-butyl, n-pentyl, isopentyl, neopentyl, or 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl), which is optionally substituted with one or more (e.g., 1 to 3) suitable substituents such as halogen (in which case the group may be referred to as "halogenated alkyl") (e.g., CH₂F, CHF₂, CF₃, CCl₃, C₂F₅, C₂Cl₅, CH₂CF₃, CH₂Cl or -CH₂CH₂CF₃, *etc.).* The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or tert-butyl).

As used herein, the term "alkenyl" refers to a linear or branched monovalent hydrocarbyl having a double bond and 2-8 carbon atoms ("C₂₋₈ alkenyl", such as "C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, 4-methyl-3-pentenyl, heptenyl and octenyl. When the compound of the present invention contains an alkenylene group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5, 6, 7 or 8 carbon atoms, e.g., ethynyl, 1-propynyl, 2-propynyl, 2-butynyl, 3-butynyl, 2-pentynyl, 3-pentynyl, 4-pentynyl, 2-hexynyl, 3-hexynyl.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6, such as 5-6 or 5-7) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the terms "heterocyclyl", "heterocyclylene" and "heterocycle" refer to a saturated (i.e., heterocycloalkyl) or partially unsaturated (i.e., having one or more double and/or triple bonds in the ring) monocyclic or bicyclic group having e.g. 3-10 (suitably having 3-8, and more suitably having 3-6; or suitably having 8-10, and more suitably having 9 or 10) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated monocyclic or bicyclic heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of monocyclic heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Other examples of monocyclic heterocycle and heterocyclyl include but are not limited to: tetrahydrofuranyl, tetrahydrothienyl, pyrrolidinyl (e.g. pyrrolidin-1-yl), oxazolidinyl, thiazolidinyl, imidazolidinyl, 1,3-dioxolanyl, 1,3-oxathiolanyl, piperidinyl, piperazinyl, morpholinyl (such as morpholino)), thiomorpholinyl, tetrahydro-2H-pyranyl, tetrahydro-2H-thiopyranyl, 1,3-oxazinanyl (1,3-oxazinane), 1,3-thiazinanyl (1,3-thiazinane), hexahydropyrimidyl, 1,3-oxathianyl (1,3-oxathiane), 1,4-oxathianyl (1,4-oxathiane), 1,3-diazepanyl (1,3-diazepane), 1,4-diazepanyl (1,4-diazepane), 1,3-oxazepanyl (1,3-oxazepane), 1,3-thiazepanyl (1,3-thiazepane). Bicyclic heterocyclylene, heterocyclyl and heterocycle include spiro ring systems, fused (e.g., benzo-fused) systems, or bridged systems. The benzo-fused heterocyclylene, heterocyclyl and heterocycle refer to the above-mentioned monocyclic heterocyclylene, heterocyclyl and heterocycle fused to benzene, for example, a benzo derivative of a saturated or partially unsaturated monocyclic group with 3-6 (suitably with 4-6, more suitably 5-6) ring atoms, in which 1, 2, 3 or 4 ring atoms are heteroatoms selected from N, O and S and the remaining ring atoms are C (i.e., "7- to 10-membered benzo fused heterocyclylene, heterocyclyl and heterocycle"), including, for example, 2,3-dihydrobenzofuranyl(ene) 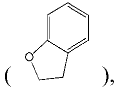 1,3-dihydroisobenzofuranyl(ene) 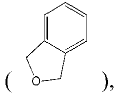 2,3-dihydrobenzo [c]thienyl(ene) 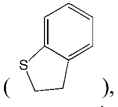 1,3-dihydrobenzo[c]thienyl(ene) 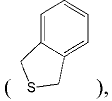 dihydroindolyl(ene) 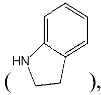 dihydroisoindolyl(ene) 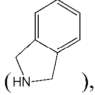 benzo [d][1,3]dioxolyl(ene) 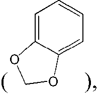 benzo[d][1,3]dithiolyl(ene) 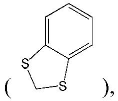 benzo[d][1,3]oxathiolyl(ene) 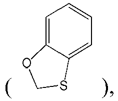 3*H*-benzo[c][1,2]oxathiolyl(ene) 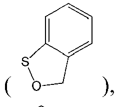 *3H-*benzo[d][1,2]oxathiolyl(ene) 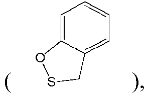 2,3-dihydrobenzo[d]oxazolyl(ene) 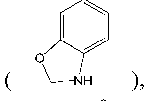 2,3-dihydrobenzo [d]thiazolyl(ene) 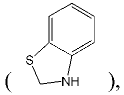 2,3-dihydro-1H-benzo[d]imidazolyl(ene) 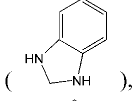 2,3-dihydrobenzo[d]isoxazolyl(ene) 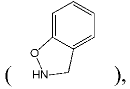 2,3-dihydrobenzo [d]isothiazolyl(ene) 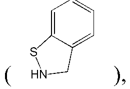 1,3-dihydrobenzo [c]isoxazolyl(ene) 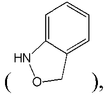 1,3-dihydrobenzo [c]isothiazolyl(ene) 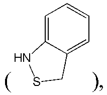 2,3-dihydro-1H-indazolyl(ene) 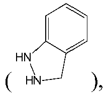 chromanyl(ene) 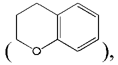 2H-chromenyl(ene) 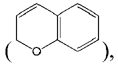 4H-chromenyl(ene) 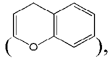 dihydrobenzothiopyranyl(ene) 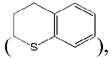 2H-thiochromenyl(ene) (2H-thiochromene, 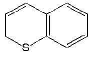 ), 4H-benzothiopyranyl(ene) (4H-thiochromene, 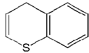 ), 1,2,3,4,4a,8a-hexahydroquinolinyl(ene) 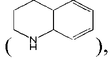 1,2,4a,8a-tetrahydroquinolinyl(ene) 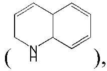 1,4,4a,8a-tetrahydroquinolinyl(ene) 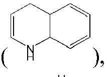 1,2,3,4,4a,8a-hexahydroisoquinolinyl(ene) 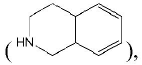 1,2,3,4,4a,8a-hexahydroquinoxalinyl(ene) 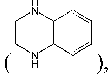 1,4,4a,8a-tetrahydroquinoxalinyl(ene) 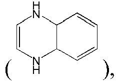 1,2,3,4,4a,8a-hexahydroquinazolinyl (ene) 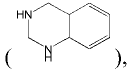 2,4,4a,8a-tetrahydro-1H-benzo[d][1,3]oxazinyl(ene) 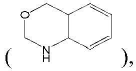 3,4,4a,8a-tetrahydro-2H-benzo[b][1,4]oxazinyl(ene) 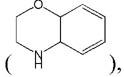 3,4,4a,8a-tetrahydro-2H-benzo[e][1,3]oxazinyl(ene) 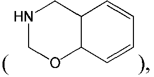 2,4,4a,8a-tetrahydro-1H-benzo[d][1,3]thiazinyl(ene) 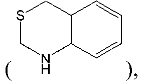 3,4,4a,8a-tetrahydro-2H-benzo[b][1,4]thiazinyl(ene) 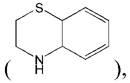 3,4,4a,8a-tetrahydro-2H-benzo[e][1,3]thiazinyl(ene) 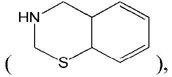 and 2,3-dihydrobenzo[b][1,4]dioxinyl 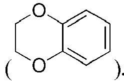 The bridged systems also include for example 8-azaspiro[4.5]decane, 3,9-diazaspiro[5.5]undecane, 2-azabicyclo[2.2.2]octane. Heterocyclylene, heterocyclyl and heterocycle may optionally be substituted with one or more (e.g. 1, 2, 3 or 4) suitable substituents.

As used herein, the terms "aryl(ene)" and "aromatic ring" refer to an all-carbon monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc.).*

As used herein, the terms "heteroaryl(ene)" and "heteroaromatic ring" refer to a monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same or different. Moreover, in each case, it can be benzo-fused. In particular, "heteroaryl(ene)" or "heteroaromatic ring" is selected from the group consisting of thienyl(ene), furyl(ene), pyrrolyl(ene), oxazolyl(ene), thiazolyl(ene), imidazolyl(ene), pyrazolyl(ene) (e.g., 1-pyrazolyl, 3-pyrazolyl, 4-pyrazolyl and 5-pyrazolyl), isoxazolyl(ene), isothiazolyl(ene), oxadiazolyl(ene), triazolyl(ene), tetrazolyl(ene) (e.g. 1-tetrazolyl or 5-tetrazolyl), thiadiazolyl(ene) *etc.,* and benzo derivatives thereof; or pyridinyl(ene), pyridazinyl(ene), pyrimidinyl(ene), pyrazinyl(ene), triazinyl(ene), etc., and benzo derivatives thereof. Other examples of "heteroaryl(ene)" or "heteroaromatic ring" also include pyrrolopyrimidinyl, pyrrolopyridyl, pyrazolopyrimidinyl, pyrazolopyridyl, imidazopyridyl, purinyl, and the like.

As used herein, the term "aralkyl" preferably means alkyl substituted with aryl or heteroaryl, wherein the aryl, heteroaryl and alkyl are as defined herein. Normally, the aryl group may have 6-14 carbon atoms, the heteroaryl group may have 5-14 ring atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may optionally further comprise one or more (e.g., one, two, three or four) ring members selected from the group consisting of N, O, C=O, S, S=O and S(=O)₂. The nitrogen containing heterocycle is attached to the rest of the molecule through the nitrogen atom and any other ring atom in said nitrogen containing heterocycle. The nitrogen containing heterocycle is optionally benzo-fused, and is preferably attached to the rest of the molecule through the nitrogen atom in said nitrogen containing heterocycle and any carbon atom in the fused benzene ring.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted", the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more from a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be at any suitable position of the substituent.

When a bond to a substituent is shown to cross a bond connecting two atoms in a ring, then such substituent may be bonded to any of the ring-forming atoms in that ring that are substitutable, including the available atoms in the bridge when the substitutable ring is a bridged ring.

The present invention also includes all pharmaceutically acceptable isotopically labeled compounds, which are identical to those of the present invention except that one or more atoms are replaced with an atom having the same atomic number, but having an atomic mass or mass number different from the atomic mass or mass number which predominates in nature. Examples of isotopes suitable for inclusion in the compound of the present invention include, but are not limited to, isotopes of hydrogen, such as ²H, ³H; isotopes of carbon, such as ¹¹C, ¹³C, and ¹⁴C; isotopes of chlorine, such as ³⁶Cl; isotopes of fluorine, such as ¹⁸F; isotopes of iodine, such as ¹²³I and ¹²⁵I; isotopes of nitrogen, such as ¹³N and ¹⁵N; isotopes of oxygen, such as ¹⁵O, ¹⁷O, and ¹⁸O; isotopes of phosphorus, such as ³²P; and isotopes of sulfur, such as ³⁵S. Certain isotopically labeled compounds of the present invention, for example those incorporating a radioactive isotope, are useful in drug and/or substrate tissue distribution studies (e.g., assays). The radioactive isotopes tritium (i.e., ³H) and carbon-14 (i.e., ¹⁴C), are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆,* or DMSO-*d₆*.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The carbon-carbon bonds of the compound of the present invention may be depicted herein using a solid line (-), a solid wedge ( 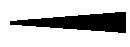 ), or a dotted wedge ( 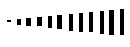 ). The use of a solid line to depict bonds to asymmetric carbon atoms is meant to indicate that all possible stereoisomers (e.g., specific enantiomers, racemic mixtures, *etc.)* at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free from, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite or prodrug, which can directly or indirectly generate the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol, for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-chloroperbenzoic acid (MCPBA), hydrogen peroxide, alkyl hydroperoxides such as tert-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems", Vol. 14, ACS Symposium Series (T. Higuchi and V. Stella). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in T.W. Greene & P.G.M. Wuts, Protective Groups in Organic Synthesis, John Wiley & Sons, 1991, which is incorporated herein by reference. The protecting groups may be removed at an appropriate subsequent stage using methods known in the art.

The term "about" refers to a range within +10%, preferably within +5%, and more preferably within +2% of the specified value.

### Pharmaceutical Compositions and Therapeutic Methods

In some embodiments, the present invention provides a pharmaceutical composition comprising a prophylactically or therapeutically effective amount of a compound of the present invention as described above, or a pharmaceutically acceptable salt, ester, stereoisomer, polymorph, solvate, N-oxide, isotopically labeled compound, metabolite, or prodrug thereof, and one or more pharmaceutically acceptable carriers. The pharmaceutical composition is preferably a solid formulation, semi-solid formulation, liquid formulation, or gaseous formulation. In some embodiments, the pharmaceutical composition may further comprise one or more additional therapeutic agents.

In some embodiments, the present invention provides the use of the compound of the present invention as described above, or a pharmaceutically acceptable salt, ester, stereoisomer, polymorph, solvate, N-oxide, isotopically labeled compound, metabolite, or prodrug thereof, or the pharmaceutical composition of the present invention, in the manufacture of a medicament for use as an angiotensin II type 2 (AT₂) receptor antagonist.

In some embodiments, the present invention provides the compound of the present invention as described above, or a pharmaceutically acceptable salt, ester, stereoisomer, polymorph, solvate, N-oxide, isotopically labeled compound, metabolite, or prodrug thereof, or the pharmaceutical composition of the present invention, for use as an angiotensin II type 2 (AT₂) receptor antagonist.

In some embodiments, the present invention provides a method for preventing or treating an AT₂ receptor-mediated disorder or symptoms associated therewith, comprising administering to an individual in need thereof an effective amount of the compound of the present invention as described above, or a pharmaceutically acceptable salt, ester, stereoisomer, polymorph, solvate, N-oxide, isotopically labeled compound, metabolite, or prodrug thereof, or the pharmaceutical composition of the present invention.

In some embodiments, the disease is peripheral neuropathic pain.

In some embodiments, the disease is peripheral neuropathic pain associated with the recruitment/accumulation of macrophages.

In some embodiments, the disease is peripheral neuropathic pain associated with high expression of AT2 receptors in macrophages.

In some embodiments, the disease is peripheral neuropathic pain associated with elevated levels of reactive oxygen/nitrogen species generated by macrophages.

In some embodiments, the disease is peripheral neuropathic pain caused by secondary neuropathy.

In some embodiments, the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).

In some embodiments, the disease is a disorder selected from the group consisting of trigeminal neuralgia, chronic neuropathic pain following peripheral nerve injury, painful polyneuropathy, postherpetic neuralgia, and painful radiculopathy, or symptoms associated therewith.

In some embodiments, the disease is a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.

In some embodiments, the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.

In some embodiments, the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (e.g., chronic post-surgical neuralgia), chemotherapy-associated neuropathic pain, and HIV (Human Immunodeficiency Virus) painful sensory neuropathy, or symptoms associated therewith.

The "pharmaceutically acceptable carriers" in the present invention refer to diluents, adjuvants, excipients, or vehicles administered together with a therapeutic agent, which are suitable, within the scope of sound medical judgment, for contact with the tissues of humans and/or other animals without excessive toxicity, irritation, allergic response, or other problems or complications commensurate with a reasonable benefit/risk ratio.

Pharmaceutically acceptable carriers for use in the pharmaceutical composition of the present invention include, but are not limited to, sterile liquids, such as water and oils, including oils of petroleum, animal, vegetable, or synthetic origin, such as peanut oil, soybean oil, mineral oil, sesame oil, and the like. When the pharmaceutical composition is administered intravenously, water is an exemplary carrier. Saline, aqueous glucose solution, and aqueous glycerol solution may also be employed as liquid carriers, particularly for injectable preparations. Suitable pharmaceutical excipients include starch, glucose, lactose, sucrose, gelatin, maltose, chalk, silica gel, sodium stearate, glyceryl monostearate, talc, sodium chloride, skimmed milk powder, glycerol, propylene glycol, water, ethanol, and the like. The composition may also contain minor amounts of wetting agents, emulsifiers, or pH buffering agents, as appropriate. Oral formulations may include standard carriers such as pharmaceutical-grade mannitol, lactose, starch, magnesium stearate, sodium saccharin, cellulose, and magnesium carbonate. Examples of suitable pharmaceutically acceptable carriers are described in Remington's Pharmaceutical Sciences (1990).

The pharmaceutical composition of the present invention may exert effect systemically and/or locally. For this purpose, they may be administered via any suitable route, for example, by injection (e.g., intravenous, intraarterial, subcutaneous, intraperitoneal, intramuscular injection, including infusion) or transdermal administration, or by oral, buccal, nasal, transmucosal, topical, or ocular administration, or by inhalation.

The pharmaceutical composition of the present invention may be administered in dosage forms suitable for these administration routes.

Such dosage forms include, but are not limited to, tablets, capsules, troches, lozenges, powders, sprays, creams, ointments, suppositories, gels, pastes, lotions, ointments, aqueous suspensions, injectable solutions, elixirs, and syrups.

As used herein, the term "effective amount" refers to an amount of the compound that, upon administration, alleviates to some extent one or more symptoms of the disorder being treated.

Dosage regimens may be adjusted to provide the optimal desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may encompass single or multiple doses. It is to be further understood that the specific dosage regimen for any particular individual shall be adjusted over time in accordance with the individual needs and the professional judgment of the skilled person administering or supervising the administration of the composition.

The amount of the compound of the present invention administered will depend on the individual being treated, the severity of the disorder or condition, the rate of administration, disposition of the compound, and judgment of the prescribing physician. In general, an effective dosage ranges from about 0.0001 to about 50 mg per kg of body weight per day, such as about 0.01 to about 10 mg/kg/day (administered as a single dose or divided doses). For a 70 kg human subject, this amounts to about 0.007 mg/day to about 3500 mg/day, such as about 0.7 mg/day to about 700 mg/day. In some instances, dosage levels below the lower limit of the aforementioned range may suffice, while in other cases relatively large doses may be administered without causing any adverse effects, provided that such large doses are first divided into several smaller doses to be administered throughout the day.

In some embodiments, the compound, or a pharmaceutically acceptable salt, ester, stereoisomer, polymorph, solvate, N-oxide, isotopically labeled compound, metabolite, or prodrug thereof, is administered in an amount of about 1 ng/kg to about 200 mg/kg per day, about 1 µg/kg to about 100 mg/kg per day, or about 1 mg/kg to about 50 mg/kg per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg of body weight per unit dose.

In some embodiments, the daily dosage of the compound, or a pharmaceutically acceptable salt, ester, stereoisomer, polymorph, solvate, N-oxide, isotopically labeled compound, metabolite, or prodrug thereof, is administered as a single daily dose, or divided into two, three, or four administrations.

In some embodiments, the compound, or a pharmaceutically acceptable salt, ester, stereoisomer, polymorph, solvate, N-oxide, isotopically labeled compound, metabolite, or prodrug thereof, is administered for at least 3 consecutive days, at least 4 consecutive days, at least 5 consecutive days, at least 6 consecutive days, at least 7 consecutive days, at least 8 consecutive days, at least 9 consecutive days, at least 10 consecutive days, at least 11 consecutive days, at least 12 consecutive days, at least 13 consecutive days, at least 14 consecutive days, at least 15 consecutive days, at least 16 consecutive days, at least 17 consecutive days, at least 18 consecutive days, at least 19 consecutive days, at least 20 consecutive days, at least 21 consecutive days, at least 22 consecutive days, at least 23 consecutive days, at least 24 consecutive days, at least 25 consecutive days, at least 30 consecutive days, at least 35 consecutive days, at least 40 consecutive days, at least 45 consecutive days, or at least 50 consecutive days.

In some embodiments, the compound, or a pharmaceutically acceptable salt, ester, stereoisomer, polymorph, solvate, N-oxide, isotopically labeled compound, metabolite, or prodrug thereof, is administered over one or more (e.g., 1, 2, 3, 4, 5, 6, 7, 8, 9, or 10) treatment courses, wherein each treatment course lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days; and there is an interval of 0, 1, 2, 3, 4, 5, 6, 7, 8, 9, 10 days, two weeks, three weeks, or four weeks between every two adjacent treatment courses.

The content or dosage amount of the compound of the present invention in a pharmaceutical composition may range from 0.005 mg/day to about 5000 mg/day, suitably from 0.01 mg to about 2000 mg, preferably 1-1500 mg, preferably 1-1200 mg, preferably 1-1000 mg, preferably 1-600 mg, 1-500 mg, 1-400 mg, 1-300 mg, 1-200 mg, 1-150 mg, 1-100 mg, or 1-50 mg; for example, the compound is administered in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2500, 3000, 3500, 4000, 4500, or 5000 mg/day, and the like.

As used herein, unless otherwise indicated, the term "treating" means reversing, alleviating, inhibiting the progress of the disorder or condition to which such term applies, or one or more symptoms of such disorder or condition; or preventing such disorder or condition, or one or more symptoms of such disorder or condition.

As used herein, the term "subject" or "individual" includes human or non-human animals. Exemplary human subjects include those suffering from diseases (e.g., the diseases described herein) (referred to as patients) or normal subjects. The term "non-human animals" in the present invention includes all vertebrates, such as non-mammal animals (e.g., birds, amphibians, reptiles) and mammals, such as non-human primates, livestock and/or domestic animals (e.g., sheep, dogs, cats, cows, pigs, etc.).

In some embodiments, the pharmaceutical composition of the present invention may further comprise one or more additional therapeutic or prophylactic agents.

### Description of the Drawings

FIG. 1 shows the effect of AT2R antagonists on AngII-induced reactive oxygen species generation in mouse peritoneal macrophages via ROS fluorescence intensity of different treatment groups. Note: ###p < 0.001 vs. control group; ***p < 0.001 vs. AngII (0.2 µM) group.
FIG. 2 shows the changes in 50% PWT of animals in each group at different administration time points during the pharmacodynamic test of AT2R antagonists on a rat diabetic foot pain model induced by a high-fat and high-sugar diet combined with STZ.
FIG. 3 shows the efficacy of AT2R antagonists in an allodynia test using a spinal nerve ligation model in SD rats.
FIG. 4 shows the pharmacodynamic evaluation results of AT2R antagonists in a rat model of chronic constriction injury of the sciatic nerve.
FIG. 5 shows the paw withdrawal threshold (PWT) of SNI mice in each group during the pharmacodynamic test ofAT2R antagonists on a mouse spared nerve injury (SNI) model (mean + SD, ***p < 0.001 vs. vehicle group at the corresponding time points).
FIG. 6 shows representative images of macrophages co-stained for AT2R (AKA: AGTR2) and F4/80 on the injured-side and uninjured-side sciatic nerves of SNI mice (Bar = 20 µm).
FIG. 7 shows the fluorescence intensity of macrophages co-stained for AT2R and F4/80 in the injured-side sciatic nerve of SNI mice. Note: ***p < 0.001 vs. vehicle group.
FIG. 8 shows the fluorescence intensity of macrophages co-stained for AT2R and F4/80 in the uninjured-side sciatic nerve of SNI mice. Note: ***p < 0.001 vs. vehicle group.
FIG. 9 shows representative images of TRPA1 immunofluorescence staining on the injured-side DRG of SNI mice (Bar = 20 µm).
FIG. 10 shows the mean fluorescence intensity of TRPA1 in the injured-side DRG neurons of SNI mice (***p < 0.001 vs. vehicle group).
FIG. 11 shows a significant increase in TRPA1 expression in the injured-side sciatic nerve of SNI mice compared with normal control (Con) mice. In the SNI mouse model, compared with the saline-treated group, the A1200 mg/kg treatment group showed a reduction in TRPA1 levels, nearly reaching the TRPA1 expression level in the normal control group.
FIG. 12 shows the inhibitory effects of A1, PD123319, and Olodanrigan on AngII-activated AT2R in R264.7 cells.
FIG. 13 shows the inhibitory effects of A1, PD123319, and Olodanrigan on the AngII-induced elevation of inflammatory factors in the supernatant of R264.7 cells.
FIG. 14 shows the inhibitory effects of A1, PD123319, and Olodanrigan on the protein expression and mRNA transcription of TRPA1 and TRPV1.
FIG. 15 shows the results of *in vitro* cytotoxicity assays, demonstrating that A1 exhibits no cytotoxicity on RAW264.7 and HT22 cells.

### Examples

### Compound Examples

The present application provides the following compounds known in the prior art, the preparation and characterization of which are disclosed in the Examples of WO2019179515A1. The entire content of the Examples in WO2019179515A1 is incorporated herein by reference.

| No. | Structure |
|---|---|
| C1 | 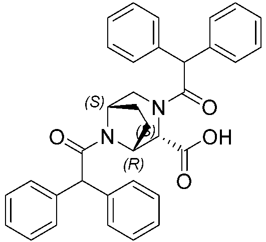 |
| C2 | 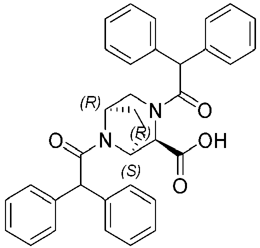 |
| C3 | 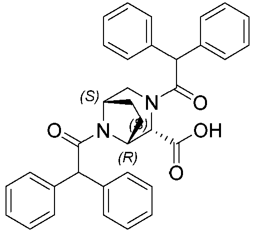 |
| C4 | 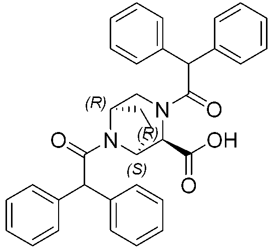 |
| C5 | 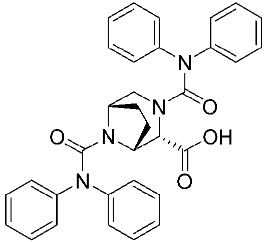 |
| C6 | 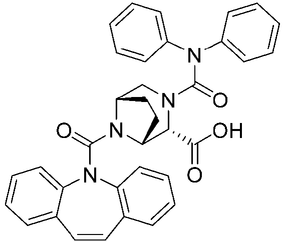 |
| C7 | 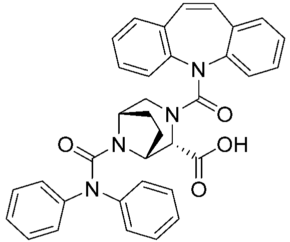 |
| C8 | 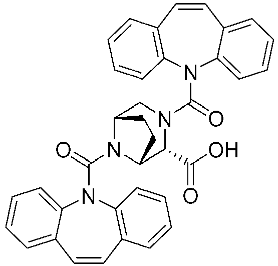 |
| C9 | 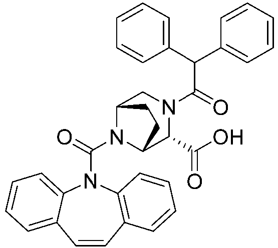 |
| C10 | 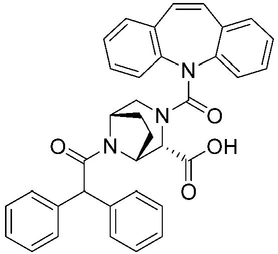 |
| C11 | 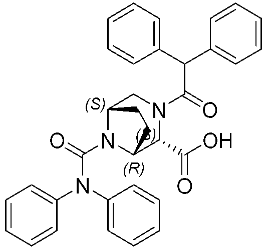 |
| C12 | 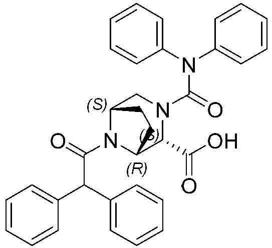 |
| C13 | 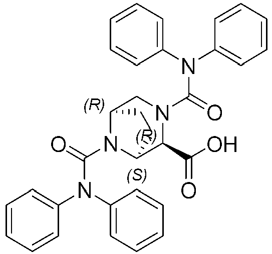 |
| C14 | 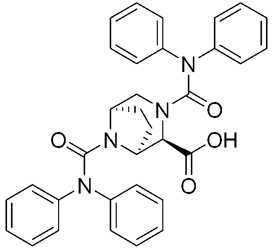 |
| C15 | 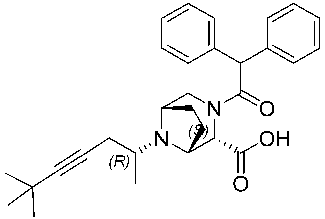 |
| C16 | 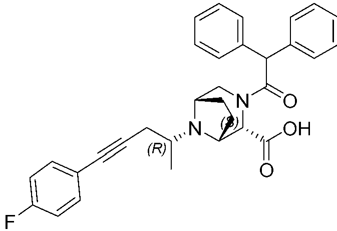 |
| C17 | 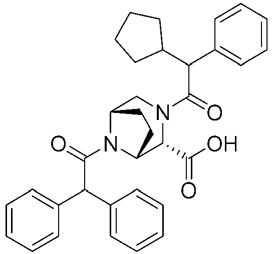 |
| C18 | 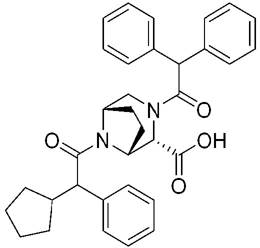 |
| C19 | 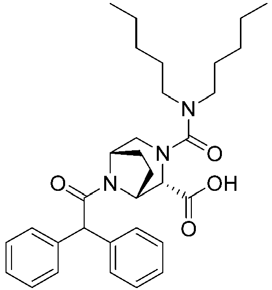 |
| C20 | 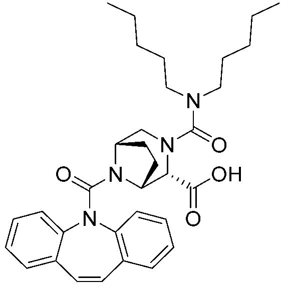 |
| C21 | 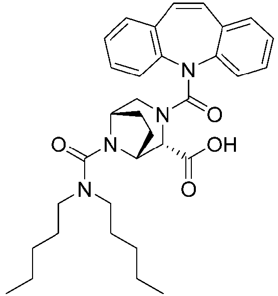 |
| C22 | 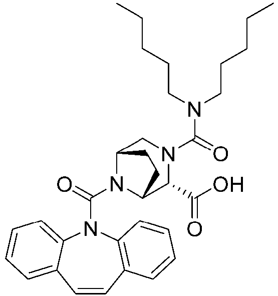 |
| C23 | 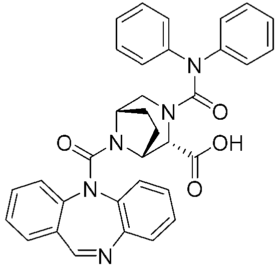 |
| C24 | 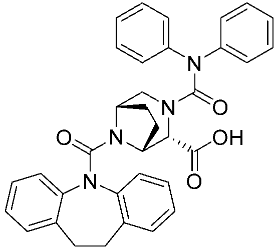 |
| C25 | 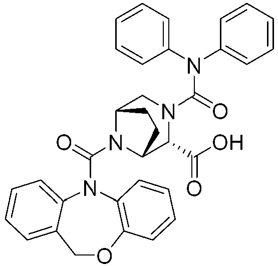 |
| C26 | 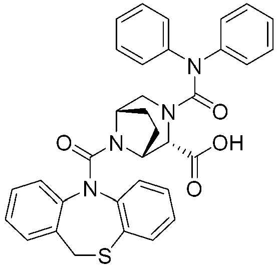 |
| C27 | 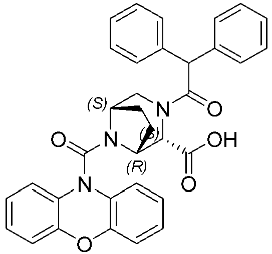 |
| C28 | 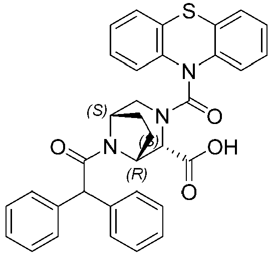 |
| C29 | 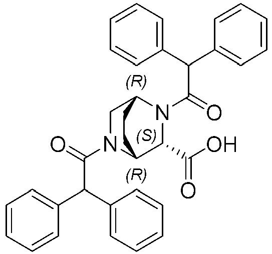 |
| C30 | 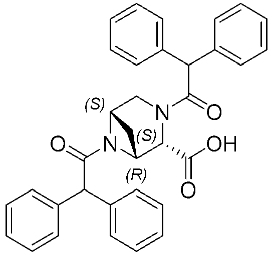 |
| C31 | 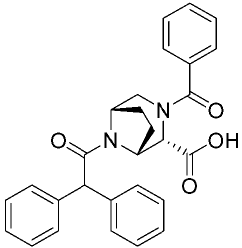 |
| C32 | 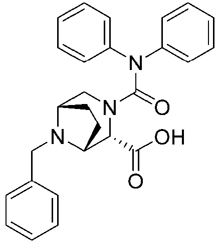 |
| C33 | 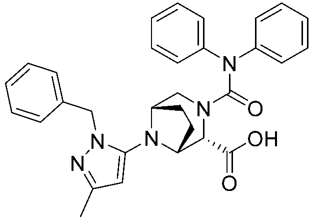 |
| C34 | 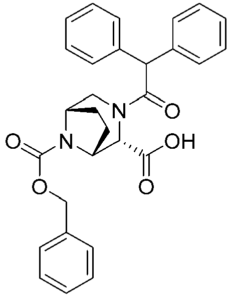 |
| C35 | 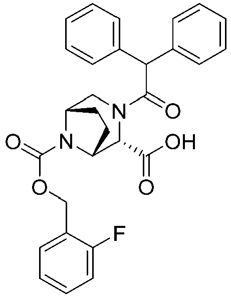 |
| C36 | 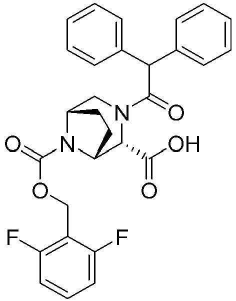 |
| C37 | 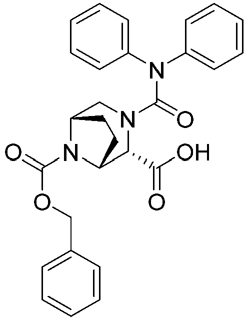 |
| C38 | 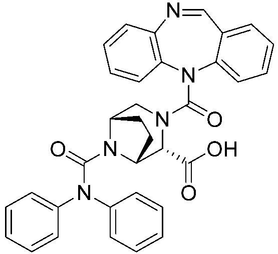 |
| C39 | 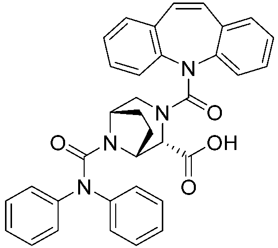 |
| C40 | 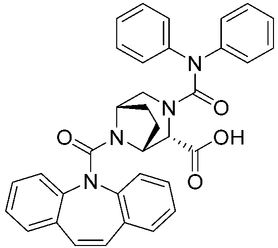 |
| C41 | 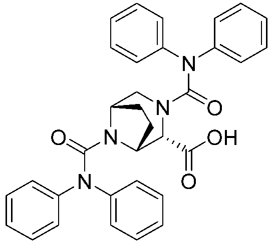 |
| C42 | 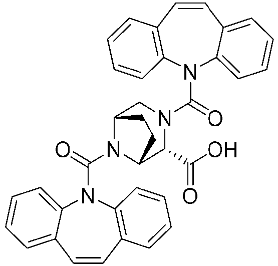 |
| C43 | 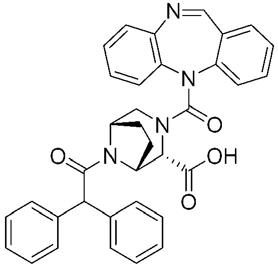 |
| C44 | 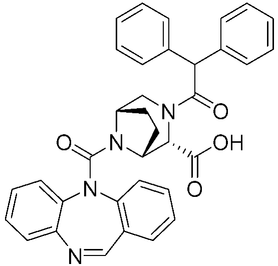 |
| C45 | 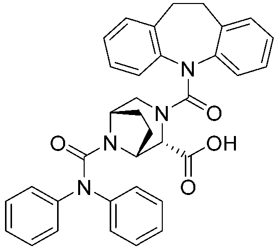 |
| C46 | 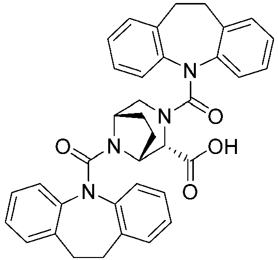 |
| C47 | 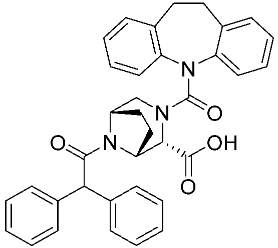 |
| C48 | 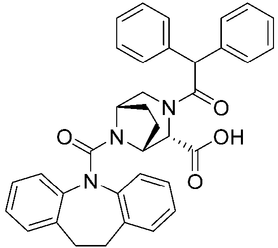 |
| C49 | 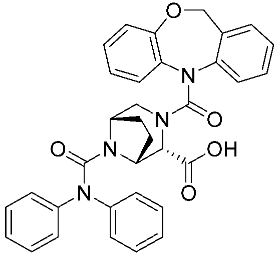 |
| C50 | 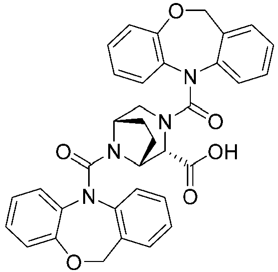 |
| C51 | 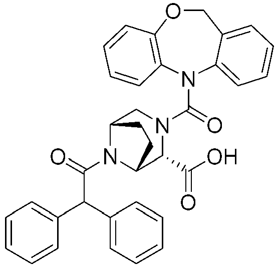 |
| C52 | 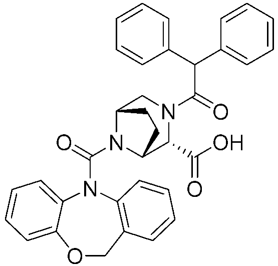 |
| C53 | 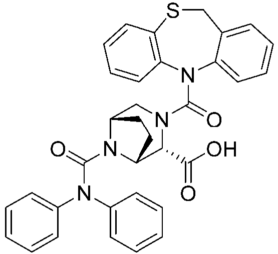 |
| C54 | 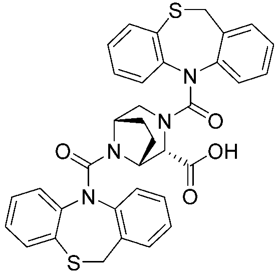 |
| C55 | 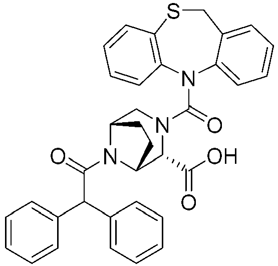 |
| C56 | 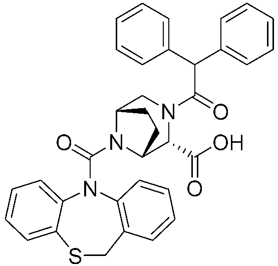 |
| C57 | 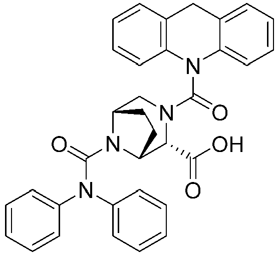 |
| C58 | 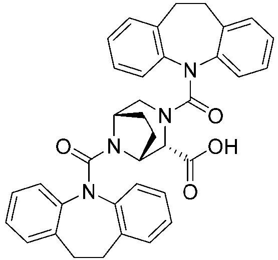 |
| C59 | 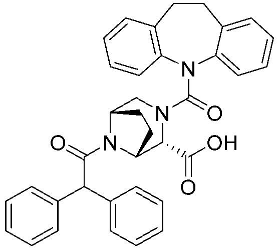 |
| C60 | 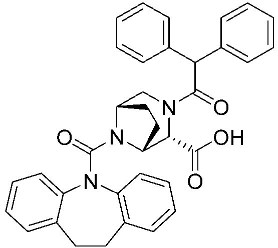 |
| C61 | 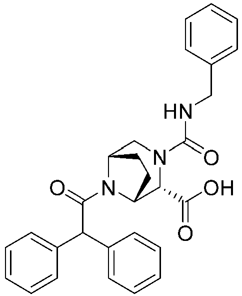 |
| C62 | 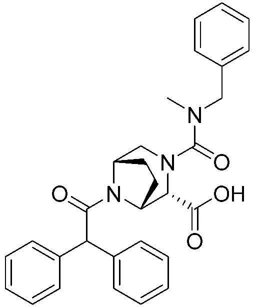 |
| C63 | 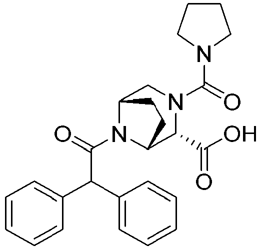 |
| C64 | 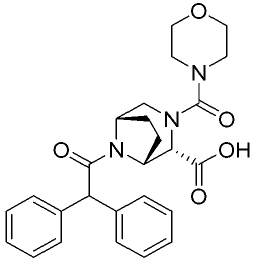 |
| C65 | 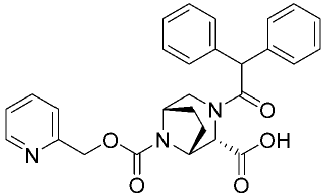 |
| C66 | 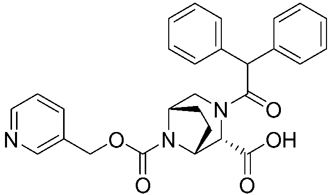 |
| C67 | 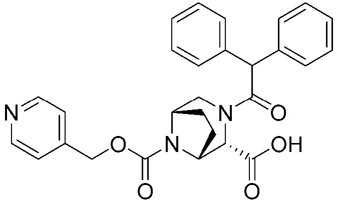 |
| C68 | 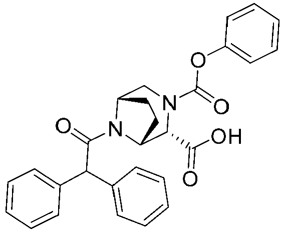 |
| C69 | 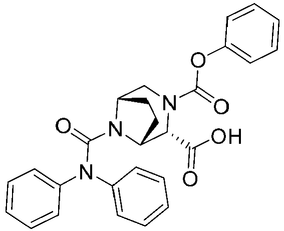 |
| C70 | 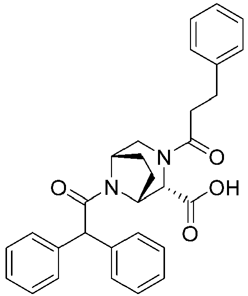 |
| C71 | 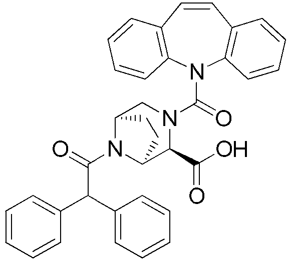 |
| C72 | 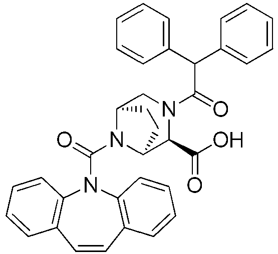 |
| C73 | 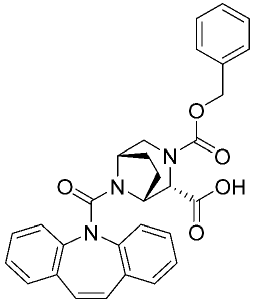 |
| C74 | 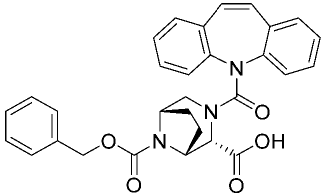 |
| C75 | 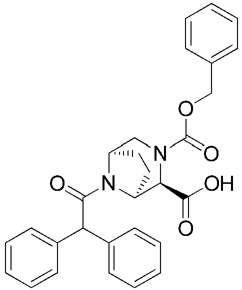 |
| C76 | 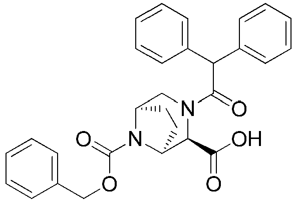 |
| C77 | 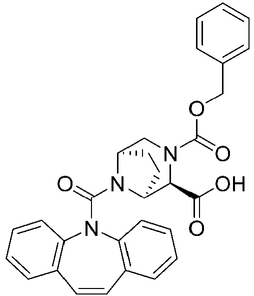 |
| C78 | 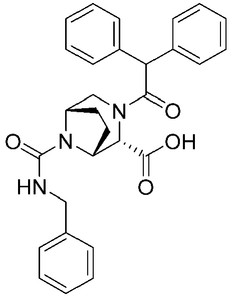 |
| C79 | 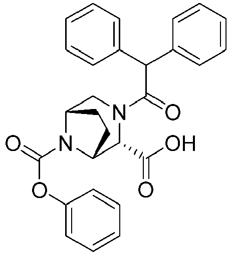 |
| C80 | 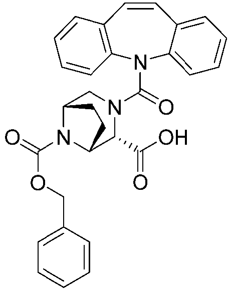 |
| C81 | 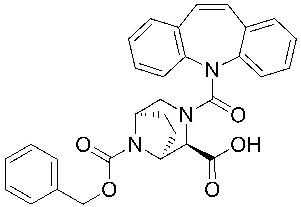 |
| C82 | 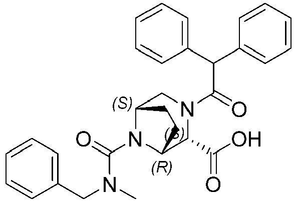 |
| C83 | 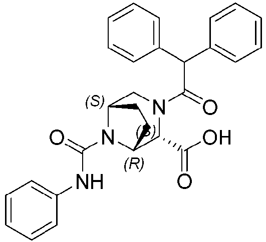 |
| C84 | 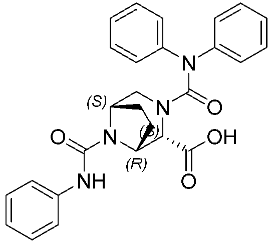 |
| C85 | 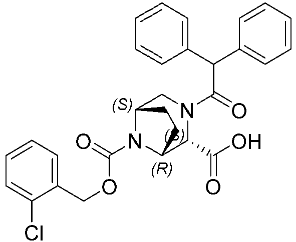 |
| C86 | 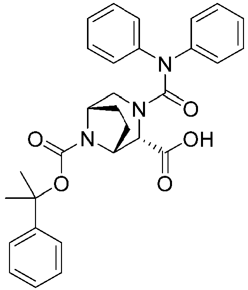 |
| C87 | 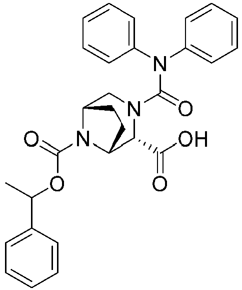 |
| C88 | 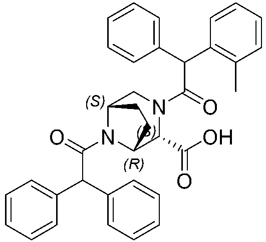 |
| C89 | 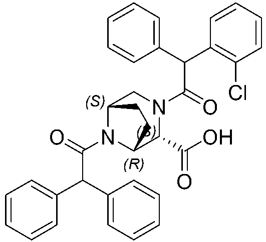 |
| C90 | 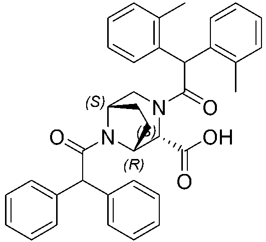 |
| C91 | 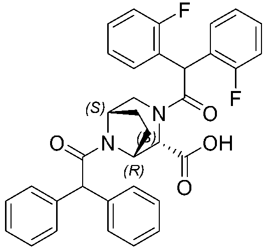 |
| C92 | 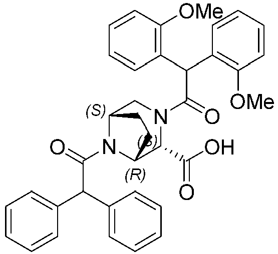 |
| C93 | 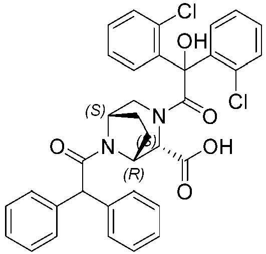 |
| C94 | 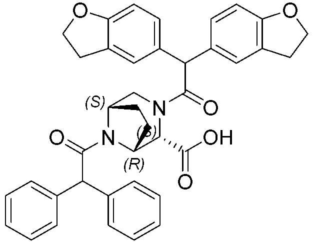 |
| C95 | 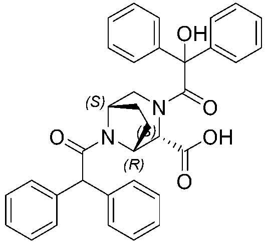 |
| C96 | 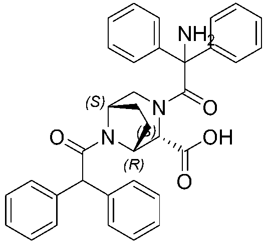 |
| C97 | 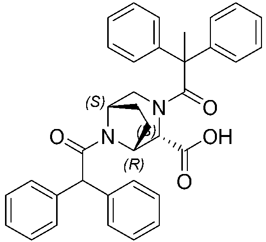 |
| C98 | 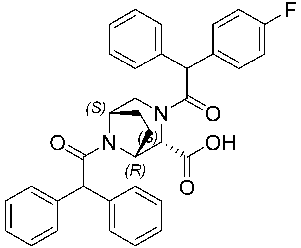 |
| C99 | 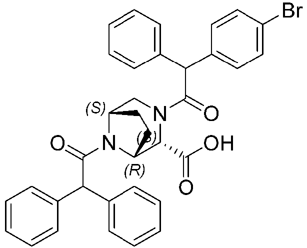 |
| C100 | 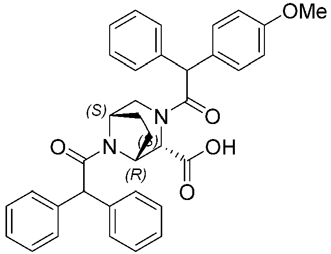 |
| C101 | 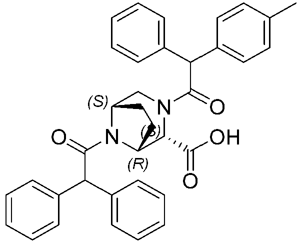 |
| C102 | 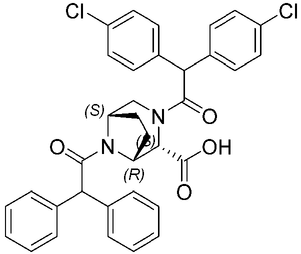 |
| C103 | 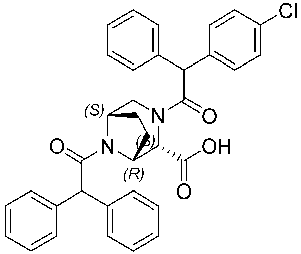 |
| C104 | 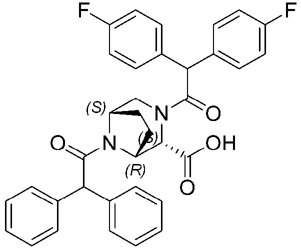 |
| C105 | 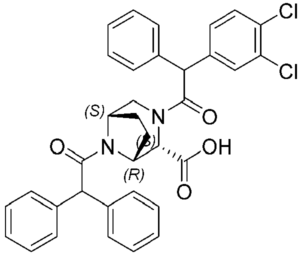 |
| C106 | 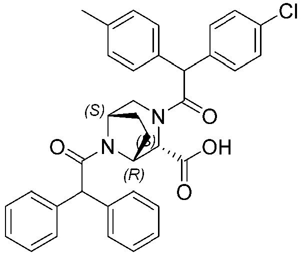 |
| C107 | 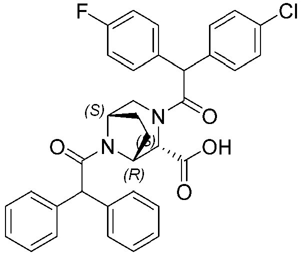 |
| C108 | 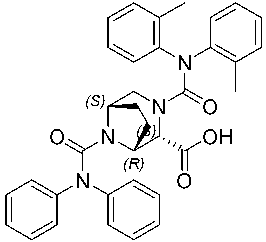 |
| C109 | 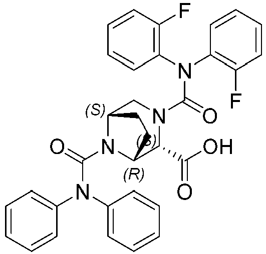 |
| C110 | 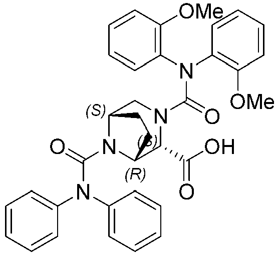 |
| C111 | 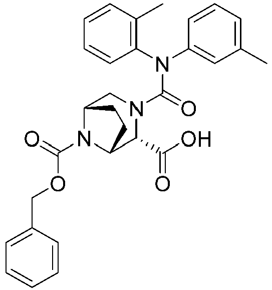 |
| C112 | 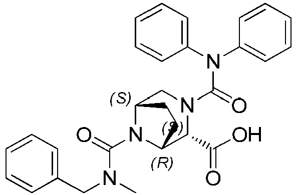 |
| C113 | 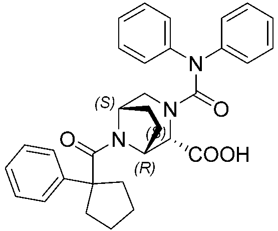 |
| C114 | 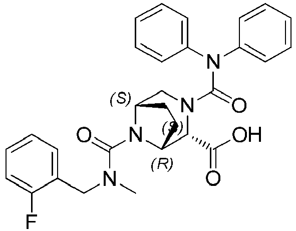 |
| C115 | 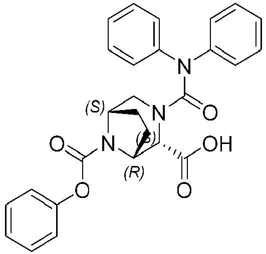 |
| C116 | 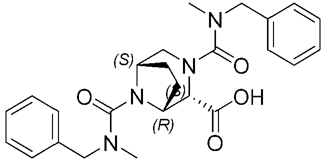 |
| C117 | 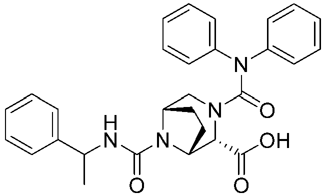 |
| C118 | 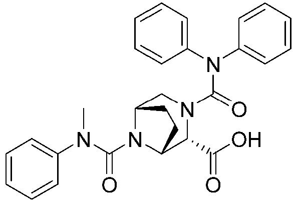 |
| C119 | 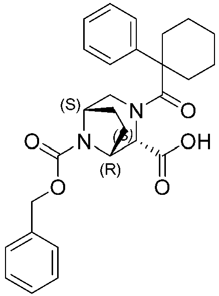 |
| C120 | 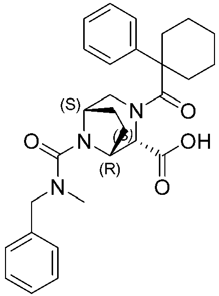 |
| C121 | 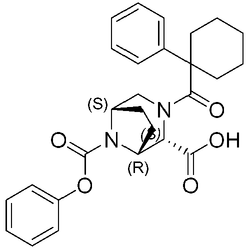 |
| C122 | 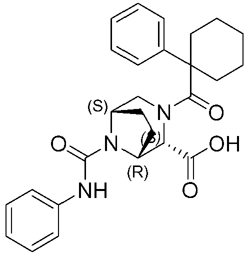 |
| C123 | 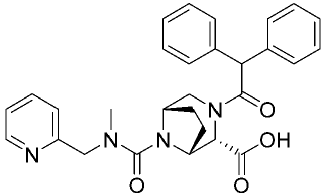 |
| C124 | 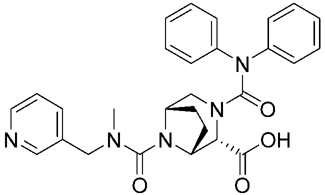 |
| C125 | 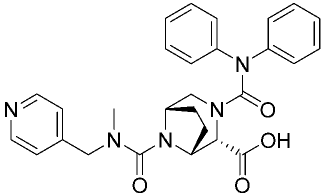 |
| C126 | 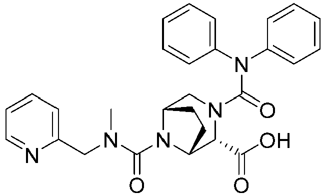 |
| C127 | 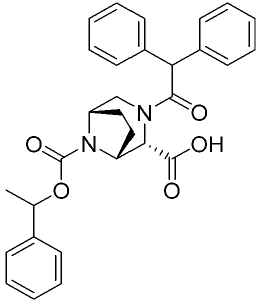 |
| C128 | 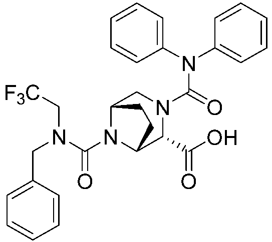 |
| C129 | 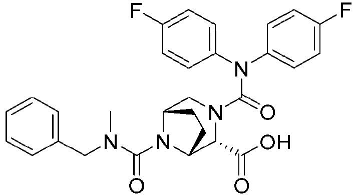 |
| C130 | 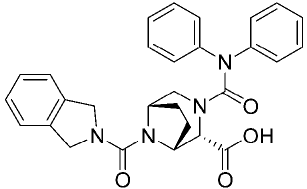 |
| C131 | 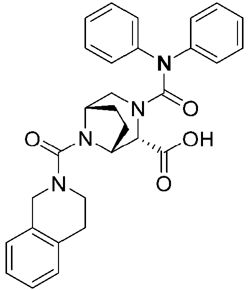 |
| C132 | 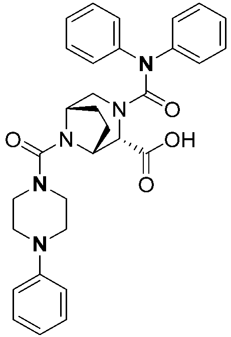 |
| C133 | 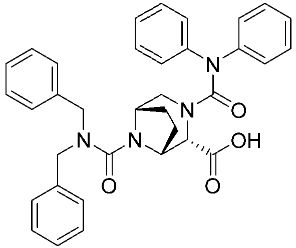 |
| C134 | 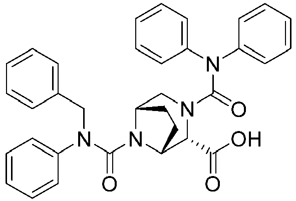 |
| C135 | 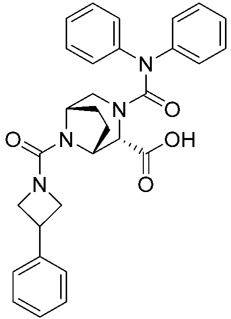 |
| C136 | 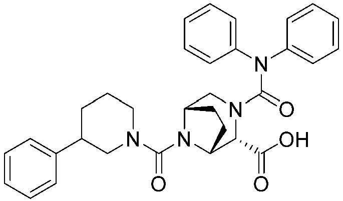 |
| C137 | 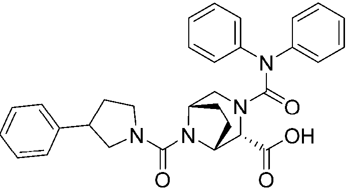 |
| C138 | 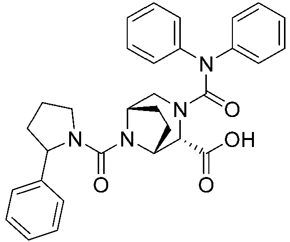 |
| C139 | 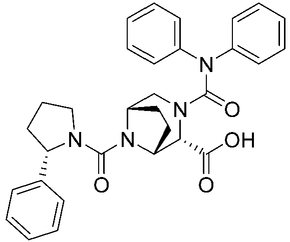 |
| C140 | 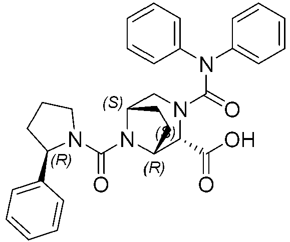 |
| C141 | 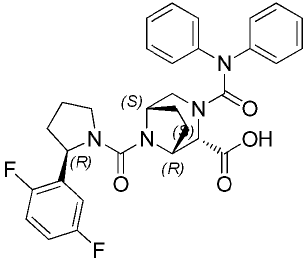 |
| C142 | 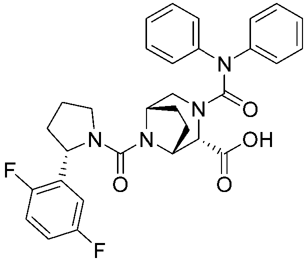 |
| C143 | 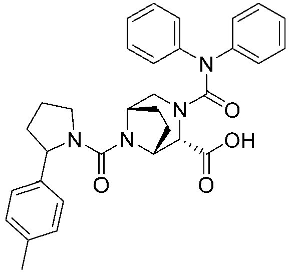 |
| C144 | 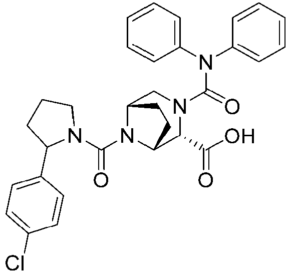 |
| C145 | 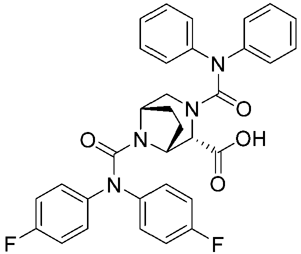 |
| C146 | 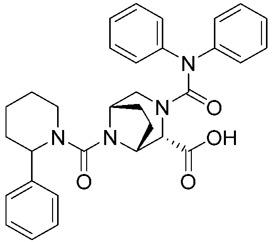 |
| C147 | 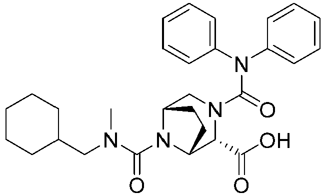 |
| C148 | 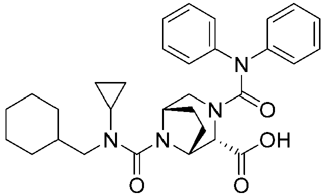 |
| C149 | 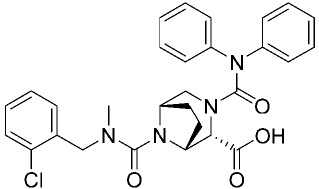 |
| C150 | 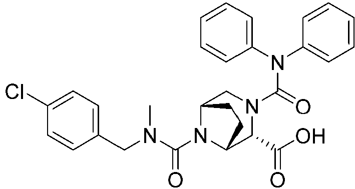 |
| C151 | 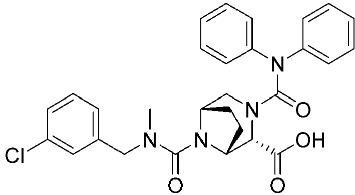 |
| C152 | 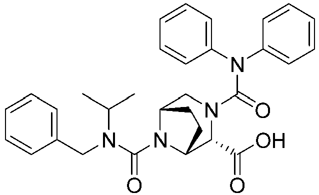 |
| C153 | 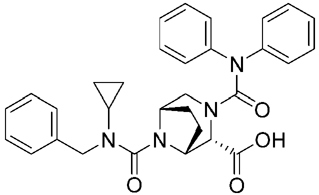 |
| C154 | 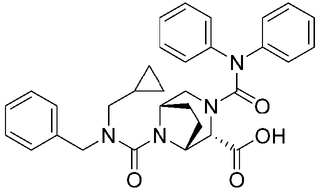 |
| C155 | 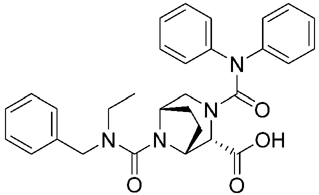 |
| C156 | 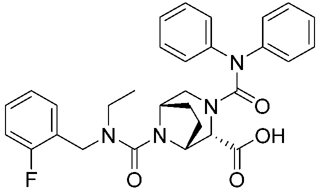 |
| C157 | 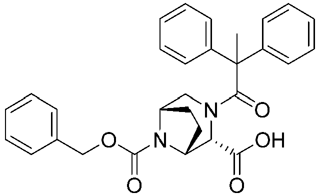 |
| C158 | 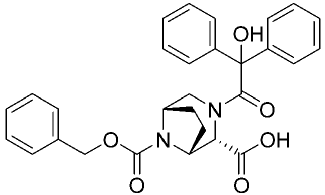 |
| C159 | 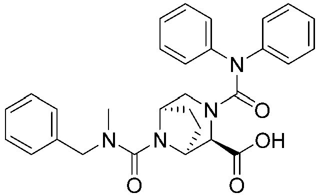 |
| C160 | 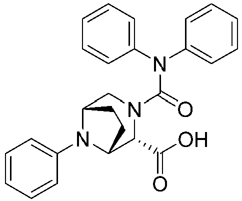 |
| C161 | 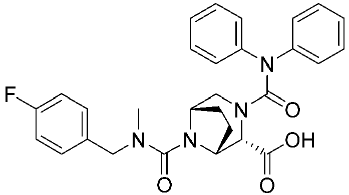 |
| C162 | 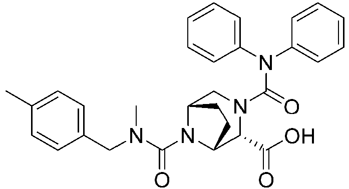 |
| C163 | 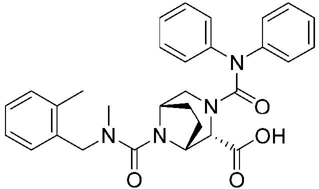 |
| C164 | 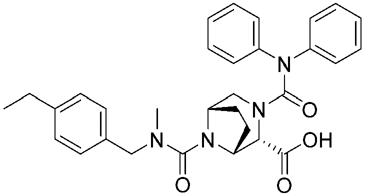 |
| C165 | 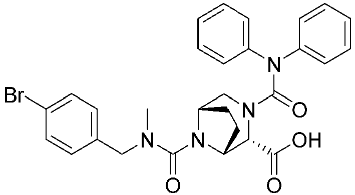 |
| C166 | 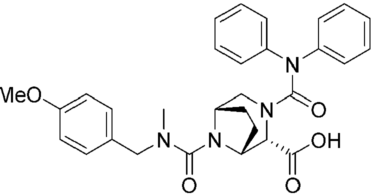 |
| C167 | 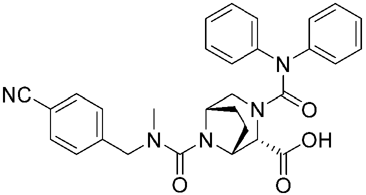 |
| C168 | 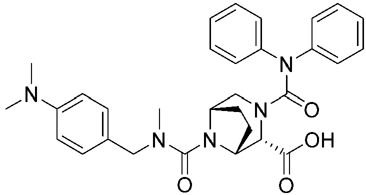 |
| C169 | 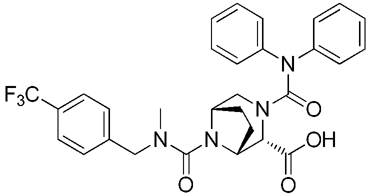 |
| C170 | 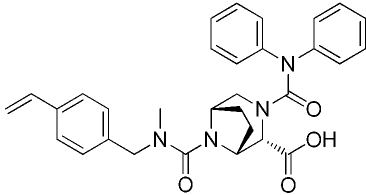 |
| C171 | 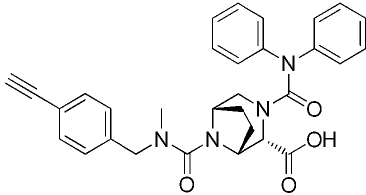 |
| C172 | 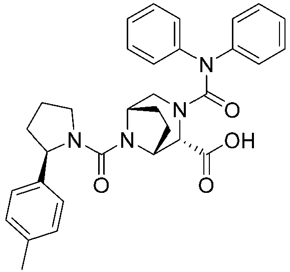 |
| C173 | 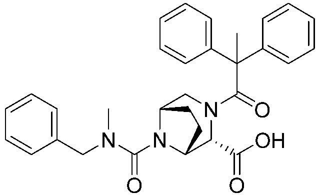 |
| C174 | 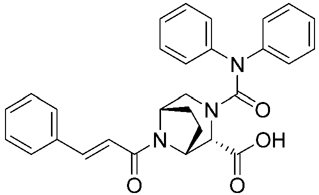 |
| C177 | 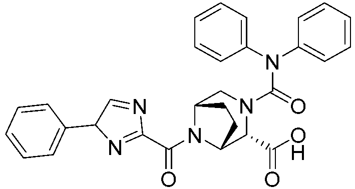 |
| C178 | 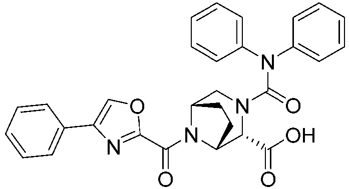 |
| C179 | 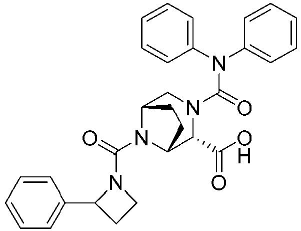 |
| C180 | 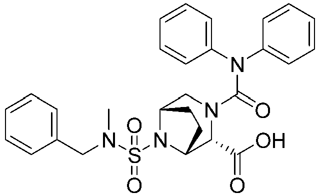 |
| C181 | 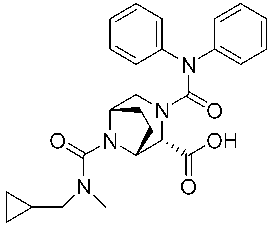 |
| C182 | 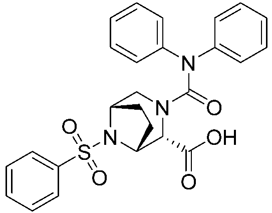 |
| C183 | 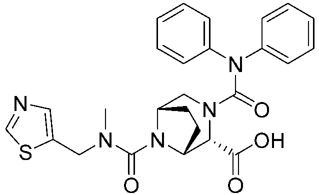 |
| C184 | 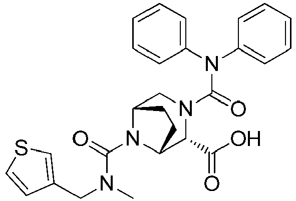 |
| C185 | 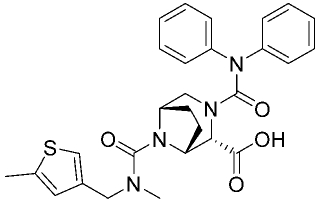 |
| C186 | 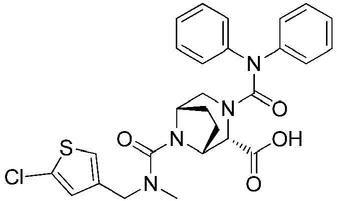 |
| C187 | 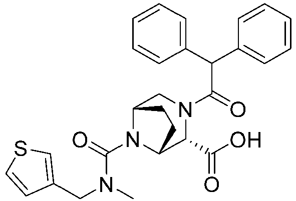 |
| C188 | 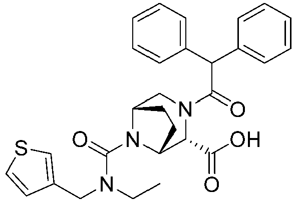 |
| C189 | 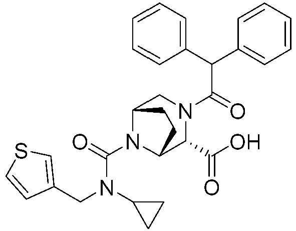 |
| C190 | 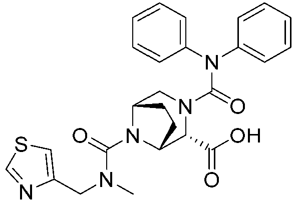 |
| C191 | 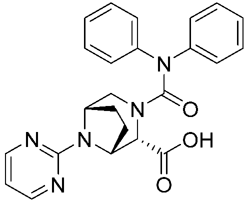 |
| C192 | 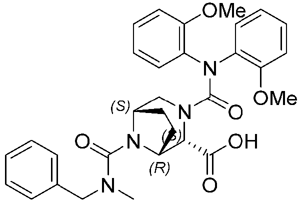 |
| C193 | 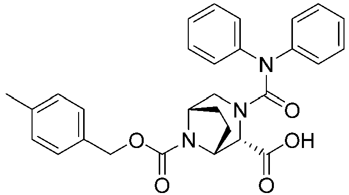 |
| C194 | 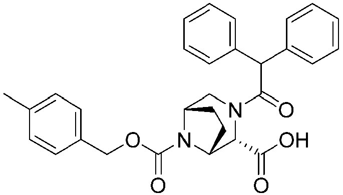 |
| C195 | 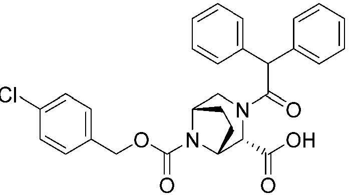 |
| C196 | 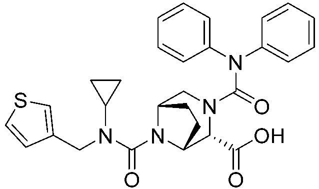 |
| C197 | 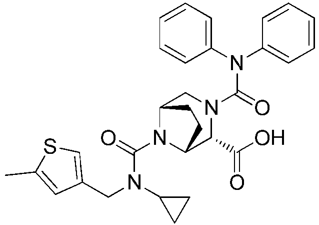 |
| C198 | 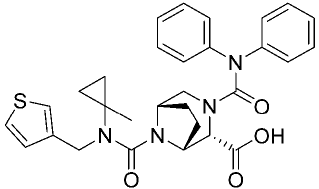 |
| C199 | 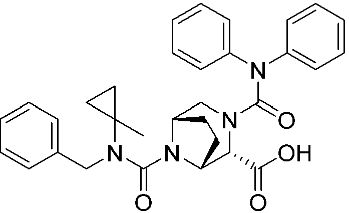 |
| C200 | 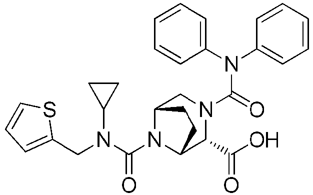 |
| C201 | 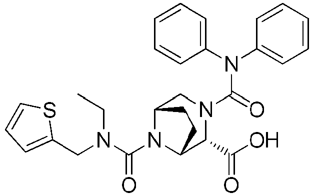 |
| C202 | 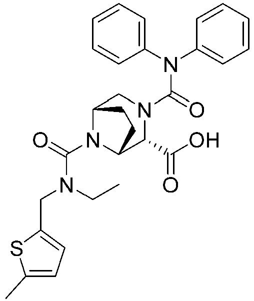 |
| C203 | 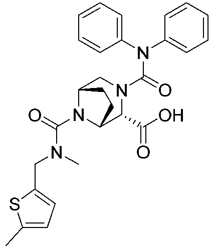 |
| C204 | 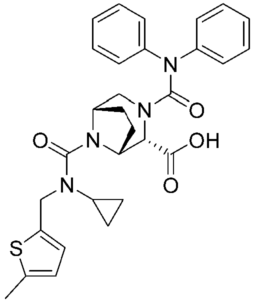 |
| C205 | 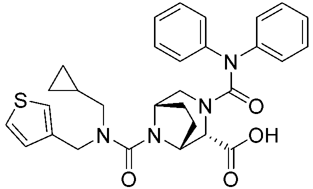 |
| C206 | 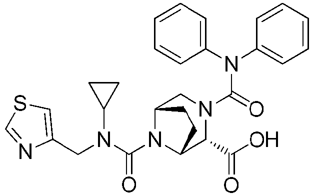 |
| C207 | 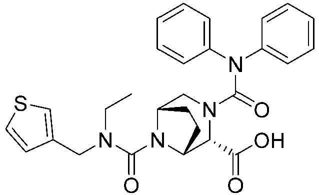 |
| C208 | 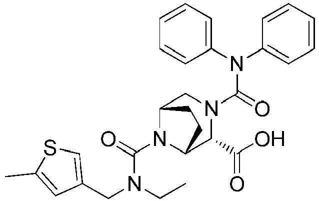 |
| C209 | 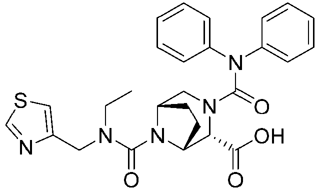 |
| C210 | 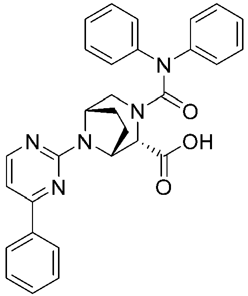 |
| C211 | 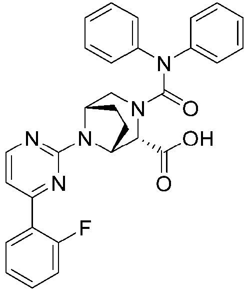 |
| C212 | 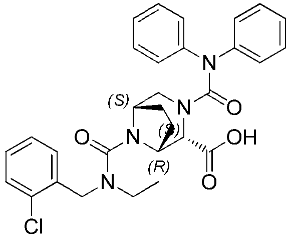 |
| C213 | 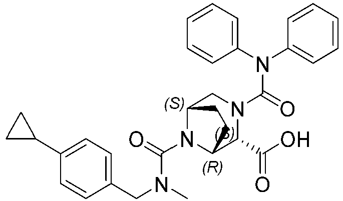 |
| C214 | 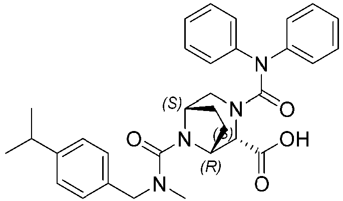 |
| C215 | 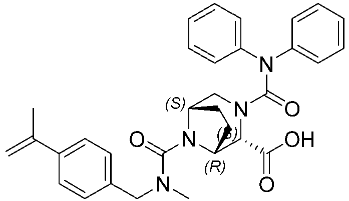 |
| C216 | 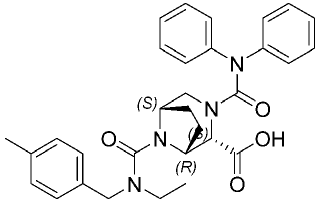 |
| C217 | 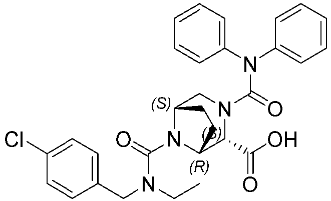 |
| C218 | 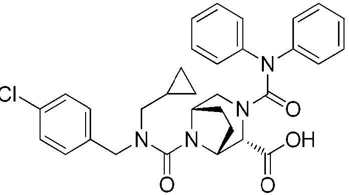 |
| C219 | 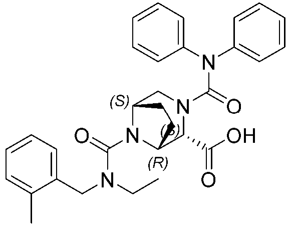 |
| C220 | 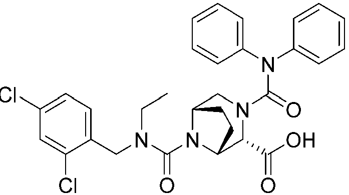 |
| C221 | 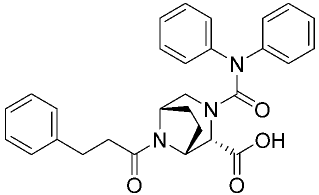 |
| C222 | 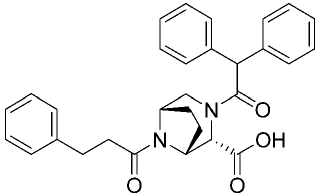 |
| C223 | 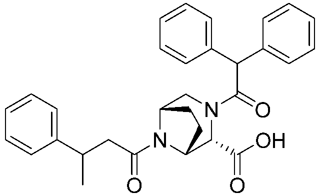 |
| C224 | 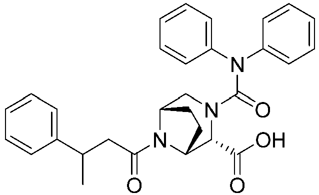 |
| C225 | 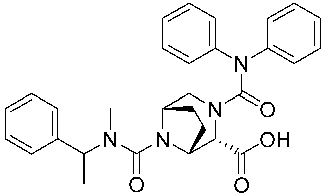 |
| C226 | 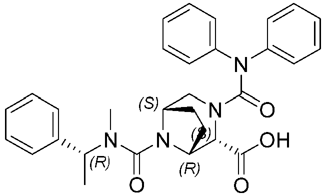 |
| C227 | 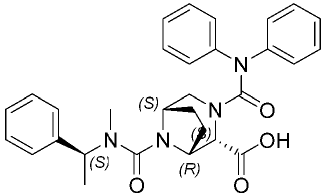 |
| C228 | 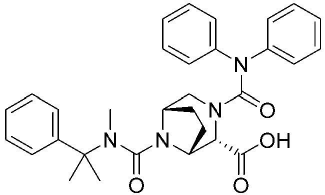 |
| C229 | 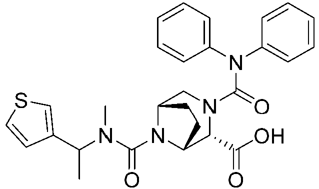 |
| C230 | 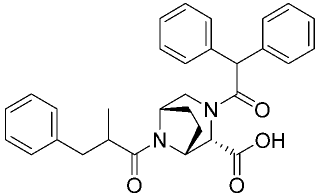 |
| C231 | 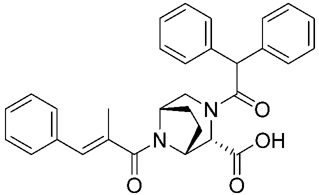 |
| C232 | 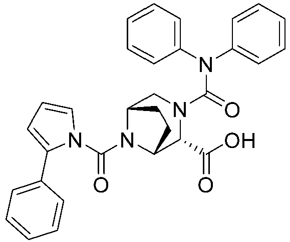 |
| C233 | 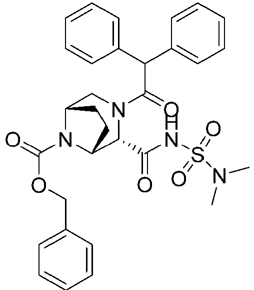 |
| C234 | 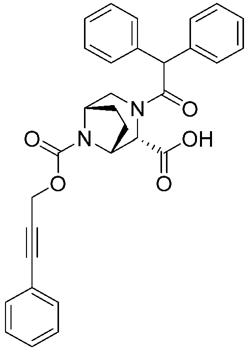 |
| C235 | 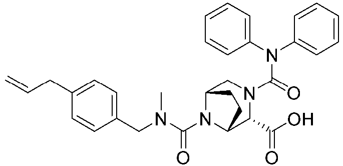 |
| C236 | 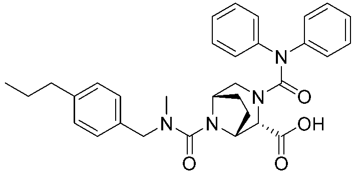 |
| C237 | 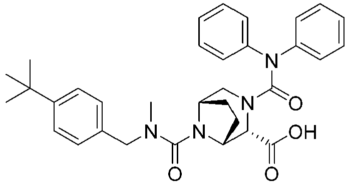 |
| C238 | 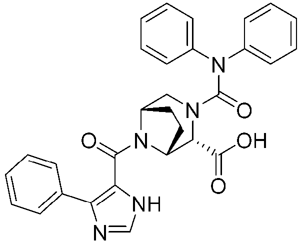 |
| C239 | 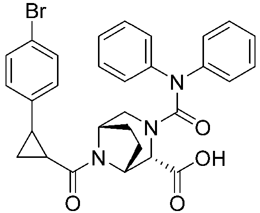 |
| C240 | 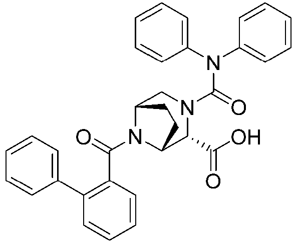 |
| C241 | 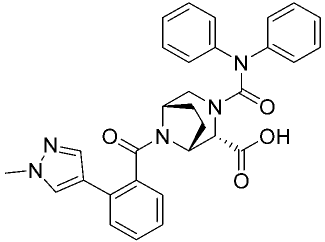 |
| C242 | 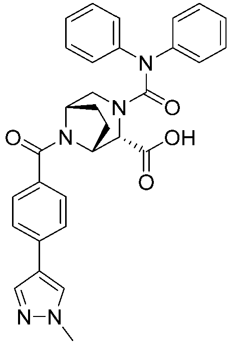 |
| C243 | 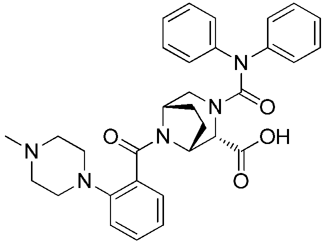 |
| C244 | 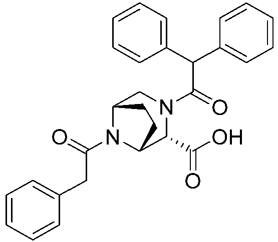 |
| C245 | 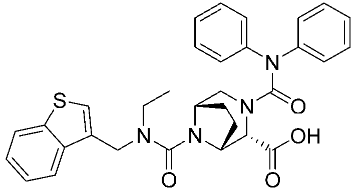 |
| C246 | 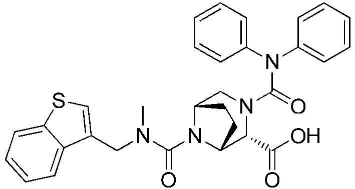 |
| C247 | 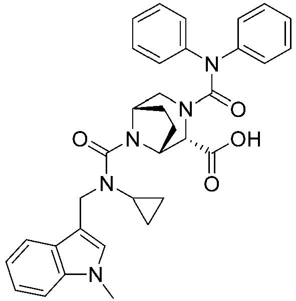 |
| C248 | 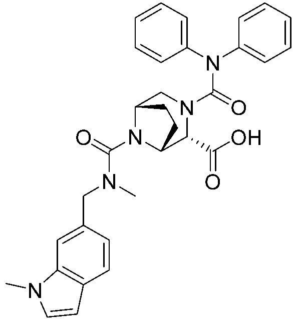 |
| C249 | 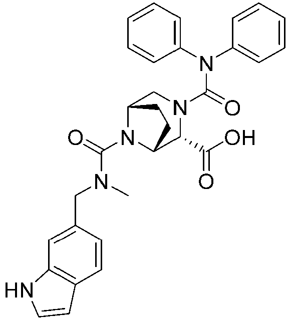 |
| C250 | 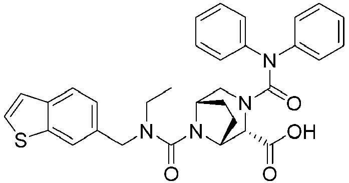 |
| C251 | 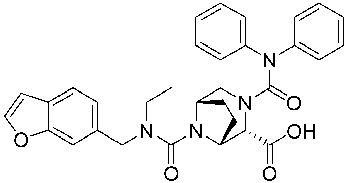 |
| C252 | 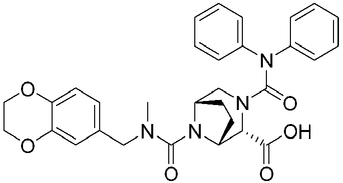 |
| C253 | 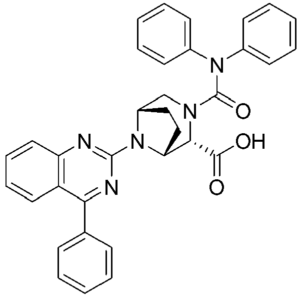 |
| C254 | 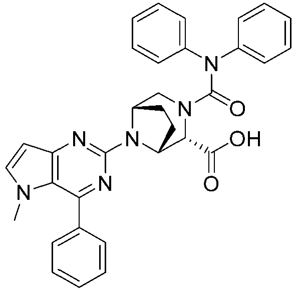 |
| C255 | 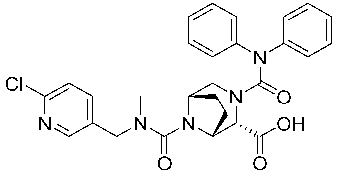 |
| C256 | 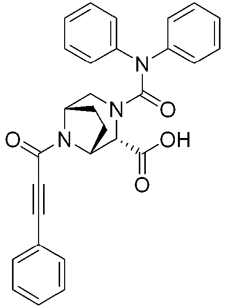 |
| C257 | 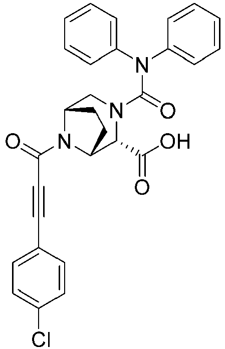 |
| C258 | 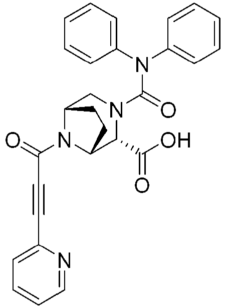 |
| C259 | 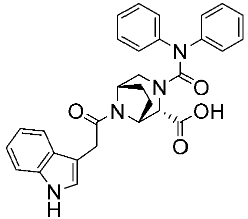 |
| C260 | 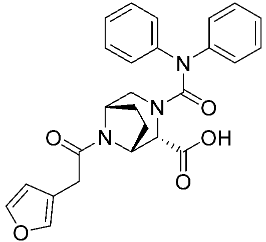 |
| C261 | 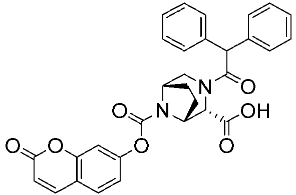 |
| C262 | 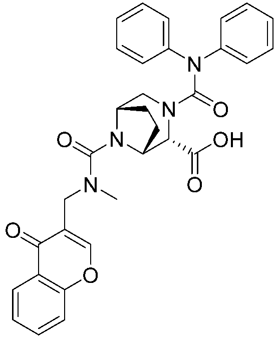 |
| C263 | 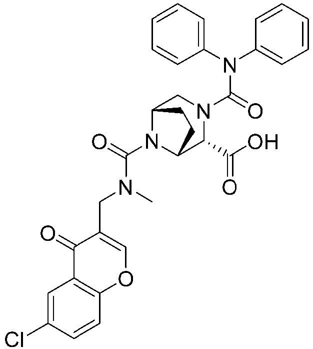 |
| C264 | 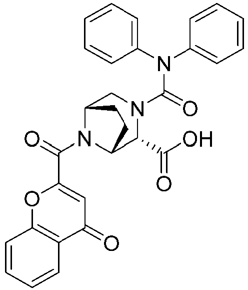 |
| C265 | 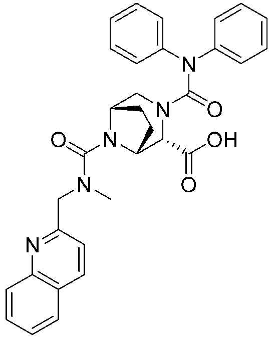 |
| C266 | 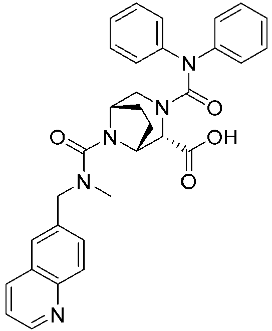 |
| C267 | 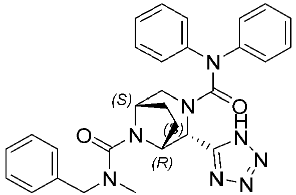 |
| C268 | 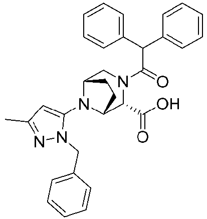 |
| C269 | 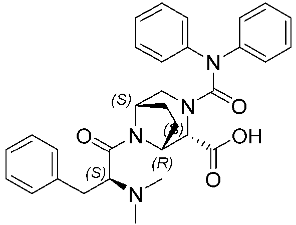 |
| C270 | 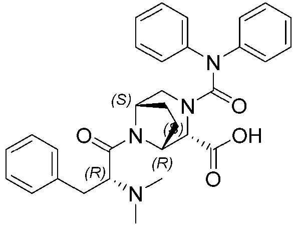 |
| C271 | 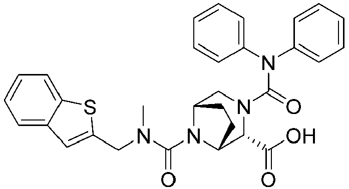 |
| C272 | 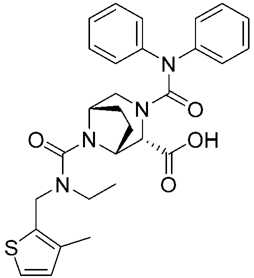 |
| C273 | 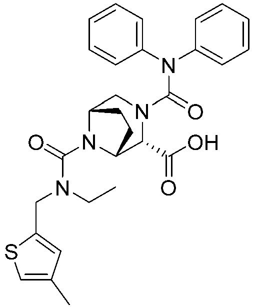 |
| C274 | 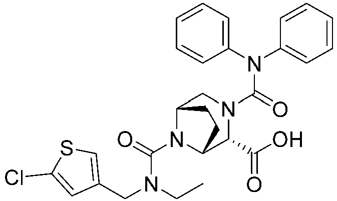 |
| C275 | 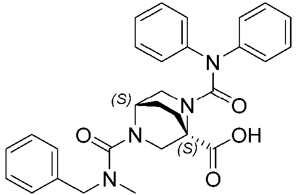 |
| C276 | 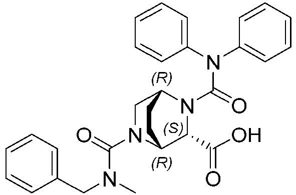 |
| C277 | 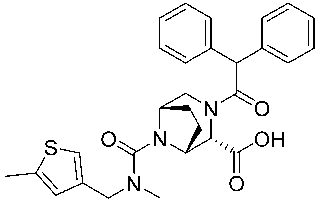 |
| C278 | 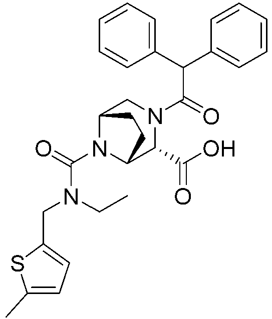 |
| C279 | 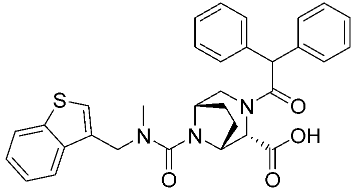 |
| C280 | 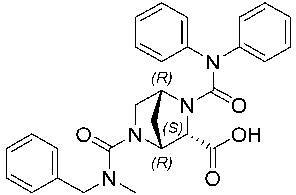 |
| C281 | 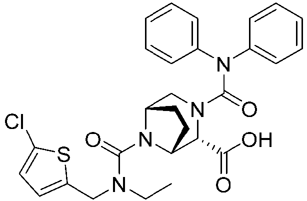 |
| C282 | 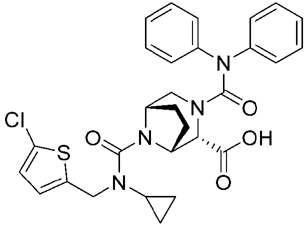 |
| C283 | 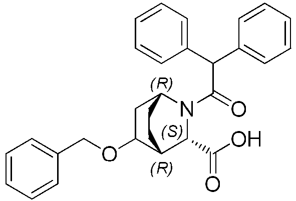 |
| C284 | 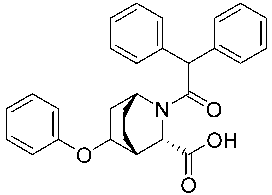 |
| C285 | 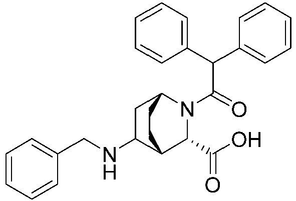 |
| C286 | 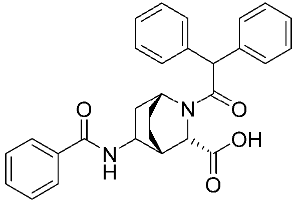 |
| C287 | 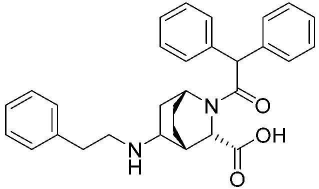 |
| C288 | 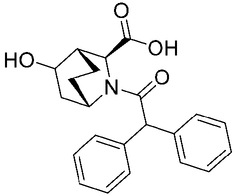 |
| C289 | 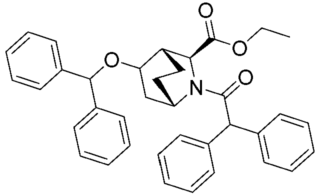 |
| C290 | 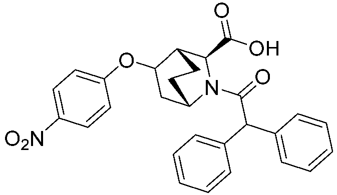 |
| C291 | 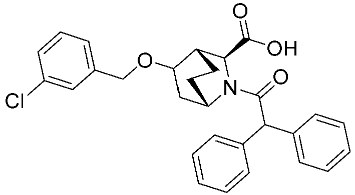 |
| C292 | 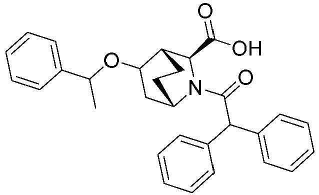 |
| C293 | 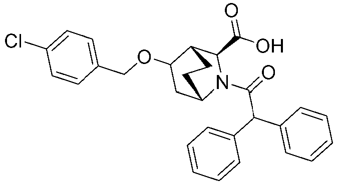 |
| C294 | 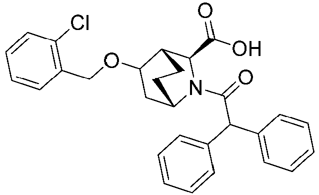 |
| C295 | 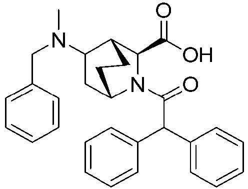 |
| C296 | 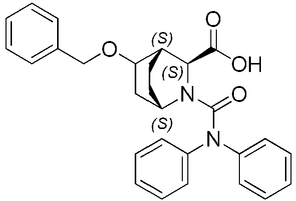 |
| C297 | 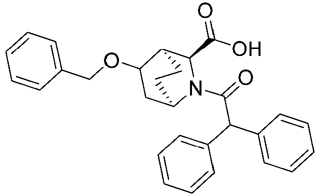 |
| C298 | 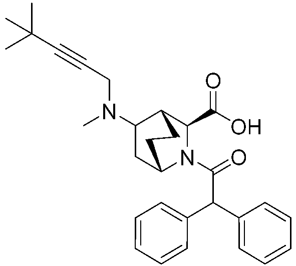 |
| C299 | 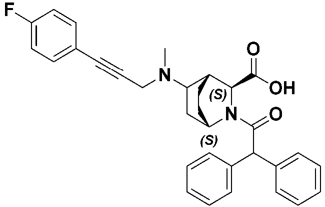 |
| C300 | 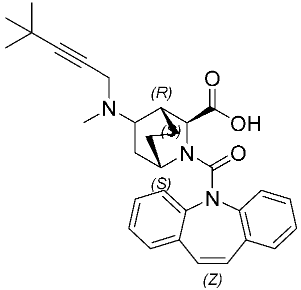 |
| C301 | 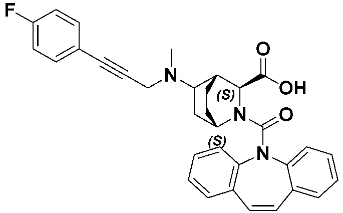 |
| C302 | 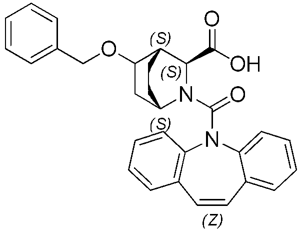 |
| C303 | 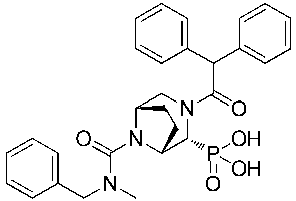 |
| C304 | 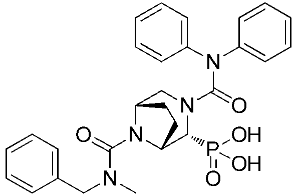 |
| C305 | 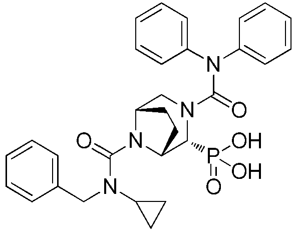 |
| C306 | F 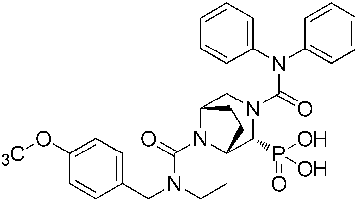 |
| C307 | 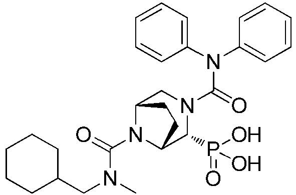 |
| C308 | 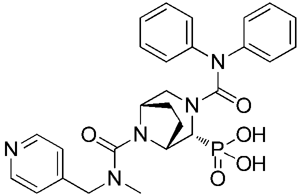 |
| C309 | 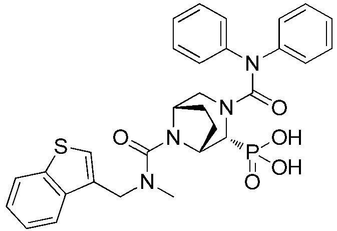 |
| C310 | 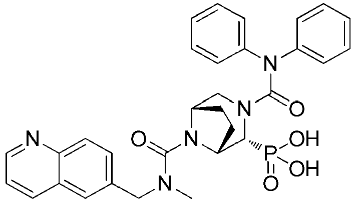 |
| C311 | 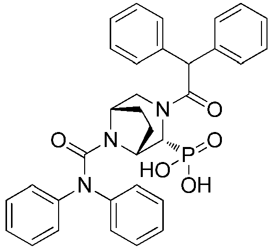 |
| C312 | 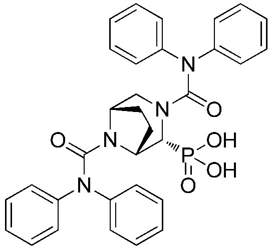 |
| C313 | 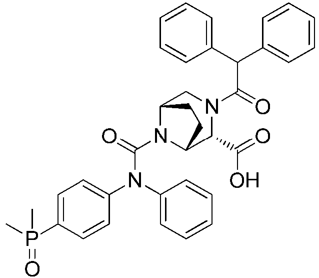 |
| C314 | 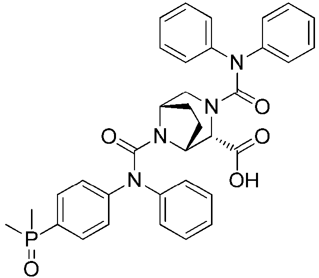 |
| C315 | 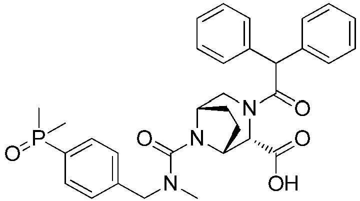 |
| C316 | 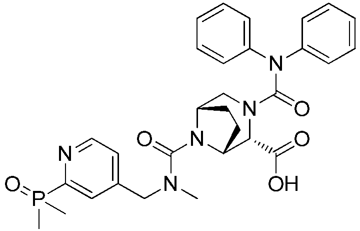 |
| C317 | 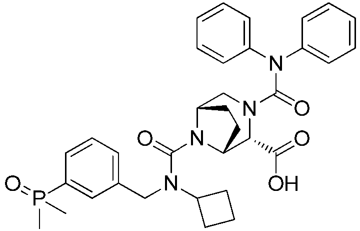 |
| C318 | 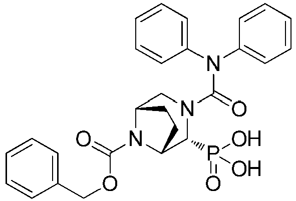 |
| C319 | 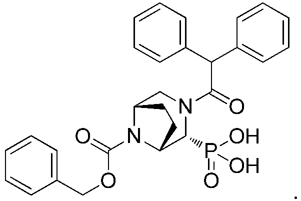 |

### Biological Examples

In the following biological assays, the correspondence between Compounds A1 to A8 used and the numbers of Compounds in the Examples is as follows: A1=C112, A2=C246, A3=C202, A4=C196, A5=C154, A6= C155, A7=C184, A8=C207. It should be noted that Compounds A1 to A8 are used as exemplary compounds in the following assays, and this is not intended to limit the present invention to Compounds A1 to A8.

### Example 1: Effect of AT2R Antagonists on AngII-Induced Reactive Oxygen Species Generation in Mouse Peritoneal Macrophages

### 1.1 Test Objective

Mouse peritoneal macrophages express angiotensin II type 2 receptors (AT2R). As a ligand for AT2R, angiotensin II (AngII) binds to AT2R and induces an increase of reactive oxygen species (ROS) produced in mouse peritoneal macrophages. This assay assesses whether AT2R antagonists inhibit AngII-induced ROS generation in mouse peritoneal macrophages by inhibiting AT2R on mouse peritoneal macrophages.

### 1.2 Instruments and Equipment

| **Name** | **Manufacturer / Model** |
|---|---|
| Electronic Balance | Sartorius (Germany) / MSA225S-1CE-DI |
| Vortex Mixer | Wiggens / QL-901 |
| -80°C Refrigerator | Thermo / 88500V |
| -20°C Refrigerator | Haier / BD-226W |
| -4°C Refrigerator | Haier / SC-350 |
| -4°C Refrigerator | Haier / SC-340 |
| Inverted Microscope | Zeiss / primover iLED |
| Cell Analyzer | Countstar / IC1000 |
| High-Content Analysis System | PerkinElmer / Opera Phenix |
| High-Speed Refrigerated Centrifuge | Thermo / Sorvall ST 8R |
| Intelligent Thermostatic Water Bath | Beijing Changfeng Instrument & Meter Co., Ltd. / HW.SY11-KP3 |
| Biological Safety Cabinet | Thermo / 1388 |
| CO₂ Incubator | Thermo / HERAcell150i |

### 1.3 Test Method

Six-week-old male C57BL/6 mice were euthanized. Primary mouse peritoneal macrophages were harvested by intraperitoneal injection of phenol red-free medium. After centrifugation, the macrophages were suspended in RPMI1640 + 5% FBS medium (containing 50 ng/µl GM-CSF) and cultured in a 37°C, 5% CO₂ incubator for 2 days. Thereafter, the macrophages were seeded into 96-well plates and allowed to adhere for 24 hours. After staining with the reactive oxygen species (ROS) indicator DCFH-DA, the macrophages were subjected to the following treatments: 1) Blank vehicle group; 2) AngII alone (0.2 µM) treatment group; 3) PD123319 combined with AngII group (PD (1 µM) + AngII (0.2 µM)): co-treatment with PD123319 and AngII for 0.5 hours; 4) A1 combined with AngII group (A1 (0.5 µM) + AngII (0.2 µM)): co-treatment with A1 and AngII for 0.5 hours; 5) A1 combined with AngII group (A1 (5 µM) + AngII (0.2 µM)): co-treatment with A1 and AngII for 0.5 hours. The ROS generation level of macrophages in each group was determined by real-time fluorescence scanning. 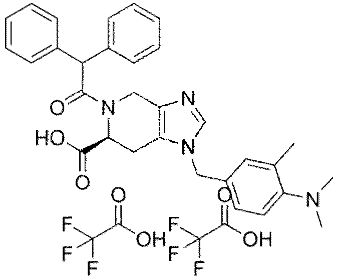 PD123319 di-trifluoroacetate (purchased from MCE, Batch No.: 14354)

### 1.4 Data Statistics

Data were collected using Excel software. Data were analyzed using Prism 6.01 (GraphPad Software, Inc.) software (one-way ANOVA). The experimental results of each group were statistically analyzed to compare statistical differences among groups, and p < 0.05 was considered statistically significant.

### 1.5 Experimental Results

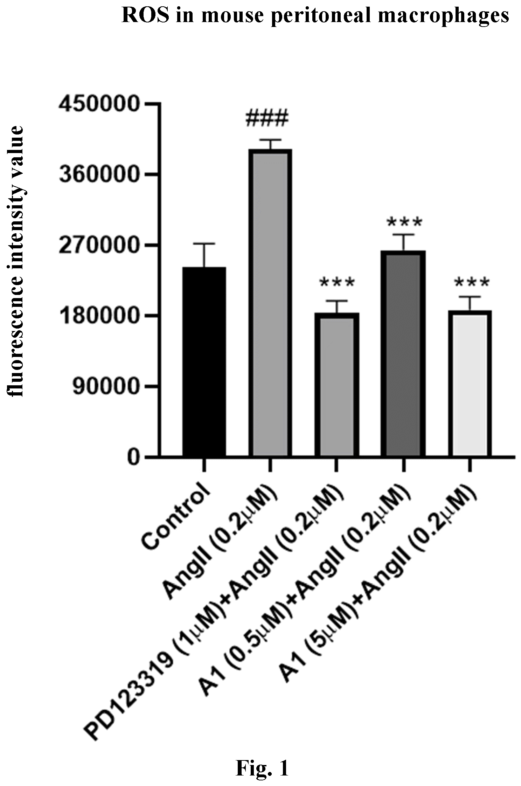

FIG. 1 shows the ROS fluorescence intensity of different treatment groups. ^{###}p < 0.001 vs. control group; ***p < 0.001 vs. AngII (0.2 µM) group. Compared with the blank vehicle control group, the level of reactive oxygen species generated by macrophages was markedly elevated following AngII treatment (p < 0.05). Compared with the AngII treatment group, the A1 (0.5 µM) + AngII (0.2 µM) group and the A1 (5 µM) + AngII (0.2 µM) group significantly attenuated the AngII-induced increase of reactive oxygen species (p < 0.05). Accordingly, A1 at both concentrations of 0.5 µM and 5 µM was capable of inhibiting AngII-induced increase of reactive oxygen species.

### Example 2: Pharmacodynamic Test of AT2R Antagonists on a Rat Diabetic Foot Pain Model Induced by High-Fat and High-Sugar Diet Combined with STZ

### 2.1 Test Objective

This test aims to establish a rat diabetic foot pain model by feeding a high-fat and high-sugar diet combined with intraperitoneal injection of streptozotocin (STZ), and to investigate the alleviating effect of AT2R antagonists on diabetes-induced peripheral neuralgia in rats.

### 2.2 Test Equipment

| **No.** | **Name** | **Manufacturer** | **Location** | **Model** | **Equipment ID** |
|---|---|---|---|---|---|
| 1 | Pharmaceutical Refrigerator | Zhongke Meiling | Room 1206, Test Article Storage Area D3 | YC-520L | EMO-08-07 |
| 2 | Medical -20°C Refrigerator | Panasonic | Room 1206, Test Article Storage Area D3 | MDF-339 | EMO-08-11 |
| 3 | 0.00001 g Analytical Balance | Sartorius | Room 1215, Balance Room D3 | Secura 125-1CN | EMO-10-07 |
| 4 | 0.01g Precision Balance | Sartorius | Second Floor, Animal Feeding Area D4 | HY2002T | EMO-10-10 |

### 2.3 Test Animals

Species: SD rats;
Grade: SPF grade;
Number and sex of animals purchased: 220, all male;
Number and sex of enrolled animals: 70, all male;
Source: Beijing Vital River Laboratory Animal Technology Co., Ltd.;
License No.: SCXK (Jing) 2016-0006;
Certificate No.: 110011211105511316;
IACUC approval No.: IACUC-202104-r-001.

### 2.4 Test Method

Rats were acclimated for one week. After being fed a high-fat and high-sugar diet for 4 weeks, plantar pain testing was performed once using Von Frey filaments in rats, and rats with a value higher than 10 g were screened out. The rats were fasted for 16 hours with free access to water, followed by a single intraperitoneal injection of STZ at 40 mg/kg (for rats weighing 220-250 g) or 50 mg/kg (for rats weighing 180-220 g). After 72 hours, random blood glucose was detected. Blood glucose ≥16.7 mmol/L indicated successful establishment of diabetic model. Rats as successfully established diabetes models were subjected to plantar pain testing training using Von Frey filaments once a week. After 4 or 5 weeks, rats whose 50% PWT fell within the range of 1-6 g were selected as diabetic foot pain model animals. The rats were randomly divided into 7 groups based on the pre-dosing pain threshold: vehicle control (ddH₂O), gabapentin 100 mg/kg, A1 (12.5, 25, 50, 100, and 200 mg/kg), with 10 rats per group. The administration volume was 10 mL/kg, and all test articles were given via oral gavage. Pain testing was performed using Von Frey filaments at 2, 4, and 6 hours after dosing, and pharmacodynamic efficacy was evaluated based on the change in 50% PWT of the rats.

The formula for calculating the 50% PWT is as follows: 50% g threshold = (10^{[Xf + kδ]}) / 10000
where X_{f} = log value of the last Von Frey nylon filament used in testing; k = value corresponding to positive/negative response patterns; δ = log value of the mean difference among nylon filament stimuli, a constant equal to 0.224 in this study.

### 2.5 Statistical Analysis

Experimental data were statistically analyzed using Excel and GraphPad Prism 8.

All measurement data were expressed as mean + SEM. GraphPad Prism 8 was used to plot the parameters of animals in each group before and after administration. Two-way ANOVA was used to analyze and compare the differences in 50% PWT values between different treatment groups and between different pain testing time points within the same group.

### 2.5 Experimental Results

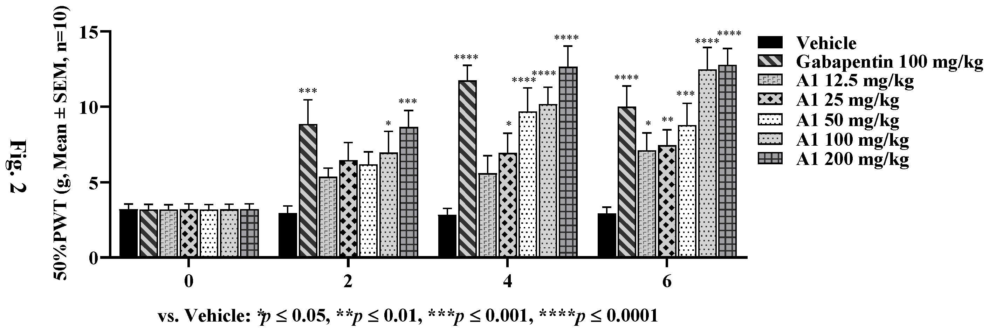

FIG. 2 shows the changes in 50% PWT of animals in each group at different administration time points. The animals were randomly grouped according to their pain thresholds. At 0 hour before administration, the 50% PWT values were evenly distributed across all groups, with no significant difference. Compared with the vehicle control group, the gabapentin 100 mg/kg group, the A1 100 mg/kg group, and the A1 200 mg/kg group significantly alleviated diabetes-induced peripheral neuralgia at 2, 4, and 6 hours after administration; the A1 50 mg/kg group and the A1 25 mg/kg group significantly alleviated diabetes-induced peripheral neuralgia at 4 and 6 hours after administration; the A1 12.5 mg/kg group significantly alleviated diabetes-induced peripheral neuralgia at 6 hours after administration. Accordingly, within this experimental system, A1 at doses of 12.5, 25, 50, 100, and 200 mg/kg significantly alleviated peripheral neuralgia in rat models of diabetic foot pain, which were established by feeding a high-fat and high-sugar diet combined with intraperitoneal injection of STZ.

### Example 3: Pharmacodynamic Test of AT2R Antagonists in the Rat Spinal Nerve Ligation (SNL) Model

3.1 This test aims to evaluate the pharmacodynamic effects of AT2R antagonists in the SD rat spinal nerve ligation model via mechanical allodynia assay.

### 3.2 Test Equipment

| Name | Product No. / Model | Supplier / Manufacturer |
|---|---|---|
| Touch-Test Sensory Evaluator | 58011 | North Coast |
| Scalpel | 4-310 | Miltex |
| Ophthalmic Scissors | Y00030 | Shanghai JZ Medical Instruments Co., Ltd. |
| Ophthalmic Forceps | JD 1020 | Shanghai Medical Instruments (Group) Co., Ltd. |
| Glass Bead Sterilizer | steri 250 | Keller |

### 3.3 Test Animals

Species: Sprague-Dawley rats;
Grade: SPF grade;
Number and sex of enrolled animals: 100, male;
Source: Shanghai SLAC Laboratory Animal Co., Ltd.
License No.: SCXK (Hu) 2017-0005;
Certificate No.: 20170005023847;
IACUC protocol number: GP02-021-2019v1.0

### 3.4 Test Procedure

SD rats were fully anesthetized via intraperitoneal injection of sodium pentobarbital (50 mg/kg). The lumbar surgical region of each animal was shaved, and the skin was disinfected three times with iodophor and 70% ethanol. Surgery was initiated once the skin had dried, and aseptic operations were adopted throughout the surgical procedure. The left L5 and L6 spinal nerves were isolated and tightly ligated with 6-0 silk sutures. The wound was sutured to complete the establishment of the rat SNL model.

Starting on day 11 post-surgery, animals were acclimated to the experimental environment for 15 minutes per day over 3 consecutive days.

On Day 13 post-surgery, the baseline mechanical allodynia threshold of the rats was measured. Animals exhibiting no mechanical allodynia (paw withdrawal threshold > 5 g) and those with a paw withdrawal threshold below 0.5 g were excluded. The remaining rats were randomly divided into 10 groups according to their baseline paw withdrawal thresholds: vehicle control (ddH2O), Olodanrigan 200 mg/kg, A1 200 mg/kg, A2 200 mg/kg, A3 200 mg/kg, A4 200 mg/kg, A5 200 mg/kg, A6 200 mg/kg, A7 200 mg/kg, and A8 200 mg/kg, with 10 rats per group and an intragastric administration volume of 5 mL/kg.

On Day 14 post-surgery, mechanical allodynia tests were performed using Von Frey filaments at 0 hour before administration, as well as at 2, 4, and 6 hours after administration. Mechanical allodynia was quantified as the paw withdrawal threshold (PWT) in rat behavioral tests and was calculated using the formula below: $50 % response threshold \left(g\right) = \left({10}^{\left(Xf+kδ\right)}\right) / 10 , 000$ Xf = final filament value used in testing, k = tabular value (Chaplan et al. 1994, page 62), δ = mean difference.

### 3.5 Statistical Analysis

Data were collected using Excel software. Data were analyzed using Prism 6.01 (GraphPad Software, Inc.) (two-way ANOVA followed by Bonferroni multiple comparison test).

### 3.6 Test Results

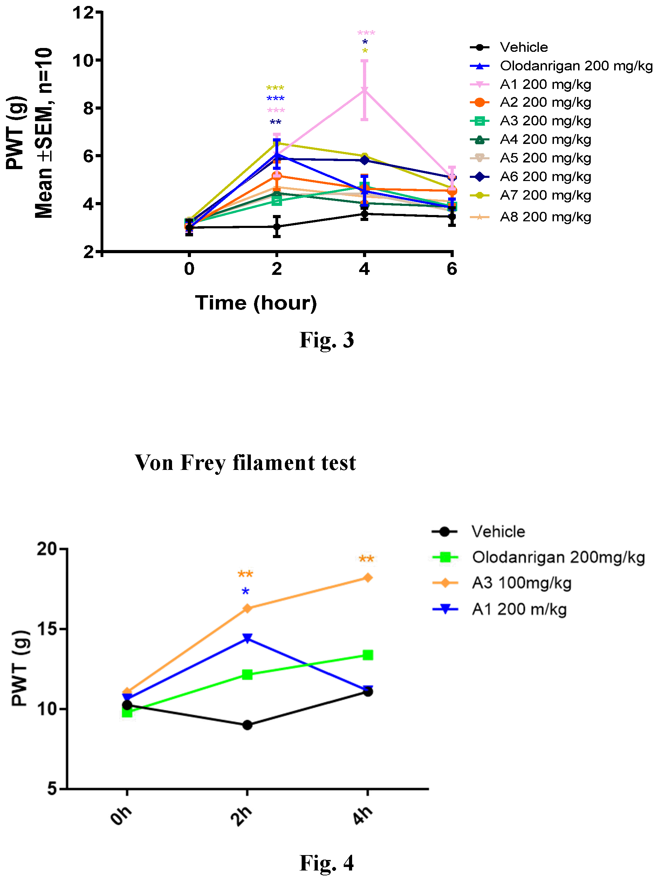

FIG. 3 shows the efficacy of AT2R antagonists in an allodynia test using a spinal nerve ligation model of SD rats. One day before administration, the baseline paw withdrawal threshold (PWT) for mechanical allodynia was measured in all model rats, and no significant differences in PWT values were observed among test groups. On Day 14 post-surgery following administration, compared with the vehicle control group, the Olodanrigan 200 mg/kg group significantly inhibited spinal nerve ligation-induced mechanical allodynia in rats at 2 h after administration; the A1 200 mg/kg group, the A6 200 mg/kg group, and the A7 200 mg/kg group significantly inhibited such allodynia at both 2 h and 4 h after administration.

### Example 4: Pharmacodynamic Test of AT2R Antagonists in the Rat Model of Chronic Constriction Injury (CCI) of the Sciatic Nerve

4.1 This test aims to assess the pharmacodynamic effects of AT2R antagonists in the SD rat model of chronic constriction injury of the sciatic nerve via mechanical allodynia assay.

### 4.2 Test Equipment

| Name | Product No. / Model | Supplier / Manufacturer |
|---|---|---|
| Touch-Test Sensory Evaluator | 58011 | North Coast |
| Scalpel | 4-310 | Miltex |
| Ophthalmic Scissors | Y00030 | Shanghai JZ Medical Instruments Co., Ltd. |
| Ophthalmic Forceps | JD1020 | Shanghai Medical Instruments (Group) Co., Ltd. |
| Glass Bead Sterilizer | steri 250 | Keller |

### 4.3 Test Animals

Species: Sprague-Dawley rats;
Grade: SPF grade;
Number and sex of enrolled animals: 32, male;
Source: Shanghai SLAC Laboratory Animal Co., Ltd.
License No.: SCXK (Hu) 2017-0005;
Certificate No.: 20170005025163;
IACUC protocol number: GP02-021-2019v1.0

### 4.4 Test Procedure

SD rats were fully anesthetized via intraperitoneal injection of sodium pentobarbital (50 mg/kg). The lumbar surgical region of each animal was shaved, and the skin was disinfected three times with iodophor and 70% ethanol. Surgery was initiated once the skin had dried, and aseptic operations were adopted throughout the surgical procedure. The left sciatic nerve was isolated, and four loose ligatures were tied approximately 7 mm upstream proximal to the nerve bifurcation with 4-0 chromic catgut sutures, spaced about 1 mm apart. The wound was sutured to complete the establishment of the rat CCI model.

Starting on day 11 post-surgery, animals were acclimated to the experimental environment for 15 minutes per day over 3 consecutive days.

On Day 13 post-surgery, the baseline mechanical allodynia threshold of the rats was measured. Animals exhibiting no mechanical allodynia (paw withdrawal threshold > 5 g) and those with a paw withdrawal threshold below 0.5 g were excluded. The remaining rats were randomly divided into 5 groups according to their baseline paw withdrawal thresholds: vehicle control (ddH2O), Olodanrigan 200 mg/kg, A3 100 mg/kg, and A 1200 mg/kg, with 8 rats per group and an intragastric administration volume of 5 mL/kg.

On Day 14 post-surgery, mechanical allodynia tests were performed using Von Frey filaments at 0 hour before administration, as well as at 2, 4, and 6 hours after administration. Mechanical allodynia was quantified as the paw withdrawal threshold (PWT) in rat behavioral tests and was calculated using the formula below: $50 % response threshold \left(g\right) = \left({10}^{\left(Xf+kδ\right)}\right) / 10 , 000$ Xf = final filament value used in testing, k = tabular value (Chaplan et al. 1994, page 62), δ = mean difference.

### 4.5 Statistical Analysis

Data were collected using Excel software. Data were analyzed using Prism 6.01 (GraphPad Software, Inc.) (two-way ANOVA followed by Bonferroni multiple comparison test).

### 4.6 Test Results

FIG. 4 shows the pharmacodynamic evaluation of AT2R antagonists in the rat model of chronic constriction injury of the sciatic nerve. Compared with the vehicle control group, the A3 100 mg/kg group significantly inhibited mechanical allodynia induced by sciatic nerve constriction in rats at both 2 h and 4 h after administration; the A1 200 mg/kg group significantly inhibited such allodynia at 2 h after administration. The Olodanrigan 200 mg/kg group failed to inhibit mechanical allodynia induced by sciatic nerve constriction in rats at either 2 h or 4 h after administration.

### Example 5: Pharmacodynamic Test of AT2R Antagonists in the Mouse Spared Nerve Injury Model

### 5.1 Test Objective

An Electronic von Frey Anesthesiometer was used to assess the analgesic effect of AT2R antagonists in the mouse spared nerve injury (SNI) model, and to study the effects of AT2R antagonists on the co-staining of AT2R and F4/80 in macrophages of the mouse SNI model, and on the expression of transient receptor potential ion channel subunit 1 (TRPA1) in dorsal root ganglion (DRG) neurons.

### 5.2 Test Instruments

Electronic von Frey Anesthesiometer (IITC, 2392); small animal anesthesia machine (R510-29); laser scanning confocal microscope (Zeiss, LSM710); Cryostat (ThermoFisher, NX70).

### 5.3 Test Animals

Strain: C57BL/6 mice
Age: 4-6 weeks old
Sex: male
Body weight: 14-20 grams
Number of animals purchased: 70
Experimental animal supplier: Hunan SJA Laboratory Animal Co., Ltd.
Production license number: SCXK (Xiang) 2019-0004

### 5.4 In Vivo Test Procedure

1) Mice were fully anesthetized via isoflurane inhalation and fixed in a prone position on an operating table to fully expose the lateral gluteal region. Hair on the left hind limb was shaved, and the skin was disinfected via an alcohol wipe. Aseptic operations were adopted throughout the surgical procedure. For the SNI surgery group: An incision was made parallel to the sciatic nerve along the femur to expose the sciatic nerve trunk until tibial nerve, common peroneal nerve, and sural nerve are visible. The tibial nerve and common peroneal nerve were tightly ligated with absorbable surgical suture (8-0). Then, a segment of nerve (approximately 2-4 mm) was transected between the ligation site of the tibial nerve and common peroneal nerve and the distal nerve end, while the sural nerve was kept intact. Muscles and skin were sutured layer by layer to complete the establishment of the mouse SNI model.
2) Starting on day 9 post-surgery, animals were acclimated to the experimental environment for 15 minutes per day over 3 consecutive days.
3) PWT measurement was performed on day 11 post-surgery. Animals with abnormal PWT (mean PWT > 4 g or < 1 g) were excluded. The remaining mice were randomly divided into 5 groups (SNI model groups) according to their body weights and PWT values: vehicle control (sterile water for injection), Gabapentin 50 mg/kg, A1 50 mg/kg, A1 100 mg/kg, and A1 200 mg/kg, with 12 mice per group and an intragastric administration volume of 10 mL/kg.
4) On day 12 post-surgery, paw withdrawal thresholds were measured using an Electronic von Frey Anesthesiometer at 0 h before administration, 2 h after administration, and 4 h after administration. Animals were placed in special multi-unit metal mesh cages for pain detection and acclimated to the environment for 30 minutes. After full acclimation to the test environment, the plantar surface of the operated-side mouse hind paw was stimulated using an Electronic von Frey Anesthesiometer (IITC Life Science Inc.). Pressure was increased continuously until the mice showed an obvious paw withdrawal response. The reading recorded by the instrument at this moment was defined as the mechanical pain reaction threshold in grams. The test was repeated 6 times. The average was used as the final detection index.

### 5.5 Tissue Harvesting and Immunofluorescence Staining Procedures

1) After pain measurement, the first six mice from the vehicle group, the A 150 mg/kg group, the A1 100 mg/kg group, and the A1 200 mg/kg group were anesthetized via isoflurane inhalation using a small animal anesthesia machine (R510-29), followed by euthanasia via cervical dislocation. The injured-side and uninjured-side sciatic nerves as well as the injured-side DRGs were isolated and fixed in 4% paraformaldehyde solution.
2) The fixed injured-side sciatic nerves, uninjured-side sciatic nerves, and injured-side dorsal root ganglia were subjected to tissue dehydration, frozen embedding, and sectioning for later use.
3) The immunofluorescence staining procedure was performed as follows:

The tissue sections were washed three times with PBS for 5 min each, incubated in 0.3% Triton-X100 in PBS for 20 min, then washed three times with PBS for 5 min each. The sections were blocked with goat serum at room temperature for 1 h.

Then, primary antibodies (AT2R: Rabbit (LSBio, LS-A1322); TRPA1: Rabbit (Invitrogen, PA1-46159); F4/80: Rat (Abcam, ab6640)) were diluted at a ratio of 1:200 with antibody diluent (NCM Biotech, Lot: 20220824) and incubated with the sections overnight at 4°C. After incubation, the sections were washed three times with PBS for 5 min each.

Thereafter, secondary antibodies (Goat Anti-Rabbit (Alexa Fluor^{®} 594) (Abcam, ab150080); Goat Anti-Rabbit (Alexa Fluor^{®} 594) (Abcam, ab150080); Goat Anti-Rat (Alexa Fluor^{®} 488) (Abcam, ab150165)) were diluted at a ratio of 1:2000 with the antibody diluent (NCM Biotech, Lot: 20220824) and incubated with the sections at room temperature for 30 min. After incubation, the sections were washed three times with PBS for 5 min each.

The sections were mounted with DAPI-containing mounting medium and imaged using a confocal microscope (Leica Microsystems, DCM8). The Find Maxima function in Image J was applied to count macrophages co-stained for AT2R and F4/80 in sciatic nerve tissues. Image J was also used to quantify TRPA1 fluorescence intensity in DRG neurons.

### 4) Immunohistochemistry

The sections were taken from a -80°C refrigerator, fixed in 4% paraformaldehyde at 4°C for 10 minutes, and rinsed three times with 1× PBS. Endogenous peroxidase activity was blocked with specific blocking buffer for 10 minutes, followed by three additional washes with 1× PBS. Subsequently, the sections were blocked with normal goat serum solution for 30 minutes. They were incubated with primary antibodies overnight at 4°C, and with secondary antibodies at room temperature for 1 hour the next day. Finally, the sections were stained with AEC reagent and hematoxylin solution. Antibodies used for immunohistochemical staining included anti-TRPA1 antibody (orb374201, 1:2000, Biorbyt, UK), anti-TRPV1 antibody (orb645490, 1:2000, Biorbyt, UK), and anti-rabbit antibody (1:1000). Micrographs were captured using a Leica Microsystems microscope and processed using Image-Pro Plus software.

### 5.6 Data Statistics

The experimental results were expressed as mean ± standard deviation. Data of each group were statistically analyzed using GraphPad Prism 8.0 software package. One-way Analysis of Variance (One-way ANOVA) or Two-way Analysis of Variance (Two-way ANOVA) were adopted according to the experiments to compare statistical differences among groups, and p < 0.05 was considered statistically significant.

### 5.7 In Vivo Test Results

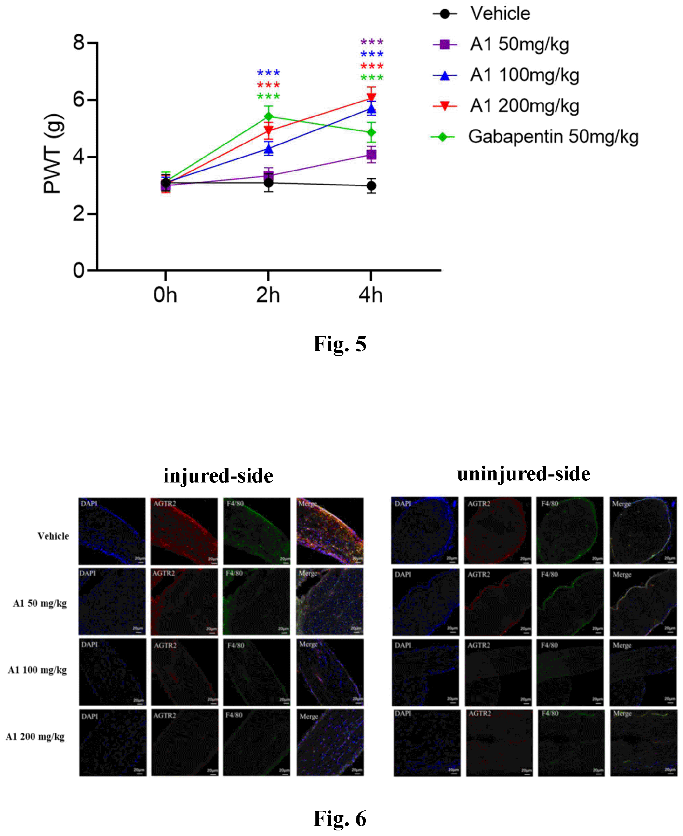

In SNI model mice, at 2 h and 4 h after intragastric administration, compared with the vehicle control group, the A 150 mg/kg group significantly inhibited sciatic nerve injury-induced mechanical allodynia in mice at 4 h after administration; the A1 100 mg/kg group significantly inhibited such allodynia at both 2 h and 4 h after administration; the A1 200 mg/kg group significantly inhibited such allodynia at both 2 h and 4 h after administration. The Gabapentin 50 mg/kg group inhibited sciatic nerve injury-induced mechanical allodynia in mice at both 2 h and 4 h after administration. FIG. 5 shows the paw withdrawal threshold (PWT) of SNI mice in each group (mean + SD, ***p < 0.001 vs. vehicle group at the corresponding time points).

### 5.8 Immunofluorescence Staining Results

FIG. 6 shows representative images of macrophages co-stained for AT2R (AKA: AGTR2) and F4/80 on the injured-side and uninjured-side sciatic nerves of SNI mice (Bar = 20 µm).

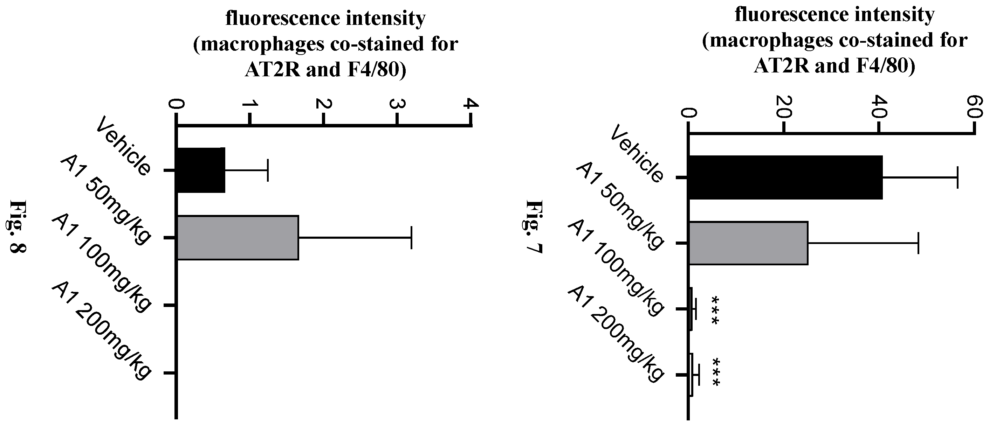

FIG. 7 shows the fluorescence intensity of macrophages co-stained for AT2R and F4/80 in the injured-side sciatic nerve of SNI mice. The results showed that compared with the vehicle control group, both the A1 100 mg/kg group and the A1 200 mg/kg group significantly reduced the number of macrophages co-stained for AT2R and F4/80 at the injury site of the sciatic nerve. FIG. 8 shows the fluorescence intensity of macrophages co-stained for AT2R and F4/80 in the uninjured-side sciatic nerve of SNI model mice. The results showed that the number of macrophages co-stained for AT2R and F4/80 in the uninjured-side sciatic nerve was relatively low in each experimental group, and there was no significant intergroup difference in the number of AT2R and F4/80 co-stained cells. These findings demonstrate that more peripheral macrophages are recruited to the injured-side sciatic nerve compared with the uninjured-side side. Intragastric administration of the AT2R antagonist A1 at 100 mg/kg and 200 mg/kg can significantly decrease the number of peripheral macrophages and the expression level of AT2R.

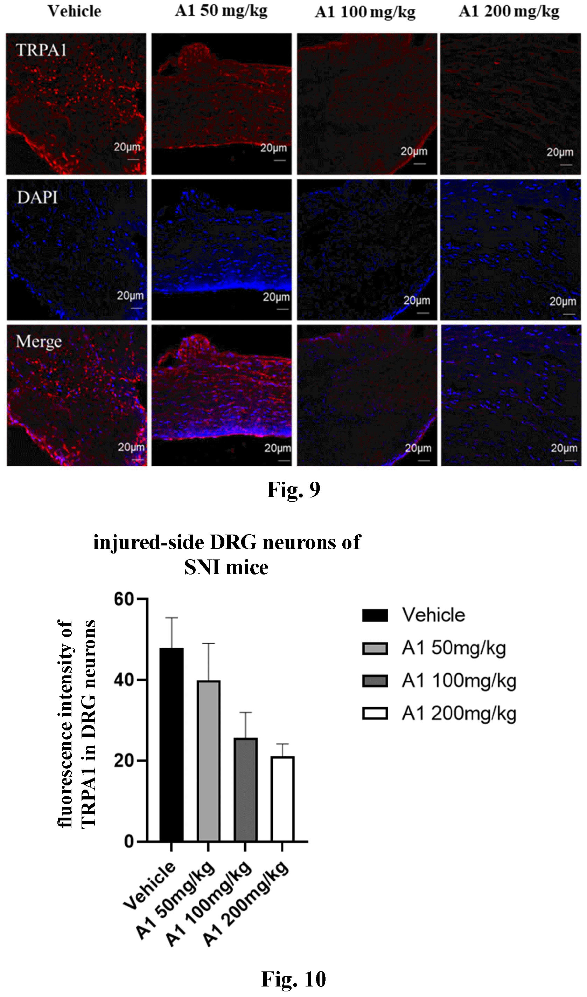

FIG. 9 shows representative images of TRPA1 immunofluorescence staining on the injured-side DRGs of SNI mice (Bar = 20 µm). FIG. 10 shows the mean fluorescence intensity of TRPA1 in the injured-side DRG neurons of SNI mice. The results showed that compared with the vehicle control group, the A1 100 mg/kg group and the A1 200 mg/kg group significantly decreased TRPA1 expression in DRG neurons.

### 5.9 Immunohistochemical Results

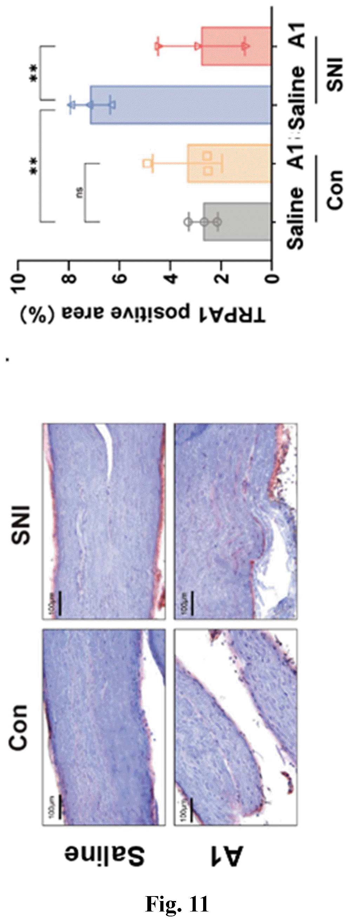

FIG. 11 shows a significant increase in TRPA1 expression in the injured-side sciatic nerve of SNI mice compared with normal control mice. In the SNI mouse model, compared with the saline-treated group, the A 1200 mg/kg treatment group showed a reduction in TRPA1 levels, nearly reaching the TRPA1 expression level in the normal control group.

### Example 6: Cellular Functional Assays

6.1 Test Objective: This assay aims to assess the effects of A1 on AT2R and neuronal ion channel expression at the cellular level.

### 6.2 Cell Culture

The macrophage cell line RAW264.7 was obtained from the Cell Resource Center, Peking Union Medical College, Beijing. The mouse hippocampal neuronal cell line HT22 (CL-0697) was kindly provided by Procell Life Science & Technology Co., Ltd.

RAW264.7 cells were cultured in DMEM supplemented with 10% FBS at 37°C. Experiments were initiated when the cell confluence reached 70%. After the RAW264.7 cells were subjected to different groups of treatments, the supernatants were collected and centrifuged at 1000 × g and 4°C to remove cell pellets. The obtained supernatants were added to HT22 cells in DMEM supplemented with 10% FBS, followed by co-cultivation at 37°C.

### 6.3 Experimental Methods

Detection of AT2R Expression: When the confluence of RAW264.7 cells reached 70%, AngII (10, 30, 50, 100, and 200 nM) was added. The negative control group was treated with an equal volume of saline. After 24 h of treatment, the cells were collected for protein and RNA extraction to assess AT2R expression levels.

Assay for the Inhibitory Effect of A1 on AT2R: When the confluence of RAW264.7 cells reached 70%, a fixed concentration of AngII and serial concentrations of A1 (10, 30, 50, 100, and 200 nM) were added. The negative control group was treated with an equal volume of saline (without AngII). After 24 h of treatment, the cells were collected for protein and RNA extraction to assess AT2R expression levels. Furthermore, an appropriate concentration was selected. When the confluence of RAW264.7 cells reached 70%, a fixed concentration of AngII and fixed concentrations of A1, PD123319, and Olodanrigan were added. The negative control group was treated with an equal volume of saline (without AngII). After 24 h of treatment, the cells were collected for protein and RNA extraction to assess AT2R expression levels.

ELISA: After RAW264.7 cells were treated with saline (negative control group), AngII, AngII + A1, AngII + PD123319, or AngII + Olodanrigan for 24 h, the supernatants were collected and centrifuged at 1000 × g and 4°C to remove cell pellets. The sample concentrations were determined using ELISA kits at a wavelength of 450 nm. The centrifuged supernatants were used to detect the concentrations of tumor necrosis factor TNF-α (Cat. No. ml002095, Shanghai Enzyme-linked Biotechnology, China), IL-1 (Cat. No. ml001816, Shanghai Enzyme-linked Biotechnology, China), 5-hydroxytryptamine (5-HT) (Cat. No. ml001891, Shanghai Enzyme-linked Biotechnology, China), IL-8 (Cat. No. ml063162, Shanghai Enzyme-linked Biotechnology, China), leukotriene (LT) (Cat. No. ml057907, Shanghai Enzyme-linked Biotechnology, China), and bradykinin (BK) (Cat. No. ml063348, Shanghai Enzyme-linked Biotechnology, China).

Western Blot (WB): 1) RAW264.7 and HT22 cells subjected to different groups of treatments were lysed with lysis buffer (containing 10 µL PMSF (100×) and 10 µL phosphatase inhibitor per milliliter of lysis buffer) for protein extraction. 2) Cell lysis was performed on ice for 30 minutes, followed by centrifugation at 12,000 rpm for 10 minutes at 4°C, and total protein was collected. 3) The protein concentration in lysates was quantified using the BCA Protein Assay Kit (Thermo Fisher Scientific). Equal amounts of protein were separated by sodium dodecyl sulfate-polyacrylamide gel electrophoresis (SDS-PAGE) and then transferred to PVDF membranes. 4) The membranes were blocked with 5% skim milk at room temperature for 1 hour, and then incubated with primary antibodies overnight at 4°C. 5) Following incubation, the membranes were washed with TBST and then incubated with horseradish peroxidase (HRP)-conjugated goat anti-mouse IgG (dilution 1:10000; Zsbio, Beijing, China) or goat anti-rabbit IgG (dilution 1: 10000; Zsbio) at room temperature for another 1 hour. 6) Signal detection was performed using an enhanced chemiluminescence (ECL) system, and image analysis was conducted using Image-Pro Plus version 6.0 software.

Real-Time Fluorescent PCR (RT-PCR): RNA was extracted from RAW264.7 and HT22 cells subjected to different groups of treatments using Trizol (Invitrogen, 15596-026). The extracted RNA was reverse-transcribed into cDNA using the FastKing cDNA First-Strand Synthesis Kit (Tiangen, KR116). The iQ5^{™} Multicolor Real-Time PCR Detection System (Bio-Rad, Hercules, CA, USA) was used to determine the differences in mRNA levels, with GAPDH used as the reference gene.

RT-PCR System and Conditions: 10 µL 2X TransStart Green qPCR SuperMix (TransGen Biotech, Beijing, China), 3 µL cDNA template, 2 µL primers (1 µmol/L), and 5 µL ddH₂O (nuclease-free). The RT-PCR program consisted of initial denaturation at 95°C for 2 minutes, followed by 40 amplification cycles: denaturation at 95°C for 15 seconds and annealing/extension at 60°C for 30 seconds.

### 6.4 CCK-8 Assay

RAW264.7 and HT22 cells in logarithmic growth phase were collected, digested with trypsin, and centrifuged to prepare single-cell suspensions. The cell number in the suspension was measured using a hemocytometer. Subsequently, the cell suspension was seeded into 96-well plates at a density of 2×10³ cells per well. At 0, 12, 24, and 48 hours post-stimulation, 10 µL of CCK-8 solution was supplemented to each well, followed by incubation at 37°C for 1 to 4 hours. Finally, the absorbance was determined at 450 nm with a spectrophotometer. 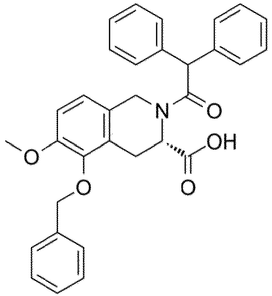 Olodanrigan, supplier: MCE, Cat. No.: HY-13106

### 6.5 Statistical Analysis

Statistical analysis was performed using GraphPad Prism 8 (GraphPad Software Inc., La Jolla, CA). All data were expressed as mean + SD. Normal and log-normal distributions were determined in all experiments. When the data conformed to a normal distribution, two-way ANOVA was used to calculate statistical significance. Otherwise, the t-test was adopted for statistical significance. P < 0.05 was considered statistically significant.

### 6.6 Results

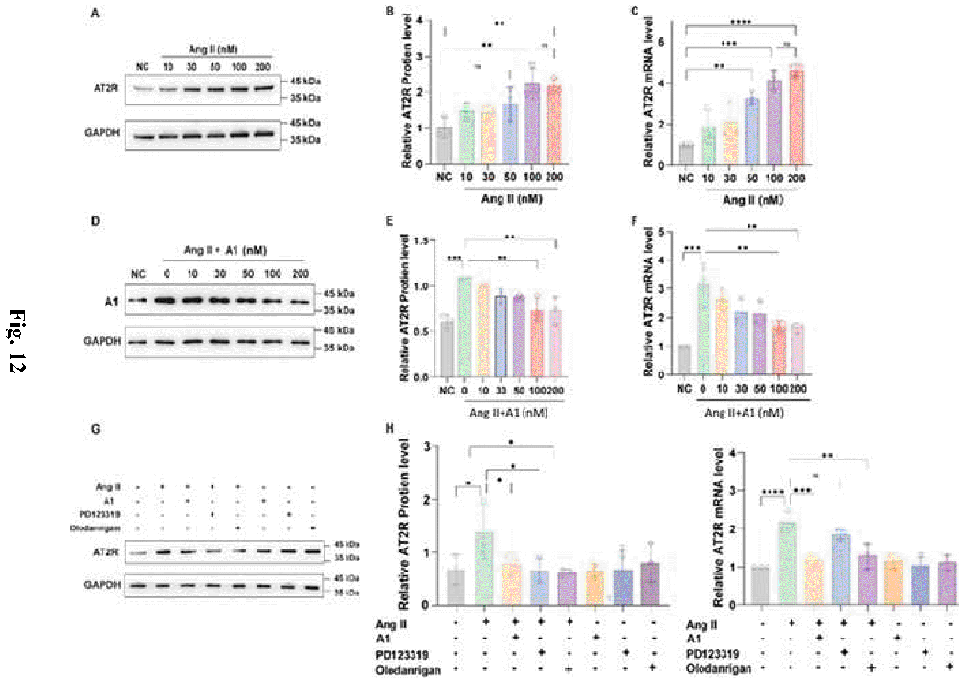

FIG. 12 shows the inhibitory effect of A1 on AngII-activated AT2R in RAW264.7 cells, indicating that A1 significantly inhibits the expression of AT2R. WB and RT-PCR in A, B, and C of FIG. 12 showed that compared with the negative control, AngII elevated the protein and mRNA levels of AT2R in RAW264.7 cells in a concentration-dependent manner at 10, 30, 50, 100, and 200 nM. WB and RT-PCR in D, E, and F of FIG. 12 showed that compared with the negative control, AngII treatment increased AT2R expression, whereas A1 inhibited AT2R expression in RAW264.7 cells in a dose-dependent manner at concentrations of 10, 30, 50, 100, and 200 nM. WB and RT-PCR in G, H, and I of FIG. 12 showed that compared with the negative control, AngII treatment increased AT2R expression, and treatment with A1, PD123319, or Olodanrigan each inhibited AT2R expression.

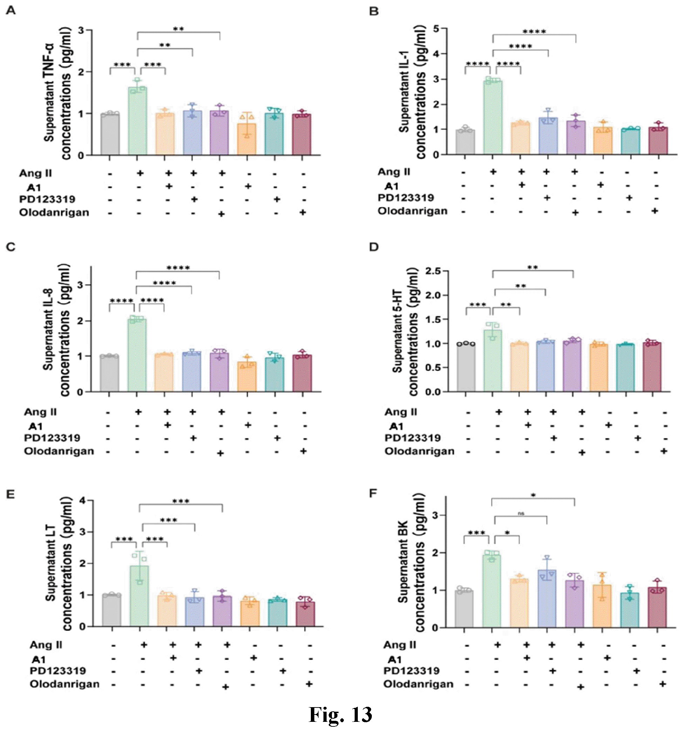

FIG. 13 shows that A1 reduced the AngII-induced elevation of inflammatory factors in the supernatant of RAW264.7 cells. The results showed that A1 reduced the elevation of cellular inflammatory factors mediated by AngII-stimulated RAW264.7 cells, including TNF-α, IL-1, 5-HT, IL-8, LT, and BK.

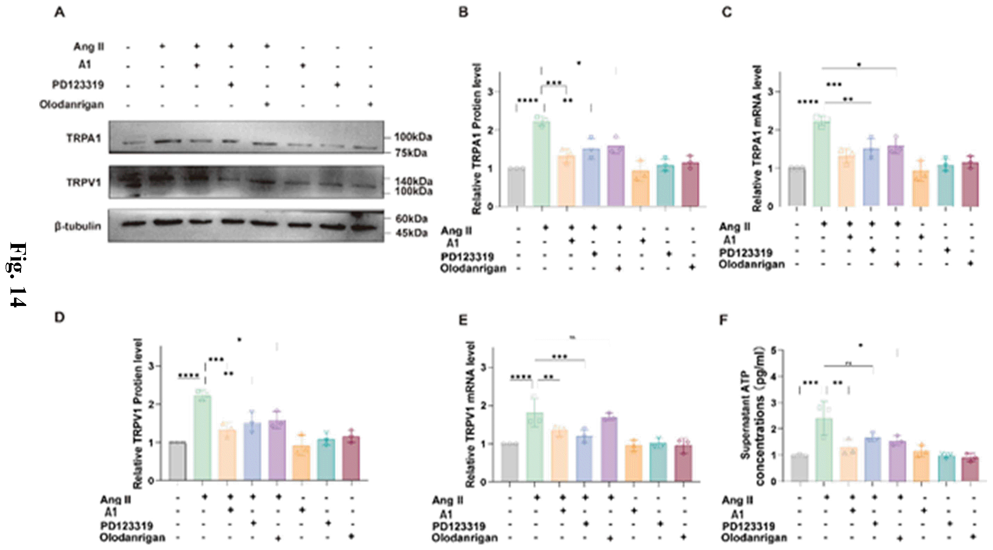

FIG. 14 shows the inhibitory effects of A1 on the protein expression and mRNA transcription of TRPA1 and TRPV1. FIG. 14A shows the Western blot of HT22 neuron cell lysates (TRPA1 and TRPV1); FIGS. 14B and 14C show the quantitative analysis of TRPA1 protein expression and mRNA transcript levels across different treatment groups; FIGS. 14D and 14E show the quantitative analyses of TRPV1 protein expression and mRNA transcript levels across different treatment groups; FIG. 14F shows the concentration of ATP in the supernatants of HT22 neurons across different treatment groups. *p < 0.05, ***p < 0.001, ****p < 0.0001. The results showed that under the co-incubation conditions of HT22 neurons with the supernatant from RAW264.7 macrophages, A1 treatment reduced the protein expression and mRNA transcription levels of TRPA1 and TRPV1.

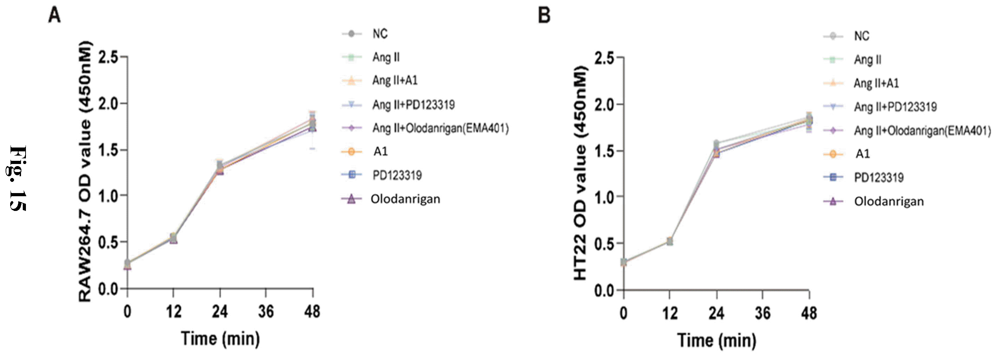

FIG. 15 shows the results of *in vitro* cytotoxicity assays, demonstrating that A1 exhibits no cytotoxicity toward RAW264.7 and HT22 cells and does not suppress neuronal viability.

In addition to the embodiments described herein, various modifications to the present invention will be apparent to those skilled in the art from the above-described description. Such modifications are also intended to fall within the scope of the appended claims. Each reference (including all patents, patent applications, journal articles, books, and any other publications) cited in the present application is incorporated herein by reference in its entirety.

## Claims

1. A compound, or a pharmaceutically acceptable salt, ester, stereoisomer, polymorph, solvate, N-oxide, isotopically labeled compound, metabolite, or prodrug thereof, wherein the compound has the structure of Formula (IV): 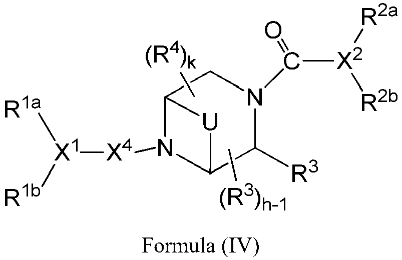 wherein:
U is C₁₋₃ alkylene;
R^{1a} is selected from the group consisting of C₂₋₈ alkenyl and C₂₋₈ alkynyl, wherein the C₂₋₈ alkenyl and C₂₋₈ alkynyl are each substituted with one C₆₋₁₀ aryl or 5- to 14-membered heteroaryl; C₆₋₁₀ aryl; -C₁₋₆ alkylene-saturated or partially unsaturated C₃₋₁₀ cyclic hydrocarbyl; -C₁₋₆ alkylene-saturated or partially unsaturated 3- to 10-membered heterocyclyl; -C₁₋₆ alkylene-C₆₋₁₀ aryl; and -C₁₋₆ alkylene-(5- to 14-membered heteroaryl);
R^{1b} is absent, or is selected from the group consisting of H; C₁₋₈ alkyl optionally substituted with 1, 2, 3, or more R¹³ groups; saturated or partially unsaturated C₃₋₁₀ cyclic hydrocarbyl; C₆₋₁₀ aryl; -C₁₋₆ alkylene-saturated or partially unsaturated C₃₋₁₀ cyclic hydrocarbyl; and -C₁₋₆ alkylene-C₆₋₁₀ aryl;
X¹ is absent, or is CR¹⁰ or N;
X⁴ is selected from the group consisting of C(=O); -O-C(=O)- and -S-C(=O)-, wherein the O and S are attached to X¹;
R^{2a} is C₆₋₁₀ aryl;
R^{2b} is C₆₋₁₀ aryl;
X² is CR¹⁰ or N;
R³ is -C(=O)OR¹¹;
R⁴ is H;
R¹⁰ is, at each occurrence, selected from the group consisting of H, -OR¹¹, -SR¹¹, and C₁₋₆ alkyl;
R¹¹ and R¹² are, at each occurrence, independently H or C₁₋₆ alkyl;
h and k are each independently 1;
the alkylene, cyclic hydrocarbyl, heterocyclyl, aryl and heteroaryl groups recited above are, at each occurrence, optionally substituted with 1, 2, 3, or more R¹³ groups;
R¹³ is, at each occurrence, independently selected from the group consisting of halogen, cyano, nitro, C₁₋₆ alkyl, C₂₋₆ alkenyl, C₂₋₆ alkynyl, C₃₋₁₀ cyclic hydrocarbyl, C₆₋₁₀ aryl, 5- to 14-membered heteroaryl, -OR¹¹, -SR¹¹, - P(O)R¹¹R¹², and -NR¹¹R¹²; wherein the alkyl, alkylene, aryl and heteroaryl groups recited for substituent R¹³ are each optionally further substituted with 1, 2, 3, or more substituents independently selected from halogen and C₁₋₆ alkyl.

2. The compound according to claim 1, or a pharmaceutically acceptable salt, ester, stereoisomer, polymorph, solvate, N-oxide, isotopically labeled compound, metabolite, or prodrug thereof, wherein
R¹³ is, at each occurrence, independently selected from the group consisting of halogen, cyano, nitro, C₁₋₆ alkyl, C₃₋₇ cyclic hydrocarbyl, C₆₋₁₀ aryl, 5- to 14-membered heteroaryl, -OR¹¹, -SR¹¹, and -NR¹¹R¹²; wherein the alkyl, aryl and heteroaryl groups recited for substituent R¹³ are each optionally further substituted with 1, 2, 3, or more substituents independently selected from halogen and C₁₋₆ alkyl.

3. The compound according to claim 1, or a pharmaceutically acceptable salt, ester, stereoisomer, polymorph, solvate, N-oxide, isotopically labeled compound, metabolite, or prodrug thereof, wherein U is methylene or ethylene.

4. The compound according to claim 1, or a pharmaceutically acceptable salt, ester, stereoisomer, polymorph, solvate, N-oxide, isotopically labeled compound, metabolite, or prodrug thereof, wherein R³ is -COOH.

5. The compound according to claim 1, or a pharmaceutically acceptable salt, ester, stereoisomer, polymorph, solvate, N-oxide, isotopically labeled compound, metabolite, or prodrug thereof, wherein R¹⁰ is, at each occurrence, independently H, C₁₋₄ alkyl, OH, or SH.

6. The compound according to claim 1, or a pharmaceutically acceptable salt, ester, stereoisomer, polymorph, solvate, N-oxide, isotopically labeled compound, metabolite, or prodrug thereof, wherein R¹¹ and R¹² are, at each occurrence, independently selected from the group consisting of H and C₁₋₄ alkyl.

7. The compound according to claim 1, or a pharmaceutically acceptable salt, ester, stereoisomer, polymorph, solvate, N-oxide, isotopically labeled compound, metabolite, or prodrug thereof, wherein R¹³ is, at each occurrence, independently selected from the group consisting of F, Cl, Br, I, amino, cyano, nitro; C₁₋₄ alkyl optionally substituted with 1, 2, 3, or more substituents independently selected from halogen; C₅₋₇ cyclic hydrocarbyl; phenyl, 5- to 6-membered heteroaryl, and 9- to 10-membered heteroaryl, each optionally substituted with 1, 2, 3, or more substituents independently selected from halogen, OH, amino, cyano and C₁₋₄ alkyl; -OR¹¹ wherein R¹¹ is C₁₋₆ alkyl optionally substituted with 1, 2, 3, or more halogens; -SR¹¹ wherein R¹¹ is C₁₋₆ alkyl optionally substituted with 1, 2, 3, or more halogens; and -NR¹¹R¹² or -P(O)R¹¹R¹² wherein R¹¹ and R¹² are, at each occurrence, independently C₁₋₆ alkyl optionally substituted with 1, 2, 3, or more halogens.

8. The compound according to claim 1, or a pharmaceutically acceptable salt, ester, stereoisomer, polymorph, solvate, N-oxide, isotopically labeled compound, metabolite, or prodrug thereof, wherein R¹³ is, at each occurrence, independently selected from the group consisting of F, Cl, Br, I, amino, cyano, nitro; C₁₋₄ alkyl optionally substituted with 1, 2, or 3 F or Cl; -OR¹¹ wherein R¹¹ is C₁₋₃ alkyl optionally substituted with 1, 2, or 3 F or Cl; -SR¹¹ wherein R¹¹ is C₁₋₃ alkyl optionally substituted with 1, 2, or 3 F or Cl; -NR¹¹R¹² or -P(O)R¹¹R¹² wherein R¹¹ and R¹² are, at each occurrence, independently C₁₋₃ alkyl; and phenyl, 5- to 6-membered heteroaryl, and 9- to 10-membered heteroaryl, each optionally substituted with 1, 2, 3, or more substituents independently selected from F, Cl, Br, I, and methyl.

9. The compound according to claim 8, or a pharmaceutically acceptable salt, ester, stereoisomer, polymorph, solvate, N-oxide, isotopically labeled compound, metabolite, or prodrug thereof, wherein the C₁₋₃ alkyl is methyl, ethyl, propyl, or isopropyl.

10. The compound according to claim 1, or a pharmaceutically acceptable salt, ester, stereoisomer, polymorph, solvate, N-oxide, isotopically labeled compound, metabolite, or prodrug thereof, wherein:
R^{1a} is selected from the group consisting of C₂₋₆ alkenyl and C₂₋₆ alkynyl, wherein the C₂₋₆ alkenyl and C₂₋₆ alkynyl are each substituted with one phenyl or 5- to 10-membered heteroaryl; phenyl; -C₁₋₃ alkylene-C₃₋₇ cyclic hydrocarbyl; -C₁₋₃ alkylene-(5- to 7-membered monocyclic heterocyclyl); -C₁₋₃ alkylene-(8- to 10-membered benzo-fused heterocyclyl); -C₁₋₃ alkylene-phenyl; and -C₁₋₃ alkylene-(5- to 10-membered heteroaryl);
R^{1b} is absent, or is selected from the group consisting of: H; C₁₋₆ alkyl; C₃₋₇ cyclic hydrocarbyl; phenyl; -C₁₋₃ alkylene-C₃₋₇ cyclic hydrocarbyl; and -C₁₋₃ alkylene-phenyl; and
the alkyl, alkylene, cyclic hydrocarbyl, heterocyclyl, aryl and heteroaryl groups recited above are, at each occurrence, optionally substituted with 1, 2, 3, or more R¹³ groups as defined in claim 1.

11. The compound according to claim 10, or a pharmaceutically acceptable salt, ester, stereoisomer, polymorph, solvate, N-oxide, isotopically labeled compound, metabolite, or prodrug thereof, wherein R^{1a} is selected from the group consisting of phenyl, -C₁₋₃ alkylene-C₃₋₇ cyclic hydrocarbyl, -C₁₋₃ alkylene-phenyl, -C₁₋₃ alkylene-(5- to 7-membered monocyclic heterocyclyl), -C₁₋₃ alkylene-(9- to 10-membered benzo-fused heterocyclyl), -C₁₋₃ alkylene-(5- to 6-membered heteroaryl), and -C₁₋₃ alkylene-(9- to 10-membered heteroaryl), each optionally substituted with 1, 2, 3, or more R¹³ groups.

12. The compound according to claim 11, or a pharmaceutically acceptable salt, ester, stereoisomer, polymorph, solvate, N-oxide, isotopically labeled compound, metabolite, or prodrug thereof, wherein R¹³ is selected from the group consisting of C₁₋₄ alkyl-O-; halogen; and C₁₋₄ alkyl optionally substituted with 1, 2, or 3 substituents independently selected from halogen.

13. The compound according to claim 11, or a pharmaceutically acceptable salt, ester, stereoisomer, polymorph, solvate, N-oxide, isotopically labeled compound, metabolite, or prodrug thereof, wherein X⁴ is C(=O).

14. The compound according to claim 10, or a pharmaceutically acceptable salt, ester, stereoisomer, polymorph, solvate, N-oxide, isotopically labeled compound, metabolite, or prodrug thereof, wherein
R^{1a} is selected from the group consisting of:
C₂₋₆ alkenyl and C₂₋₆ alkynyl, wherein the C₂₋₆ alkenyl and C₂₋₆ alkynyl are each substituted with one phenyl, 5- to 6-membered heteroaryl, or 9- to 10-membered heteroaryl, wherein the phenyl, 5- to 6-membered heteroaryl, and 9- to 10-membered heteroaryl are each optionally substituted with 1, 2, or 3 substituents independently selected from F, Cl, Br, I, and C₁₋₄ alkyl;
phenyl optionally substituted with 1, 2, or 3 substituents independently selected from F, Cl, Br, I, and C₁₋₄ alkyl; and
-C₁₋₃ alkylene-phenyl, -C₁₋₃ alkylene-(5- to 6-membered heteroaryl), and -C₁₋₃ alkylene-(9- to 10-membered heteroaryl), wherein the alkylene is, at each occurrence, optionally substituted with one - NR¹¹R¹², and the phenyl, 5- to 6-membered heteroaryl, and 9- to 10-membered heteroaryl are each optionally substituted with 1, 2, or 3 substituents independently selected from F, Cl, Br, I, and C₁₋₄ alkyl;
R^{1b} is absent;
X¹ is absent; and
X⁴ is C(=O) or -O-C(=O)-.

15. The compound according to claim 10, or a pharmaceutically acceptable salt, ester, stereoisomer, polymorph, solvate, N-oxide, isotopically labeled compound, metabolite, or prodrug thereof, wherein the C₁₋₆ alkyl is methyl, ethyl, propyl, isopropyl, or t-butyl.

16. The compound according to claim 14, or a pharmaceutically acceptable salt, ester, stereoisomer, polymorph, solvate, N-oxide, isotopically labeled compound, metabolite, or prodrug thereof, wherein the C₂₋₆ alkenyl is ethenyl, 1-propenyl, or 2-propenyl.

17. The compound according to claim 14, or a pharmaceutically acceptable salt, ester, stereoisomer, polymorph, solvate, N-oxide, isotopically labeled compound, metabolite, or prodrug thereof, wherein the C₂₋₆ alkynyl is ethynyl, 1-propynyl, or 2-propynyl.

18. The compound according to claim 14, or a pharmaceutically acceptable salt, ester, stereoisomer, polymorph, solvate, N-oxide, isotopically labeled compound, metabolite, or prodrug thereof, wherein R¹³ is phenyl, pyridyl, indolyl or furanyl, each optionally substituted with 1, 2, or 3 substituents independently selected from F, Cl, Br, and methyl.

19. The compound according to claim 18, or a pharmaceutically acceptable salt, ester, stereoisomer, polymorph, solvate, N-oxide, isotopically labeled compound, metabolite, or prodrug thereof, wherein R^{1a} is selected from the group consisting of: 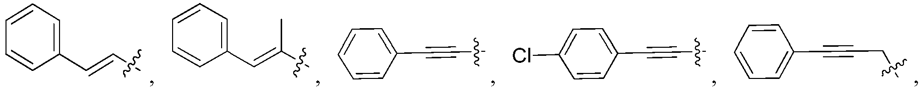 and

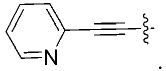

20. The compound according to claim 1, or a pharmaceutically acceptable salt, ester, stereoisomer, polymorph, solvate, N-oxide, isotopically labeled compound, metabolite, or prodrug thereof, wherein
R^{1a} is selected from the group consisting of: optionally substituted phenyl, -optionally substituted C₁₋₃ alkylene-(optionally substituted C₃₋₇ cyclic hydrocarbyl), -optionally substituted C₁₋₃ alkylene-(optionally substituted 5- to 7-membered monocyclic heterocyclyl), -optionally substituted C₁₋₃ alkylene-(optionally substituted 8- to 10-membered benzo-fused heterocyclyl), -optionally substituted C₁₋₃ alkylene-optionally substituted phenyl, and - optionally substituted C₁₋₃ alkylene-(optionally substituted 5- to 10-membered heteroaryl);
R^{1b} is selected from the group consisting of: H; C₁₋₈ alkyl optionally substituted with 1, 2, 3, or more R¹³ groups; saturated or partially unsaturated C₃₋₁₀ cyclic hydrocarbyl; C₆₋₁₀ aryl; -C₁₋₆ alkylene-saturated or partially unsaturated C₃₋₁₀ cyclic hydrocarbyl; and -C₁₋₆ alkylene-C₆₋₁₀ aryl;
X¹ is CR¹⁰ or N;
X⁴ is C(=O);
wherein "optionally substituted" means substituted with 1, 2, 3, or more R¹³ groups; and
R¹³ is as defined in claim 1.

21. The compound according to claim 20, or a pharmaceutically acceptable salt, ester, stereoisomer, polymorph, solvate, N-oxide, isotopically labeled compound, metabolite, or prodrug thereof, wherein R^{1b} is selected from the group consisting of H, optionally substituted C₁₋₄ alkyl, optionally substituted C₃₋₇ cyclic hydrocarbyl, optionally substituted phenyl, -optionally substituted C₁₋₃ alkylene-(optionally substituted C₃₋₇ cyclic hydrocarbyl), and - optionally substituted C₁₋₃ alkylene-optionally substituted phenyl;
wherein "optionally substituted" means substituted with 1, 2, 3, or more R¹³ groups.

22. The compound according to claim 1, or a pharmaceutically acceptable salt, ester, stereoisomer, polymorph, solvate, N-oxide, isotopically labeled compound, metabolite, or prodrug thereof, wherein
R^{2a} is optionally substituted phenyl; and/or
R^{2b} is optionally substituted phenyl;
wherein "optionally substituted" means substituted with 1, 2, 3, or more R¹³ groups.

23. The compound according to claim 1, or a pharmaceutically acceptable salt, ester, stereoisomer, polymorph, solvate, N-oxide, isotopically labeled compound, metabolite, or prodrug thereof, wherein the compound has the structure of Formula (II): 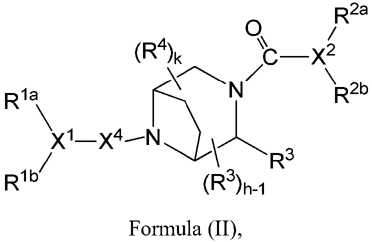 wherein R^{1a}, R^{1b}, X¹, X⁴, R^{2a}, R^{2b}, X², R³, R⁴, h, and k are as defined in claim 1.

24. The compound according to claim 1, or a pharmaceutically acceptable salt, ester, stereoisomer, polymorph, solvate, N-oxide, isotopically labeled compound, metabolite, or prodrug thereof, wherein 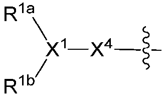 is:

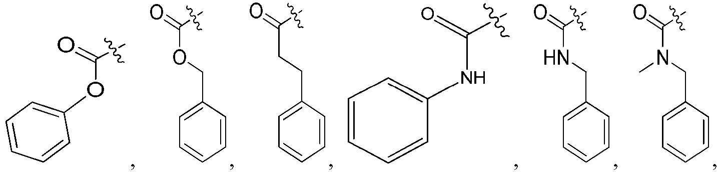

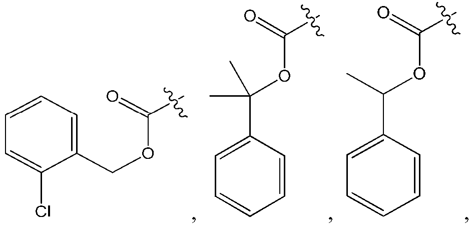

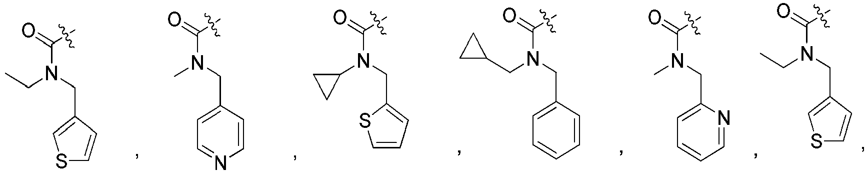

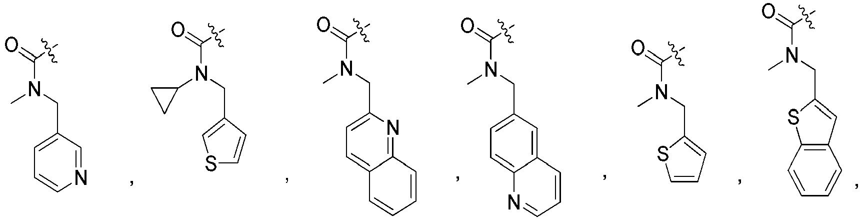

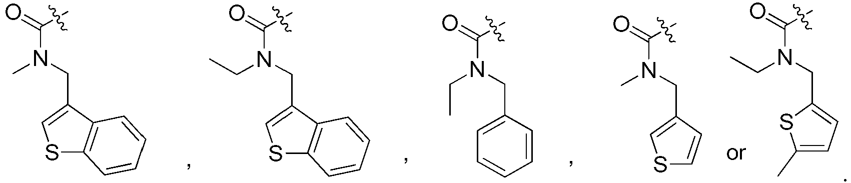

25. The compound according to claim 1, or a pharmaceutically acceptable salt, ester, stereoisomer, polymorph, solvate, N-oxide, isotopically labeled compound, metabolite, or prodrug thereof, wherein 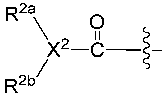 is:

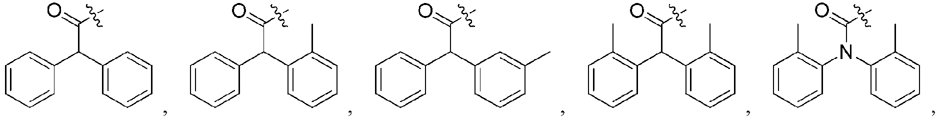

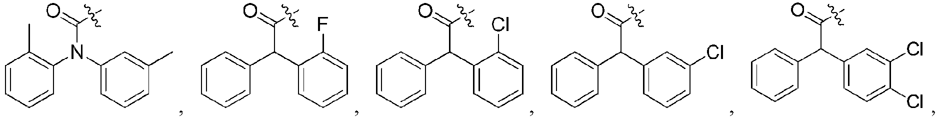

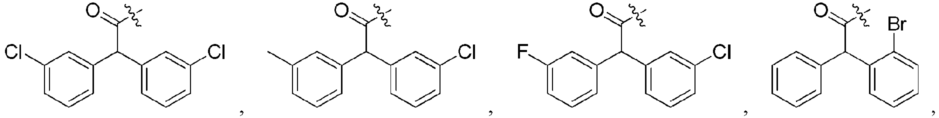

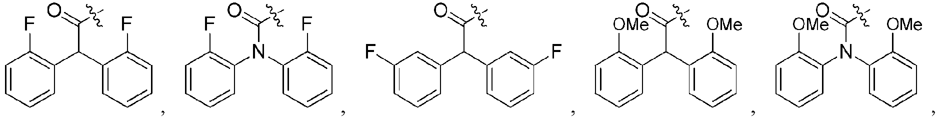

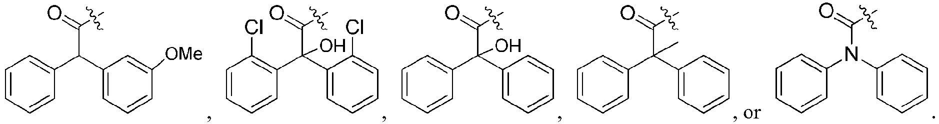

26. The compound according to claim 1, or a pharmaceutically acceptable salt, ester, stereoisomer, polymorph, solvate, N-oxide, isotopically labeled compound, metabolite, or prodrug thereof, wherein R¹⁰ is H or methyl.

27. The compound according to claim 20, or a pharmaceutically acceptable salt, ester, stereoisomer, polymorph, solvate, N-oxide, isotopically labeled compound, metabolite, or prodrug thereof, wherein R^{1a} is selected from the group consisting of optionally substituted phenyl, -C₁₋₃ alkylene-(optionally substituted C₃₋₇ cyclic hydrocarbyl), - C₁₋₃ alkylene-(optionally substituted 5- to 7-membered monocyclic heterocyclyl), -C₁₋₃ alkylene-(optionally substituted 8- to 10-membered benzo-fused heterocyclyl), -C₁₋₃ alkylene-optionally substituted phenyl, and -C₁₋₃ alkylene-(optionally substituted 5- to 10-membered heteroaryl).

28. The compound according to claim 20, or a pharmaceutically acceptable salt, ester, stereoisomer, polymorph, solvate, N-oxide, isotopically labeled compound, metabolite, or prodrug thereof, wherein R^{1a} is selected from the group consisting of:
optionally substituted phenyl;
-C₁₋₃ alkylene-(optionally substituted C₃₋₇ cyclic hydrocarbyl), wherein the cyclic hydrocarbyl is cyclopropyl, cyclobutyl, cyclopentyl, or cyclohexyl;
-C₁₋₃ alkylene-(optionally substituted 8- to 10-membered benzo-fused heterocyclyl), wherein the heterocyclyl is 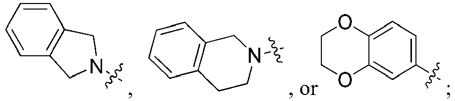
-C₁₋₃ alkylene-(optionally substituted 5- to 10-membered heteroaryl), wherein the heteroaryl is 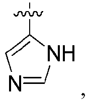 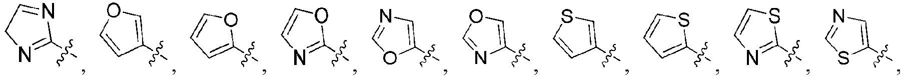 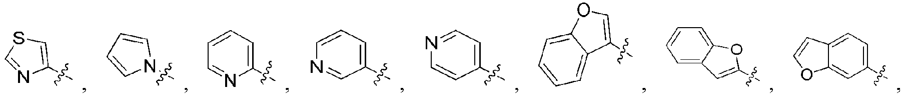 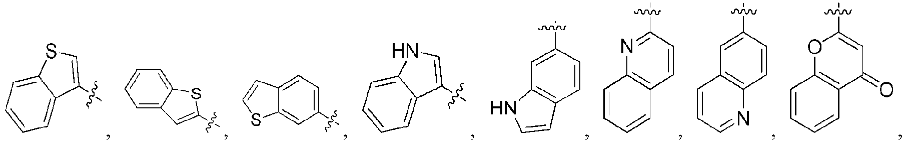 or

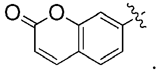

29. The compound according to claim 20, or a pharmaceutically acceptable salt, ester, stereoisomer, polymorph, solvate, N-oxide, isotopically labeled compound, metabolite, or prodrug thereof, wherein R¹³ is selected from the group consisting of: halogen; -OR¹¹ wherein R¹¹ is C₁₋₆ alkyl optionally substituted with 1, 2, 3, or more halogens; cyano; C₃₋₇ cyclic hydrocarbyl; C₁₋₄ alkyl, C₂₋₄ alkenyl, and C₂₋₄ alkynyl, each optionally substituted with 1, 2, 3, or more halogens; -NR¹¹R¹² wherein R¹¹ and R¹² are each independently selected from the group consisting of H and C₁₋₄ alkyl; and -P(O)R¹¹R¹² wherein R¹¹ and R¹² are each independently C₁₋₆ alkyl optionally substituted with 1, 2, 3, or more halogens.

30. The compound according to claim 20, or a pharmaceutically acceptable salt, ester, stereoisomer, polymorph, solvate, N-oxide, isotopically labeled compound, metabolite, or prodrug thereof, wherein R¹³ is selected from the group consisting of: halogen; -OR¹¹ wherein R¹¹ is C₁₋₃ alkyl optionally substituted with 1, 2, or 3 F or Cl; cyano; C₃₋₇ cyclic hydrocarbyl; C₁₋₄ alkyl, C₂₋₄ alkenyl, and C₂₋₄ alkynyl, each optionally substituted with 1, 2, 3, or more halogens; -NR¹¹R¹² wherein R¹¹ and R¹² are each independently selected from the group consisting of H and methyl; and -P(O)R¹¹R¹² wherein R¹¹ and R¹² are each independently C₁₋₃ alkyl optionally substituted with 1, 2, or 3 F or Cl.

31. The compound according to claim 20, or a pharmaceutically acceptable salt, ester, stereoisomer, polymorph, solvate, N-oxide, isotopically labeled compound, metabolite, or prodrug thereof, wherein R¹³ is selected from the group consisting of: F, Cl, Br, OH, -OC₁₋₄ alkyl, -N(C₁₋₄ alkyl)₂, cyano, C₃₋₇ cyclic hydrocarbyl, C₂₋₄ alkenyl, and C₂₋₄ alkynyl; C₁₋₄ alkyl optionally substituted with 1, 2, 3, or more F, Cl, or Br; and -P(O)R¹¹R¹² wherein R¹¹ and R¹² are each independently methyl, ethyl, propyl, or isopropyl.

32. The compound according to claim 20, or a pharmaceutically acceptable salt, ester, stereoisomer, polymorph, solvate, N-oxide, isotopically labeled compound, metabolite, or prodrug thereof, wherein R¹³ is selected from the group consisting of: F, Cl, Br, -OCH₃, -N(CH₃)₂, cyano, cyclopropyl, ethenyl, 1-propenyl, 2-propenyl, ethynyl, 1-propynyl, 2-propynyl, methyl, ethyl, n-propyl, isopropyl, tert-butyl, and CF₃; and -P(O)R¹¹R¹² wherein R¹¹ and R¹² are each independently methyl.

33. The compound according to claim 20, or a pharmaceutically acceptable salt, ester, stereoisomer, polymorph, solvate, N-oxide, isotopically labeled compound, metabolite, or prodrug thereof, wherein R^{1a} is selected from the group consisting of: 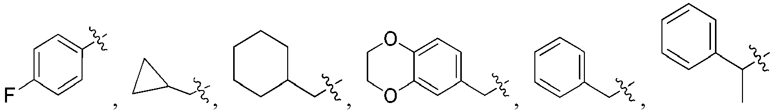 (including 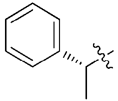 and

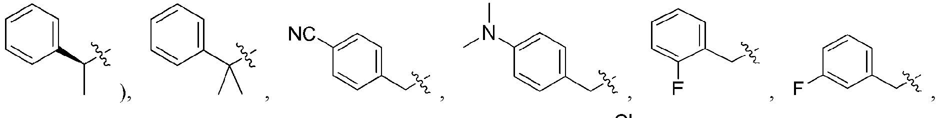

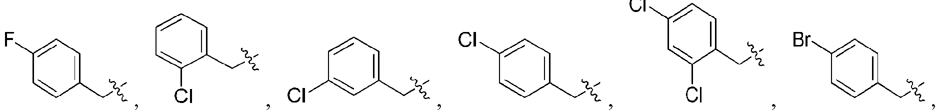

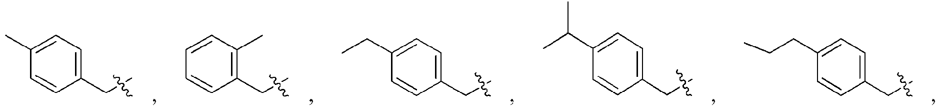

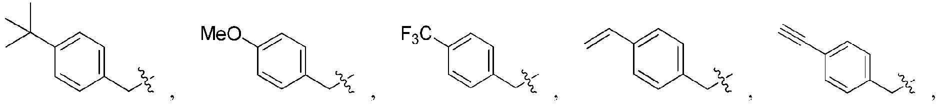

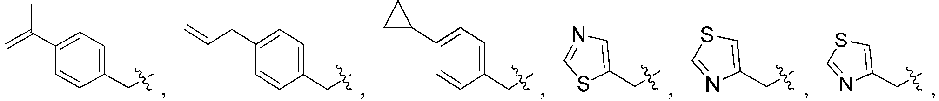

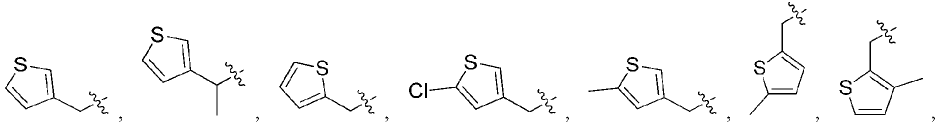

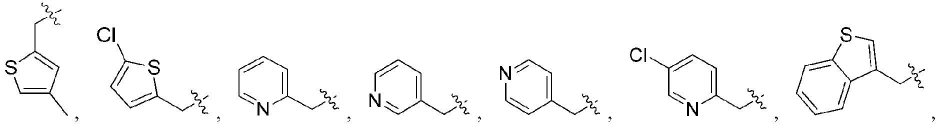

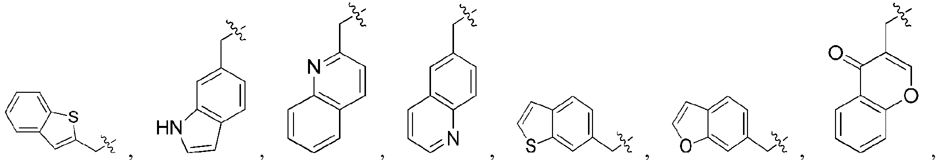

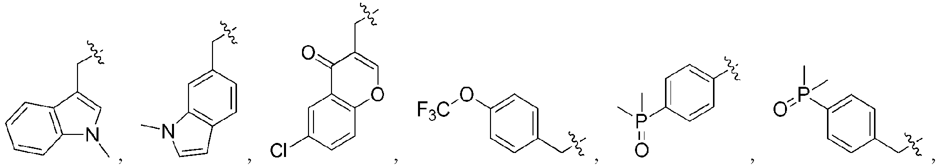

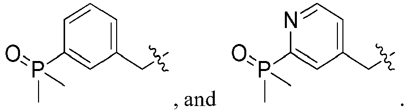

34. The compound according to claim 21, or a pharmaceutically acceptable salt, ester, stereoisomer, polymorph, solvate, N-oxide, isotopically labeled compound, metabolite, or prodrug thereof, wherein R^{1b} is selected from the group consisting of: H, optionally substituted C₁₋₄ alkyl, optionally substituted C₃₋₇ cyclic hydrocarbyl, optionally substituted phenyl, -C₁₋₃ alkylene-(optionally substituted C₃₋₇ cyclic hydrocarbyl), and -C₁₋₃ alkylene-optionally substituted phenyl.

35. The compound according to claim 21, or a pharmaceutically acceptable salt, ester, stereoisomer, polymorph, solvate, N-oxide, isotopically labeled compound, metabolite, or prodrug thereof, wherein R^{1b} is selected from the group consisting of:
H, phenyl;
optionally substituted C₁₋₄ alkyl, wherein the alkyl is methyl, ethyl, or isopropyl;
optionally substituted C₃₋₇ cyclic hydrocarbyl and -C₁₋₃ alkylene-(C₃₋₇ cyclic hydrocarbyl), wherein the cyclic hydrocarbyl is cyclopropyl, cyclobutyl, cyclopentyl, or cyclohexyl; and
-C₁₋₃ alkylene-phenyl.

36. The compound according to claim 21, or a pharmaceutically acceptable salt, ester, stereoisomer, polymorph, solvate, N-oxide, isotopically labeled compound, metabolite, or prodrug thereof, wherein R¹³ is selected from the group consisting of halogen and C₁₋₄ alkyl.

37. The compound according to claim 21, or a pharmaceutically acceptable salt, ester, stereoisomer, polymorph, solvate, N-oxide, isotopically labeled compound, metabolite, or prodrug thereof, wherein R¹³ is selected from the group consisting of F, Cl, Br, and methyl.

38. The compound according to claim 21, or a pharmaceutically acceptable salt, ester, stereoisomer, polymorph, solvate, N-oxide, isotopically labeled compound, metabolite, or prodrug thereof, wherein R^{1b} is selected from the group consisting of H, methyl, ethyl, isopropyl, CF₃CH₂, cyclopropyl, 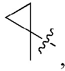 phenyl, 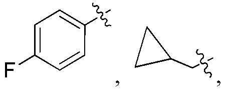 and

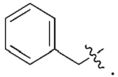

39. The compound according to claim 22, or a pharmaceutically acceptable salt, ester, stereoisomer, polymorph, solvate, N-oxide, isotopically labeled compound, metabolite, or prodrug thereof, wherein R¹³ is selected from the group consisting of halogen and -OR¹¹, and wherein R¹¹ is selected from the group consisting of C₁₋₄ alkyl.

40. The compound according to claim 22, or a pharmaceutically acceptable salt, ester, stereoisomer, polymorph, solvate, N-oxide, isotopically labeled compound, metabolite, or prodrug thereof, wherein R¹³ is selected from the group consisting of F, Cl, Br, and -OCH₃.

41. The compound according to claim 22, or a pharmaceutically acceptable salt, ester, stereoisomer, polymorph, solvate, N-oxide, isotopically labeled compound, metabolite, or prodrug thereof, wherein R^{2a} and R^{2b} are each selected from the group consisting of phenyl,

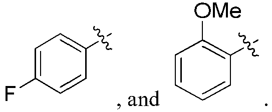

42. The compound according to claim 1, or a pharmaceutically acceptable salt, ester, stereoisomer, polymorph, solvate, N-oxide, isotopically labeled compound, metabolite, or prodrug thereof, wherein:
U is ethylene;
R^{1a} is selected from the group consisting of -C₁₋₆ alkylene-C₆₋₁₀ aryl; and -C₁₋₆ alkylene-(5- to 14-membered heteroaryl);
R^{1b} is selected from the group consisting of C₁₋₈ alkyl; saturated C₃₋₁₀ cyclic hydrocarbyl; and -C₁₋₆ alkylene-saturated C₃₋₁₀ cyclic hydrocarbyl;
X¹ is N; X⁴ is C(=O); X² is N; R^{2a} and R^{2b} are both phenyl;
R³ is -C(=O)OH;
R⁴ is H;
h and k are each independently 1; and
the cyclic hydrocarbyl, aryl and heteroaryl groups recited above are, at each occurrence, optionally substituted with one R¹³ group; said R¹³ is C₁₋₆ alkyl;
preferably, the C₆₋₁₀ aryl is phenyl, the saturated C₃₋₁₀ cyclic hydrocarbyl is cyclopropyl, and the 5- to 14-membered heteroaryl is thienyl or benzothienyl.

43. The compound according to claim 1, or a pharmaceutically acceptable salt, ester, stereoisomer, polymorph, solvate, N-oxide, isotopically labeled compound, metabolite, or prodrug thereof, wherein the compound is selected from the group consisting of:

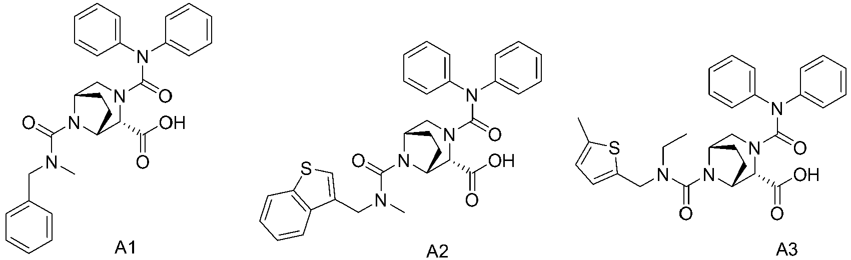

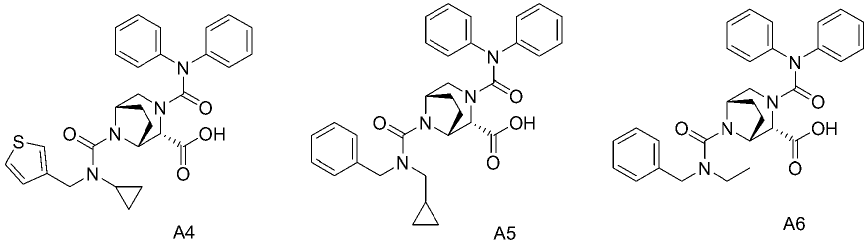

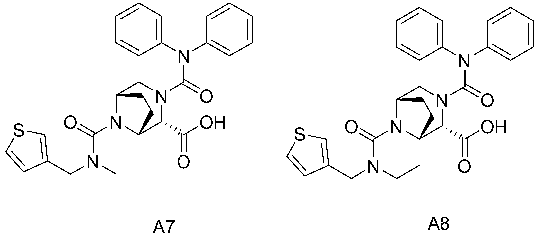

44. Use of the compound according to any one of claims 1 to 43, or a pharmaceutically acceptable salt, ester, stereoisomer, polymorph, solvate, N-oxide, isotopically labeled compound, metabolite, or prodrug thereof, in the manufacture of a medicament for preventing or treating peripheral neuropathic pain.

45. Use of the compound according to any one of claims 1 to 43, or a pharmaceutically acceptable salt, ester, stereoisomer, polymorph, solvate, N-oxide, isotopically labeled compound, metabolite, or prodrug thereof, in the manufacture of a medicament for preventing or treating macrophage-associated peripheral neuropathic pain.

46. The use according to claim 44 or 45, wherein the medicament is for preventing or treating peripheral neuropathic pain associated with the recruitment/accumulation of macrophages.

47. The use according to claim 44 or 45, wherein the medicament is for preventing or treating peripheral neuropathic pain associated with high expression of AT2 receptors in macrophages.

48. The use according to claim 44 or 45, wherein the medicament is for preventing or treating peripheral neuropathic pain associated with elevated levels of reactive oxygen/nitrogen species generated by macrophages.

49. The use according to any one of claims 44 to 48, wherein the medicament is for preventing or treating peripheral neuropathic pain caused by secondary neuropathy.

50. The use according to claim 49, wherein the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).

51. The use according to any one of claims 44 to 50, wherein the medicament is for preventing or treating a disorder selected from the group consisting of trigeminal neuralgia, chronic neuropathic pain following peripheral nerve injury, painful polyneuropathy, postherpetic neuralgia, and painful radiculopathy, or symptoms associated therewith.

52. The use according to any one of claims 44 to 50, wherein the medicament is for preventing or treating a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.

53. A method for preventing or treating peripheral neuropathic pain, comprising administering to a patient in need thereof a therapeutically effective amount of the compound according to any one of claims 1 to 43, or a pharmaceutically acceptable salt, ester, stereoisomer, polymorph, solvate, N-oxide, isotopically labeled compound, metabolite, or prodrug thereof.

54. A method for preventing or treating macrophage-associated peripheral neuropathic pain, comprising administering to a patient in need thereof a therapeutically effective amount of the compound according to any one of claims 1 to 43, or a pharmaceutically acceptable salt, ester, stereoisomer, polymorph, solvate, N-oxide, isotopically labeled compound, metabolite, or prodrug thereof.

55. The method according to claim 53 or 54, wherein the disease is peripheral neuropathic pain associated with the recruitment/accumulation of macrophages.

56. The method according to claim 53 or 54, wherein the disease is peripheral neuropathic pain associated with high expression of AT2 receptors in macrophages.

57. The method according to claim 53 or 54, wherein the disease is peripheral neuropathic pain associated with elevated levels of reactive oxygen/nitrogen species generated by macrophages.

58. The method according to any one of claims 53 to 57, wherein the disease is peripheral neuropathic pain caused by secondary neuropathy.

59. The method according to claim 58, wherein the secondary neuropathy comprises: diabetic neuropathy; herpes zoster-associated neuropathy; uremia-associated neuropathy; amyloid neuropathy; HIV sensory neuropathy; hereditary motor and sensory neuropathy; hereditary sensory neuropathy; hereditary sensory and autonomic neuropathy; hereditary ulcero-mutilating neuropathy; nitrofurantoin-induced neuropathy; tomaculous neuropathy; neuropathy caused by nutritional deficiency; neuropathy caused by renal failure and complex regional pain syndrome; neuropathy caused by repetitive activities (e.g., typing or working on an assembly line); peripheral neuropathy caused by antiretroviral drugs (e.g., zalcitabine and didanosine), antibiotics (e.g., metronidazole and isoniazid), gold compounds, chemotherapeutic drugs (e.g., vincristine), alcohol, lead, arsenic, mercury, and organophosphate pesticides; and peripheral neuropathy associated with infectious processes (e.g., Guillain-Barré syndrome).

60. The method according to any one of claims 53 to 59, wherein the disease is a disorder selected from the group consisting of trigeminal neuralgia, chronic neuropathic pain following peripheral nerve injury, painful polyneuropathy, postherpetic neuralgia, and painful radiculopathy, or symptoms associated therewith.

61. The method according to any one of claims 53 to 59, wherein the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain, chemotherapy-associated neuropathic pain, and HIV (Human Immunodeficiency Virus) painful sensory neuropathy, or symptoms associated therewith.

62. The compound according to any one of claims 1 to 43, or a pharmaceutically acceptable salt, ester, stereoisomer, polymorph, solvate, N-oxide, isotopically labeled compound, metabolite, or prodrug thereof, for use in the prevention or treatment of peripheral neuropathic pain.

63. The use according to any one of claims 44 to 50, wherein the medicament is for preventing or treating a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.

64. The use according to any one of claims 44 to 50, wherein the medicament is for preventing or treating a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (including chronic post-surgical neuralgia), chemotherapy-associated neuropathic pain, and HIV (Human Immunodeficiency Virus) painful sensory neuropathy, or symptoms associated therewith.

65. The method according to any one of claims 53 to 59, wherein the disease is a disorder selected from the group consisting of post-surgical neuralgia, trigeminal neuralgia, chronic neuropathic pain following peripheral nerve injury, painful polyneuropathy, postherpetic neuralgia, and painful radiculopathy, or symptoms associated therewith.

66. The method according to any one of claims 53 to 59, wherein the disease is a disorder selected from the group consisting of painful diabetic peripheral neuropathy, postherpetic neuralgia, peripheral nerve entrapment syndromes (e.g., carpal tunnel syndrome), cranial neuralgia, post-surgical or post-traumatic neuropathic pain (including chronic post-surgical neuralgia), chemotherapy-associated neuropathic pain, and HIV (Human Immunodeficiency Virus) painful sensory neuropathy, or symptoms associated therewith.
